# EUROPÄISCHE PATENTANMELDUNG

(11) **EP 1 584 676 A1**
(43) Veröffentlichungstag der Anmeldung: **12.10.2005**
(21) Anmeldenummer: 05004522.8
(22) Anmeldetag: 02.03.2005
(51) Int. Cl.: C12N 9/02, C12Q 1/26, G01N 33/68

(54) **Kristallstruktur der mitochondrialen Protoporphyrinogen-IX-Oxidase**

(30) Priorität: 15.03.2004 DE 102004012730; 24.05.2004 DE 102004025412
(71) Anmelder: Bayer CropScience AG, 40789 Monheim (DE); Max-Planck-Gesellschaft zur Förderung der Wissenschaften e.V., 80539 München (DE)
(72) Erfinder: Freigang, Jörg, Dr., 40764 Langenfeld (DE); Koch, Michael, 81375 Leverkusen (DE); Breithaupt, Constanze, 81377 München (DE); Kiefersauer, Reiner, Dr., 82061 Neuried (DE); Messerschmidt, Albrecht, Prof. Dr., 81241 München (DE); Huber, Robert, Prof. Dr., 82110 Germering (DE)
(74) Vertreter: Krieg, Robert Alexander

(57) **Zusammenfassung**

Die vorliegende Erfindung betrifft Kristalle einer Protoporphyrinogen-IX-Oxidase, Verfahren zur Herstellung von Kristallen einer Protoporphyrinogen-IX-Oxidase, Verfahren zur Bestimmung der Struktur von Protoporphyrinogen-IX-Oxidasen alleine oder im Komplex mit einem Liganden, sowie Verfahren zum Identifizieren von Liganden der mitochondrialen Protoporphyrinogen-IX-Oxidase, die als Herbizide Verwendung finden können. Die vorliegende Erfindung betrifft auch die Verwendung der hier beschriebenen Kristallstruktur der mitochondrialen Protoporphyrinogen-IX-Oxidase aus *Nicotiana tabacum* zur Erzeugung von Proteinmodellen verwandter Enzyme und die Identifizierung von Inhibitoren verwandter Proteine.

## Beschreibung

Die vorliegende Erfindung betrifft Kristalle einer Protoporphyrinogen-IX-Oxidase, Verfahren zur Herstellung von Kristallen einer Protoporphyrinogen-IX-Oxidase, Verfahren zur Bestimmung der Struktur von Protoporphyrinogen-IX-Oxidasen alleine oder im Komplex mit einem Liganden, sowie Verfahren zum Identifizieren von Liganden der mitochondrialen Protoporphyrinogen-IX-Oxidase, die als Herbizide Verwendung finden können. Die vorliegende Erfmdung betrifft auch die Verwendung der hier beschriebenen Kristallstruktur der mitochondrialen Protoporphyrinogen-IX-Oxidase aus *Nicotiana tabacum* zur Erzeugung von Proteinmodellen verwandter Enzyme und die Identifizierung von Inhibitoren verwandter Proteine.

Die enzymatische Aktivtät der Protoporphyrinogen-IX-Oxidase (im Folgenden auch kurz als "PPO" bezeichnet) besteht in der Katalyse der Oxidation von Protoporphyrinogen-IX (Formel I) zu Protoporphyrin-IX (Formel II) durch molekularen Sauerstoff:

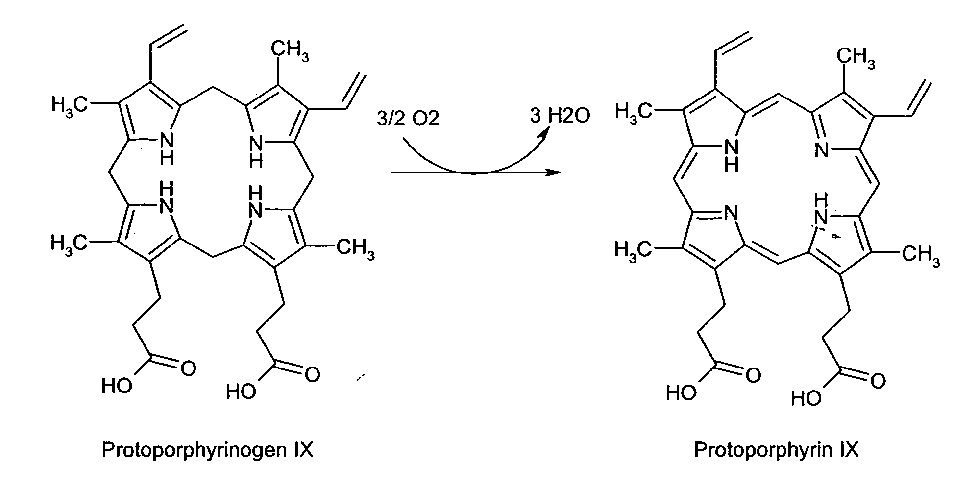

Das Protein ist ein Repräsentant einer hochkonservierten Enzymfamilie, bei der die Membranassoziierung vermutlich durch einen konservierten, hydrophoben Bereich im Protein erreicht wird (Arnould et al., 1999). In Pflanzen kommen mit der plastidischen und der mitochondrialen Protoporphyrinogen-IX-Oxidase zwei Isoformen des Enzyms vor (Lermontova et al., 1997). Während die plastidische Isoform sich in Thylakoidmembranen sowie innerer und äußerer Plasmamembran befindet, ist die mitochondriale Form in der inneren Membran von Mitochondrien lokalisiert.

Die Protoporphyrinogen-IX-Oxidase stellt bekanntermaßen ein wichtiges Target peroxidierender Herbizide wie z.B. Acifluorfen dar. Die Inhibition des Enzyms führt zu einer Akkumulation von Protoporphyrin-IX, das sich durch spontane Reaktion von Protoporphyrinogen-IX bildet. Die Entkopplung der Protoporphyrinbildung und der anschließenden, durch das Protein Ferrochelatase katalysierten Einführung eines Eisenions resultiert in einem Verlust der Rückkopplungskontrolle der Häm-Biosynthese durch das Endprodukt Häm. Das dadurch akkumulierte, photosensitive Protophorphyrin-IX erzeugt Singulett-Sauerstoff, der durch Peroxidation von Lipiden zum Zelltod führt. Das beobachtete Ausbleichen von photosynthetisch aktiven Pflanzenteilen ist auf diesen Effekt zurückzuführen (Lermontova et al., 1997). Die Protoporphyrinogen-IX-Oxidase ist daher ein für die Suche nach weiteren, verbesserten herbiziden Wirkstoffen besonders geeignetes Enzym. Dabei ist insbesondere auch die räumliche Struktur dieses Proteins von besonderem Interesse, die es ermöglichen könnte, ohne aufwendige biochemische Verfahren Inhibitoren des Enzyms und damit potentielle Herbizide zu identifizieren.

### Beschreibung der Abbildungen und des Sequenzprotokolls:

- **Abbildung 1:**: Abbildung 1 zeigt die Struktur des Homodimers der PPO mit eingebundenem Inhibitor 4-Brom-3-(5'-carboxy-4'-chlor-2'-fluorphenyl)-1-methyl-5-trifluormethyl-pyrazol (beschriftet mit INH), Kofaktor FAD und unspezifisch gebundenem Detergensmolekül Triton X-100.
- **Abbildung 2:**: Abbildung 2 zeigt eine schematische Darstellung der PPO-Ligandenbindenische mit eingebundenem Inhibitor 4-Brom-3-(5'-carboxy-4'-chlor-2'-fluorphenyl)-1-methyl-5-trifluormethyl-pyrazol.
- **SEQ ID NO: 1:**: SEQ ID NO: 1 (PPO) zeigt die Aminosäuresequenz der PPO aus *Nicotiana tabacum* mit den Mutationen 1M- und L224Q:

Die Aufgabe der vorliegenden Erfindung war es, die räumliche Struktur der kristallinen Form der mitochondrialen Protoporphyrinogen-IX-Oxidase und insbesondere die räumliche Struktur der Ligandenbindenische der PPO zur Verfügung zu stellen, um deren Verwendung bei der Suche nach neuen herbiziden Wirkstoffen zu ermöglichen.

Die Aufgabe wurde gelöst durch die Bereitstellung eines Kristalls der PPO im Komplex mit dem Inhibitor 4-Brom-3-(5'-carboxy-4'-chlor-2'-fluorphenyl)-1-methyl-5-trifluormethyl-pyrazol, der Strukturkoordinaten dieses Kristalls und Verfahren zum Identifizieren von Liganden der PPO bzw. von Inhibitoren der enzymatischen Aktivität dieses Enzyms.

Gegenstand der vorliegenden Erfmdung ist somit der Kristall einer PPO, insbesondere einer pflanzlichen PPO. Bevorzugt handelt es sich bei der erfindungsgemäßen kristallinen PPO um die aus *Nicotiana tabacum.* Besonders bevorzugt umfasst die erfindungsgemäße PPO die Aminosäuresequenz gemäß SEQ ID NO: 1.

Bevorzugt handelt es sich beim erfindungsgemäßen Kristall einer PPO um eine PPO mit dem gebundenem Inhibitor 4-Brom-3-(5'-carboxy-4'-chlor-2'-fluorphenyl)-1-methyl-5-trifluormethylpyrazol. Der erfindungsgemäße Kristall einer PPO mit dem gebundenem Inhibitor 4-Brom-3-(5'-carboxy-4'-chlor-2'-fluorphenyl)-1-methyl-5-trifluormethyl-pyrazol weist vorzugsweise die in Tabelle 2 defmierten Strukturkoordinaten auf.

Die Erfindung bezieht sich insbesondere auf einen Kristall mit der Raumgruppe C222₁ und den Zellkonstanten a = 119,1 Å, b = 147,3 Å, c = 127,0 Å (siehe auch Tabelle 1). Eine solche Struktur umfasst auch einen Kristall mit den Strukturkoordinaten gemäß Tabelle 2.

Die Erfindung bezieht sich auch auf ein Verfahren zur Herstellung eines erfindungsgemäßen Kristalls, in dem ein mit Seleno-Methionin substituiertes Protein eingesetzt wird. Die Erfindung bezieht sich weiterhin auf ein Verfahren zur Herstellung eines erfindungsgemäßen Kristalls, in dem eine PPO in einem bakteriellen Wirt (z.B. *E. coli)* rekombinant bei Anwesenheit von Seleno-Methionin im Medium hergestellt wird, das mit Seleno-Methionin substituierte Protein aus den bakteriellen Zellen gewonnen und gereinigt wird und Kristalle dieses Proteins hergestellt werden. Die Kristalle können mit jeder gängigen Methode hergestellt werden, z.B. im hängenden Tropfen.

Die Erfindung bezieht sich daher auch auf ein Verfahren zur Herstellung eines erfindungsgemäßen Kristalls, in dem der Kristall durch Dampfdiffusion in einem sitzendem Tropfen erzeugt wird, wobei die Kristallisationslösung 10% Polyethylenglykol 1000, 0,1 M Natriumcitrat/HCl, pH 4,0 und 0,2 M Natriumchlorid umfasst.

Die dreidimensionale Struktur wurde mit Hilfe von Proteinkristallen, die einer Röntgenstrukturanalyse zugänglich sind, mittels anomaler Dispersion bei einer einzelnen Wellenlänge gelöst und bei einer Auflösung von 2.9 Å vollständig verfeinert. Gegenstand der vorliegenden Erfindung ist somit auch die anhand dieser Strukturkoordinaten ermittelbare dreidimensionale Struktur des Komplexes aus PPO und gebundenem Inhibitor 4-Brom-3-(5'-carboxy-4'-chlor-2'-fluorphenyl)-1-methyl-5-trifluormethyl-pyrazol.

Gegenstand der vorliegenden Erfindung ist daher insbesondere ein Kristall einer PPO wie vorstehend beschrieben, der eine Ligandenbindenische umfasst, welche durch die folgenden Aminosäuren im 4.5 Å Abstand vom gebundenen Liganden definiert ist: Arg⁹⁸, Ala¹⁷⁴, Gly¹⁷⁵, Thr¹⁷⁶, Cys¹⁷⁷, Gly¹⁷⁸, Leu³³⁴, Phe³⁵³, Gly³⁵⁴, Val³⁵⁵, Leu³⁵⁶, Leu³⁶⁹, Gly³⁷⁰, Thr³⁷¹, Leu³⁷², Phe³⁹², Phe⁴³⁹. Die Sequenz- und Positionsangaben beziehen sich auf die Aminosäuren gemäß SEQ ID NO: 1.

Weiterhin ist der Kristall eines PPO-Liganden-Komplexes Gegenstand der vorliegenden Erfindung, welcher eine Ligandenbindenische umfasst, die durch die oben beschriebenen Aminosäuren definiert ist, und in der eine oder mehrere dieser Aminosäuren mutiert sind. Vorzugsweise handelt es sich dabei um konservative Mutationen, in der ein Austausch durch eine Aminosäure mit ähnlichen physikalischen Eigenschaften erfolgt.

Die Koordinaten der Tabelle 2 geben die Koordinaten der Atome in Ångstrom wieder. Die Koordinaten sind ein relativer Datensatz von Positionen, die eine Struktur in drei Dimensionen definiert. So ist es möglich, dass ein völlig anderer Datensatz von Koordinaten, der auf einem anderen Ursprung und/oder Achsen basiert, dennoch eine gleiche oder identische Struktur wiedergibt. Ändert man die relativen Positionen der Atome der Struktur in einer Weise, dass bei der Überlagerung mit den entsprechenden Koordinaten gemäß Tabelle 2 die Wurzel der mittleren quadratischen Abweichung der Atome des Peptidrückgrats des Proteins kleiner ist als 1,5 Å, bevorzugt kleiner als 1,0 Å, besonders bevorzugt kleiner als 0,5 Å, ergibt sich in der Regel ebenfalls eine Struktur, die im Wesentlichen hinsichtlich ihrer strukturellen Charakteristika und ihrer Verwendbarkeit für die strukturbasierte Identifizierung von Liganden des Proteins einer Struktur gemäß Tabelle 2 entspricht. Ebenso ist zu bemerken, dass die Änderung der Anzahl und/oder der Positionen der Wassermoleküle und/oder Substratmoleküle der Tabelle 2 die Nutzbarkeit der Strukturkoordinaten gemäß Tabelle 2 nicht grundsätzlich beeinträchtigt.

Vom Umfang der vorliegenden Erfindung wird es deshalb als umfasst betrachtet, wenn die in Tabelle 2 gezeigten Strukturkoordinaten zu einem anderen Ursprung und/oder anderen Achsen hin verschoben werden, oder wenn die relativen Positionen der Atome der Struktur in einer Weise geändert werden, dass die Wurzel der mittleren quadratischen Abweichung der Atome des Peptidrückgrats des Proteins im Vergleich zu den Koordinaten gemäß Tabelle 2 kleiner ist als 1,5 Å, bevorzugt kleiner als 1,0 Å, besonders bevorzugt kleiner als 0,5 Å, oder wenn die Anzahl und/oder die Positionen der Wassermoleküle und/oder der Substratmoleküle verändert werden.

Durch die Bindung eines Liganden bzw. Inhibitors an den PPO-Komplex kann die Struktur des PPO-Komplexes Veränderungen erfahren. Oft beschränken sich diese Veränderungen auf Seitenkettenkonformationen, jedoch können sich auch ganze Gruppen von Aminosäuren mit ihrem Peptidrückgrat bewegen. Darüberhinaus treten in Enzymen mit unbesetzter Ligandenbindenische oder in Enzymen, deren Ligandenbindenische mit Inhibitoren besetzt sind, typische Konfonnationsänderungen auf. Solche Veränderungen werden von der vorliegenden Erfindung ebenfalls umfasst.

Dem Fachmann ist bewusst, dass für verschiedene der hierein beschriebenen erfindungsgemäßen Verfahren nicht der gesamte Datensatz gemäß Tabelle 2 benötigt wird, sondern oft nur Teile desselben. Zum Beispiel können, wie im Folgenden noch beschrieben, für die Modellierung einer chemischen Verbindung basierend auf den Strukturdaten eines erfindungsgemäßen Kristalls ausgewählte Koordinaten des PPO-Kristalls verwendet werden. Oft genügen bereits 5, bevorzugt 10, besonders bevorzugt 50 und ganz besonders bevorzugt 100 Atome der PPO-Struktur, um eine chemische Verbindung zu modellieren, die die Fähigkeit besitz in Wechselwirkung mit der PPO zu treten. In entsprechender Weise können auch für die anderen hierin beschriebenen Verfahren, wie z.B. dem Homologie-basierten Modelling und der Homologie-basierten Strukturaufklärung alle oder nur Teile der Strukturkoordinaten gemäß Tabelle 2 verwendet werden.

Die vorstehend beschriebenen Aspekte der Erfindung stellen einzeln oder in Kombination miteinander bevorzugte Ausgestaltungen der Erfindung dar.

Neben der hierin beschriebenen dreidimensionalen Struktur eines Kristalles einer PPO, die bereits unmittelbar von großer Bedeutung für die Suche nach Liganden und damit auch nach Inhibitoren des Enzyms ist, bezieht sich die vorliegenden Erfindung auch auf deren Einsatz bei Strukturbestimmung von zur vorliegenden PPO homologen Proteinen. So funktioniert die Strukturvorhersage derzeit dann zufrieden stellend, d.h. ohne extrem großen Aufwand, wenn man die Struktur eines homologen Proteins, d.h. eines Proteins mit gleicher Abstammung, kennt. Dann kann man davon ausgehen, dass eine ähnliche 3D-Struktur vorliegt, und die bekannte Struktur kann als Vorlage (Template) benutzt werden, um mittels Homologie-basiertem Modelling ("Homology Modelling") ein Modell für die 3D-Struktur des anderen Proteins zu erstellen. So ermöglicht es die erfmdungsgemäße dreidimensionale Struktur der PPO auch, die dreidimensionale Struktur einer PPO aus anderen Organismen als *Nicotiana tabacum* durch Modelling vorherzusagen. Solche Proteinmodelle können in der gleichen Weise verwendet werden, wie die hier gelöste dreidimensionale Struktur.

Durch Vergleich der Unterschiede in den Aminosäuresequenzen ist es dann auch möglich, Unterschiede in den Ligandenbindenischen der PPO verschiedener Organismen vorherzusagen. Dies ist nützlich, wenn für spezifische Organismen spezifische Liganden gesucht werden, oder wenn im umgekehrten Fall gerade breit wirksame unspezifische Liganden gesucht werden. Darüberhinaus kann die erfindungsgemäße dreidimensionale Struktur auch zur Erstellung von Proteinmodellen anderer in der Sequenz verwandter Enzyme dienen.

Wenn also Röntgenstrukturdaten für eine PPO z.B. aus einem anderen Organismus, einen Komplex aus einer PPO und einem Liganden vorliegen, können die hier zur Verfügung gestellten Strukturdaten gemäß Tabelle 2 verwendet werden, um die erhaltenen Daten in einfacher Weise zu interpretieren und eine Struktur zu erstellen. Dazu werden Verfahren verwendet, die dem Fachmann geläufig sind. Ein Verfahren, das zu diesem Zweck eingesetzt werden kann, wird als "Molecular Replacement" bezeichnet. Anhand dieser Methode kann die neue Struktur schneller und effizienter bestimmt werden als bei dem Versuch diese Struktur *ab initio* zu erstellen. Beispiele für geeignete Computer Programme, die gängigerweise verwendet werden sind CNX (Brunger et al., 1998) und AMORE (Navaza, 1994).

Der Grad der Übereinstimmung zweier Proteinstrukturen korreliert dabei gut mit dem Grad der Sequenzidentität. In der Regel kann die Methode des Molecular Replacement bereits bei einer Identität zwischen der Sequenz des Templates und der Sequenz der zu lösenden Struktur von 40% verwendet werden. Unter dem Begriff "Homologie" bzw. "Identität" soll die Anzahl der übereinstimmenden Aminosäuren (Identität) mit anderen Proteinen, ausgedrückt in Prozent verstanden werden. Bevorzugt wird die Identität durch Vergleiche einer gegebenen Sequenz zu anderen Proteinen mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Die Identität kann standardmäßig mittels bekannten und der Öffentlichkeit zur Verfügung stehenden Computerprogrammen wie z.B. ClustalW (Thompson et al., *Nucleic Acids Research* 22 (1994), 4673-4680) ermittelt werden. ClustalW wird z.B. öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Genetique et de Biologie Moléculaire et Cellulaire, B.P.163, 67404 Illkirch Cedex, France; ftp://ftp-igbmc.u-strasbg.fr/pubn und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK), heruntergeladen werden. Wenn das ClustalW Computerprogramm der Version 1.8 benutzt wird, um die Identität zwischen z.B. einem gegebenen Referenzprotein und anderen Proteinen zu bestimmen, sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP. Eine Möglichkeit zum Auffinden von ähnlichen Sequenzen ist die Durchführung von Sequenzdatenbankrecherchen. Hierbei wird eine oder werden mehrere Sequenzen als sogenannte Abfrage ("query") vorgegeben. Diese Abfragesequenz wird dann mittels statistischen Computerprogrammen mit Sequenzen, die in den ausgewählten Datenbanken enthalten sind, verglichen. Solche Datenbankabfragen ("blast searches") sind dem Fachmann bekannt und können bei verschiedenen Anbietern durchgeführt werden. Wird eine solche Datenbankabfrage z.B. beim NCBI (National Center for Biotechnology Information, http://www.ncbi.nlm.nih.gov/) durchgeführt, so sollen die Standardeinstellungen, die für die jeweilige Vergleichsanfrage vorgegeben sind, benutzt werden. Für Proteinsequenzvergleiche ("blastp") sind dieses folgende Einstellungen: Limit entrez = nicht aktiviert; Filter = low complexity aktiviert; Expect value = 10; word size = 3; Matrix = BLOSUM62; Gap costs: Existence =11, Extension = 1. Als Ergebnis einer solchen Abfrage werden neben anderen Parametern auch der Anteil an Identität zwischen der Abfragesequenz und den in den Datenbanken aufgefundenen ähnlichen Sequenzen dargestellt. Unter einem erfindungsgemäßen Protein sollen daher im Zusammenhang mit der vorliegenden Erfindung solche Proteine verstanden werden, die bei der Verwendung mindestens einer der vorstehend beschriebenen Methoden zur Identitätsbestimmung eine Identität von mindestens 40 % aufweisen, bevorzugt von mindestens 50 %, besonders bevorzugt von mindestens 60 %, weiter bevorzugt von mindestens 70 %, und insbesondere von mindestens 80 %.

Daher ist Gegenstand der vorliegenden Erfindung auch ein Verfahren zur Bestimmung der Struktur eines Proteins mit der enzymatischen Aktivität einer PPO, welches vorzugsweise eine Identität von zumindest 40%, bevorzugt von 50%, besonders bevorzugt von 60% und ganz besonders bevorzugt von 70% zu der Sequenz gemäß SEQ ID NO:1 aufweist, umfassend die folgenden Schritte:
(a) Alignment einer der Aminosäuresequenzen einer PPO mit einer Identität von zumindest 40% zu SEQ ID NO:1 mit der Aminosäuresequenz gemäß SEQ ID NO:1,
(b) Modellieren der Struktur der übereinstimmenden homologen Regionen auf Basis der Strukturdaten gemäß Tabelle 2 für diese Regionen, und
(c) Bestimmen der Konformation der PPO, wobei die Konformation im Wesentlichen die Struktur der übereinstimmenden homologen Regionen beibehält.

Der Begriff "homologe Region(en)" bezieht sich auf Aminosäurereste zweier Sequenzen, die identisch sind oder ähnlich (z.B. aliphatisch, aromatisch, polar, negativ geladen, positiv geladen etc.). Solche Reste werden häufig als "invariant" bzw. "konserviert" bezeichnet.

Vorzugsweise werden einer oder alle der Schritte (a) bis (c) mittels Computer-gestütztem Modelling durchgeführt. Dieses "Homology Modelling" ist ein dem Fachmann bekanntes Verfahren (siehe z.B. Greer, 1985 und Blundell et al., 1988).

Die Strukturen von Aminosäuren, die in nicht-konservierten Regionen liegen, können von Hand zugeordnet werden, indem man die übliche Peptidgeometrie oder Simulationstechniken verwendet, wie z.B. die Computersimulation von Moleküldynamik ("Molecular Dynamics"). Der abschließende Schritt des Verfahrens besteht in der Verbesserung ("Refinement") der Gesamtstruktur durch Molekulardynamik und/oder Energieminimierung ("Energy Minimization").

Die vorliegende Erfindung umfasst auch Mutanten, wobei sich dieser Begriff auf Polypeptide bezieht, die man durch den Ersatz von zumindest einer Aminosäure der PPO gemäß SEQ ID NO:1 durch eine andere Aminosäure und/oder durch die Insertion und/oder Deletion einer Aminosäure erhält, und die noch die im Wesentlichen gleiche dreidimensionale Struktur haben wie die PPO gemäß Tabelle 2. Als eine im Wesentlichen gleiche Struktur wird hier ein Satz von Strukturkoordinaten bezeichnet, bei denen die Wurzel der mittleren quadratischen Abweichung kleiner oder gleich 2,0 Å ist, wenn man die Strukturkoordinaten mit den Strukturkoordinaten gemäß Tabelle 2 überlagert, und wenn zumindest 50%, bevorzugt 70% und besonders bevorzugt alle der C_{α}-Atome der PPO der bekannten Struktur in dieser Überlagung enthalten sind.

Unter Einsatz der erfindungsgemäßen Kristallstruktur der PPO können mit Hilfe etablierter automatisierter Computerprozesse Datenbanken durchgemustert werden, die die Strukturen einer großen Zahl von Verbindungen enthalten (Virtual Screening), die als Liganden einer PPO bzw. Inhibitoren der enzymatischen Aktivität einer PPO von Interesse sind ("Kandidatenmoleküle"). Bei diesen Kandidatenmolekülen handelt sich im Allgemeinen um kleine organische (niedermolekulare) Moleküle, die zum Einsatz als Wirkstoffe z.B. im Pflanzenschutz geeignet sind. Solche Wirkstoffe sollten einer chemischen Synthese zugänglich sein. Für das virtuelle Screenen können zum Beispiel Algorithmen wie FLEXX (Rarey et al., 1996) oder GOLD (Jones et al., 1997) verwendet werden. Durch diese Vorgehensweise können Verbindungen identifiziert werden, deren dreidimensionale Struktur es ermöglicht, in die Bindetasche zu gelangen und dort zu binden, beispielsweise durch Bildung von Wasserstoffbrücken, durch hydrophobe Wechselwirkung, durch Ladungswechselwirkungen, durch van-der-Waals-Wechselwirkungen oder durch Dipol-Wechselwirkungen. Die so identifizierten Verbindungen können synthetisiert werden und dann z.B. als Herbizide Verwendung finden.

Gegenstand der vorliegenden Erfindung ist daher auch ein Verfahren zum Identifizieren von Liganden der PPO bzw. von Inhibitoren deren enzymatischer Aktivität umfassend die folgenden Schritte:
(a) Erstellen einer dreidimensionalen Struktur einer PPO,
(b) Erstellen der dreidimensionalen Struktur eines Kandidatenmoleküls, und
(c) Einpassen der dreidimensionalen Struktur des Kandidatenmoleküls in die dreidimensionale Struktur der PPO.

Bevorzugt werden zur Erstelllung der dreidimensionalen Struktur einer PPO die Daten aus Tabelle 2 verwendet. In gleicher Weise können jedoch auch die Strukturkoordinaten anderer, zur vorliegenden PPO gemäß SEQ ID NO:1 homologer PPO verwendet werden, die mit einem wie vorstehend beschriebenen Verfahren basierend auf den Strukturkoordinaten gemäß Tabelle 2 bestimmt wurden.

Alternativ dazu können auch nur die Koordinaten der Atome der PPO gemäß Tabelle 2 verwendet werden, die sich in räumlicher Nähe der Bindungsregion des putativen Liganden bzw. Inhibitors befinden. Diese können genutzt werden, um eine Ligandenbindenische zu modellieren, in der der putative Ligand bzw. Inhibitor binden kann. Diese Koordinaten werden also verwendet, um einen Raum zu definieren, der dann "*in silico*" zum Screenen von Kandidatenmolkülen mit Hilfe geeigneter Computer-basierter Verfahren wie oben angegeben genutzt werden kann.

Die vorliegende Erfindung bezieht sich daher auch auf ein Verfahren zum Identifizieren von Liganden der PPO bzw. von Inhibitoren deren enzymatischer Aktivität umfassend die folgenden Schritte:
(a) Erstellen der Koordinaten von zumindest zwei Atomen einer PPO ("selektierte Koordinaten"),
(b) Erstellen der dreidimensionalen Struktur eines Kandidatenmoleküls, und
(c) Einpassen der dreidimensionalen Struktur des Kandidatenmoleküls in die selektierten Koordinaten der PPO.

Bevorzugt werden zur Erstelllung der selektierten Koordinaten einer PPO die Daten aus Tabelle 2 verwendet. In gleicher Weise können jedoch auch die Koordinaten anderer, zur vorliegenden PPO gemäß SEQ ID NO:1 homologer PPO verwendet werden, die mit einem wie vorstehend beschriebenen Verfahren basierend auf den Strukturkoordinaten gemäß Tabelle 2 bestimmt wurden.

In der Regel wird es von Vorteil sein wenn das Modell der Ligandenbindenische auf einer ausreichenden Anzahl von Atomen einer PPO beruht. Bevorzugt werden daher die Koordinaten von Atomen der Reste Arg⁹⁸, Phe³⁹², Leu³⁵⁶ und Leu³⁷² verwendet. Besonders bevorzugt werden die Koordinaten von Atomen der Reste Arg⁹⁸, Ala¹⁷⁴, Gly¹⁷⁵, Thr¹⁷⁶, Cys¹⁷⁷, Gly¹⁷⁸, Leu ³³⁴, Phe³⁵³, Gly³⁵⁴, Val³⁵⁵, Leu³⁵⁶, Leu³⁶⁹, Gly³⁷⁰, Thr³⁷¹, Leu³⁷², Phe³⁹², Phe⁴³⁹ gemäß SEQ ID NO:1 1 verwendet.

Der Begriff "Einpassen" ("Fitting") beschreibt die Computer-gestützte oder teilweise Computer-gestützte Bestimmung der Interaktion zwischen zumindest einem Atom des Kandidatenmoleküls und zumindest einem Atom der PPO sowie die Berechnung der Stabilität dieser Interaktion. Für dieses "Fitting" stehen verschiedene, dem Fachmann bekannte Verfahren zur Verfügung.

Eine weitere Anwendung der erfindungsgemäßen dreidimensionalen Struktur des PPO-Komplexes liegt in der Generierung neuer Liganden. Hierzu werden unter Einsatz der Struktur und mit Hilfe etablierter *de novo* Designprogramme am Computer Strukturformeln für neue Liganden generiert, die in die Bindetasche gelangen und dort binden können, beispielsweise durch Bildung von Wasserstoffbrücken, durch hydrophobe Wechselwirkung, durch Ladungswechselwirkungen, durch van-der-Waals-Wechselwirkungen oder durch Dipol-Wechselwirkungen. Beispiele für mögliche *de novo* Designprogramme sind LUDI (Böhm, 1992), LEGEND (Nishibata & Itai, 1991) oder GROW (Moon & Howe, 1991). Solcherart generierte Verbindungen können synthetisiert und dann ebenfalls z.B. als Herbizide genutzt werden.

Liganden bzw. potentielle Inhibitoren, die entweder in einem *in silico* Screening Verfahren wie vorstehend beschrieben gefunden wurden, oder die wie vorstehen beschrieben *de novo* generiert und synthetisiert wurden, können in einem folgenden Schritt auf ihre herbizide Aktivität geprüft werden.

Die erfindungsgemäßen Verfahren umfassen daher gegebenenfalls auch die folgenden weiteren Schritte:
(a) Synthese der identifizierten Liganden bzw. potentiellen Inhibitoren,
(b) Inkontaktbringen dieser Liganden bzw. potentiellen Inhibitoren mit einer PPO, und
(c) Bestimmen der enzymatischen Aktivität der PPO.

Alternativ können die Liganden bzw. potentiellen Inhibitoren auch unmittelbar, gegebenenfalls in einer geeigneten Formulierung, mit einer Pflanze oder pflanzlichen Zellen in Kontakt gebracht werden und die wachstumsinhibierende oder abtötende Wirkung der Verbindungen auf diese Pflanze bestimmt werden.

Anstelle oder zusätzlich zu den genannten *in vitro* oder *in vivo* Verfahren zur Überprüfung der identifizierten Verbindungen, können die vorstehend genannten Verfahren auch die folgenden weiteren Schritte umfassen:
(a) Synthese der identifizierten Liganden bzw. potentiellen Inhibitoren,
(b) Bildung eines Komplexes aus einer PPO und dem Liganden bzw. potentiellen Inhibitor, und
(c) Analyse des Komplexes mittels Röntgenstrukturanalyse zur Bestimmung der Fähigkeit des Liganden bzw. potentiellen Inhibitors mit der PPO zu interagieren.

Auf diese Weise können detaillierte Informationen über die Bindung des Liganden und potentiellen Inhibitors erhalten werden. Diese Informationen können auch genutzt werden um Anpassungen an der Struktur des Liganden vorzunehmen, zum Beispiel um dessen Wirksamkeit und/oder Spezifität z.B. für die PPO eines bestimmten Organismus zu verbessern. Die vorstehend beschriebenen Schritte der Auswahl eines Liganden im Screening oder dessen *de novo* Generierung, der Prüfung in einem biochemischen *in vitro* oder in einem *in vivo* Test, und die Analyse des Komplexes mittels der Bestimmung der Struktur können auch mehrfach wiederholt werden, um die Charakteristika des Liganden bzw. Inhibitors nach und nach zu verbessern.

Die Bestimmung der Struktur des Komplexes aus einer PPO und einem identifizierten Liganden bzw. Inhibitor ist dabei mittels des vorstehend genannten Verfahrens basierend auf den Strukturkoordinaten gemäß Tabelle 2 zur Bestimmung der Struktur einer homologen PPO oder eines neuen Komplexes aus einer PPO gemäß SEQ ID NO:1 und einem neuen Liganden in effizienter Weise möglich. Dabei kann ein neuer Ligand durch das so genannte "Soaking" in dem Fachmann bekannter Weise in bereits vorliegende PPO-Kristalle eingeführt werden. PPO und Ligand können jedoch auch kokristallisiert werden.

Die identifzierten Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emu-Igier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Beim Einsatz der erfindungsgemäßen Verbindungen als Fungizide können die Aufwandmengen je nach Applikation innerhalb größerer Bereiche variiert werden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Emtegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die nachfolgenden Beispiele illustrieren verschiedene Aspekte der vorliegenden Erfindung und sind nicht limitieren auszulegen.

### Beispiele

### Beispiel 1:

### Proteinherstellung und Kristallisation

Bei der proteolytischen Spaltung des verwendeten Fusionsprotein wurde neben der gewünschten Abtrennung des N-terminalen Teils eine weitere Spaltung innerhalb der PPO beobachtet. Zur Unterdrückung dieser unerwünschten Nebenreaktion wurde Lysin224 zu Glutamin224 mutiert. Die PPO aus *Nicotiana tabacum* wurde in Form von Fusionsproteinen mit Thioredoxin und einem Hexahistidinteil mit Hilfe des Expressionsvektors pET32a exprimiert. Der dazu verwendete Bakterienstamm war B834(DE3). Die Zellen wurden in einem Minimalmedium kultiviert, das anstelle von Methionin Seleno-Methionin enthielt. Nach Aufschluss der Zellen wurde ein dreistufiges Reinigungsprotokoll zur Isolierung der PPO verwendet. Dazu wurde in einem ersten Schritt das Zelllysat an einem Ni-NTA-Gel säulenchromatographisch gereinigt. Nach proteolytischem Entfernen des aminoterminalen Fusionsteils wurde das Protein anschliessend über eine Gelfiltrationssäule (Sephacryl S-200) und danach über eine Anionenaustauschersäule (ResourceQ) chromatographisch gereinigt. Zur Kristallisation wurde das Protein in einen Puffer überführt, der 5mM Tris/HCl, pH8.0, 0.1% Triton X-100 und 50 mM Natriumchlorid enthielt. Die Kristalle wurden mit der Methode der Dampfdiffusion im hängenden Tropfen erzeugt. Die verwendete Kristallisationslösung enthielt 10% Polyethylenglykol 1000, 0.1 M Natriumcitrat/HCl, pH 4.0 und 0.2 M Natriumchlorid. In einem von 2 Tagen bildeten sich Kristalle mit Dimensionen von 150 x 100 x100 µm³. Die intensiv gelben Kristalle gehörten der Raumgruppe C222₁ an und wiesen Achsenlängen von a=119.1 Å, b=147.3 Å und c=127.0 Å auf.

### Beispiel 2:

### Kristallographische Datenaufnahme und Analyse

Die ursprünglich nur bis zu einer Auflösung von ca. 8 Å beugenden Kristalle wurden mit hilfe eines Free-Mounting-Systems (Firma Proteros biostructures GmbH, Martinsried) in eine andere Kristallform transformiert. Röntgenbeugungsdaten wurden bei einer Wellenlänge von 0.9790 Å am ESRF (European Radiotion Synchrotron Facility) in Grenoble, Frankreich aufgenommen. Die Beugungsbilder wurden mit dem Programm XDS (Kabsch, 1993) prozessiert. Anomale Streuzentren wurden mit dem Programm SnB (Weeks & Miller, 1999) identifiziert. Schwermetallatomparameter wurden mit dem Programm SHARP (Fortell & Bricogne, 1997) verfeinert. Die Modellierung des Strukturmodells in die resultierende Elektronendichte erfolgte mit dem Programm o (Jones et al., 1991). Die iterative Verfeinerung des Modells wurde mit dem Programm CNS (Brünger et al., 1998) durchgeführt (vgl. Tabelle 1).

### Beispiel 3:

### Architektur des Inhibitor-Komplexes der PPO

Die 503 Aminosäuren der PPO bilden eine kompakte Gesamtstruktur, die drei Domänen umfasst: Eine FAD-bindende Domäne, eine substratbindende Domäne mit p-Hydroxybenzoathydrolase-ähnlicher Topologie und eine membranbindende Domäne. Die an der FAD-Bindung beteiligten Strukturteile zeigen signifikante Sequenz- und Strukturhomologien zu anderen Flavoenzymen, wie humane Monoamine-Oxidase B (Binda et al., 2002) mit 1.68 Å rmsd ("root mean square deviation") über 256 Cα-Atome und einer Sequenzidentität von 15.1 %, Polyamin-Oxidase (Binda et al., 1999) mit 1.71 Å rmsd über 190 Cα-Atome und einer Sequenzidentität von 14.9 %, sowie D-Aminosäure-Oxidase (Mitsutani et al., 1996) mit 1.98 Å rmsd über 148 Cα-Atome und einer Sequenzidentität von 14.9 %. Das Modell impliziert, daß die acht Helices α4 bis α11, die die konservierte Sequenzregion von Rest 150 bis Rest 213 enthalten, den Membrananker darstellen und vermutlich monotop in die Membran eingebettet sind. In diesem Modell sind überwiegend apolare Reste zum hydrophoben Teil der Membran gerichtet. Geladene Reste, die ebenfalls im membraneingebetteten Oberflächenbereicht lokalisiert sind, sind an der Ausbildung von Salzbrücken beteiligt oder können den positiv geladenen Zuckerphosphat-Bereich der Membran erreichen. Diese Reste sind ausschließlich Lysine oder Histidine. In der PPO Kristallstruktur stehen die Membranbindedomänen von jeweils vier Protomeren in Kontakt miteinander. Dabei bedecken zwei Kontakte jeweils 800 Å³ solvenszugänglicher Oberfläche. Senkrecht dazu existieren zwei weitere Kontakte, die je 400 Å³ Oberfläche bedecken. Letztere sind vermutlich die Kontakte, die das biologisch relevante Dimer aufbauen. Diese Wechselwirkung könnte in vivo zusätzlich durch Phospholipide der umgebenden Membran stabilisiert werden. In der dimeren Anordnung zweier PPO-Protomere liegen die aktiven Zentren auf der Aussenseite des Dimer, in der Nähe der Membranoberfläche. Die Dimer-Kontaktfläche wird durch die Helices α5 und α14 gebildet, wobei wesentliche Beiträge von Leuzin-Resten stammen, die mit den entsprechenden Leuzin-Resten im zweiten Monomer wechselwirken. Daraus resultiert ein T-förmiges Dimer, in dem zwei membranbindende Domänen einen hydrophoben Oberflächenbereich ausbilden, der hervoragend zur Insertion in die Membran geeignet ist. Diese Architektur ähnelt der ebenfalls monotop membrangebundenen Prostaglandin-H₂-Synthase (Loll et al., 1995).

### Beispiel 4:

### Das aktive Zentrum und die strukturelle Grundlage der Katalyse

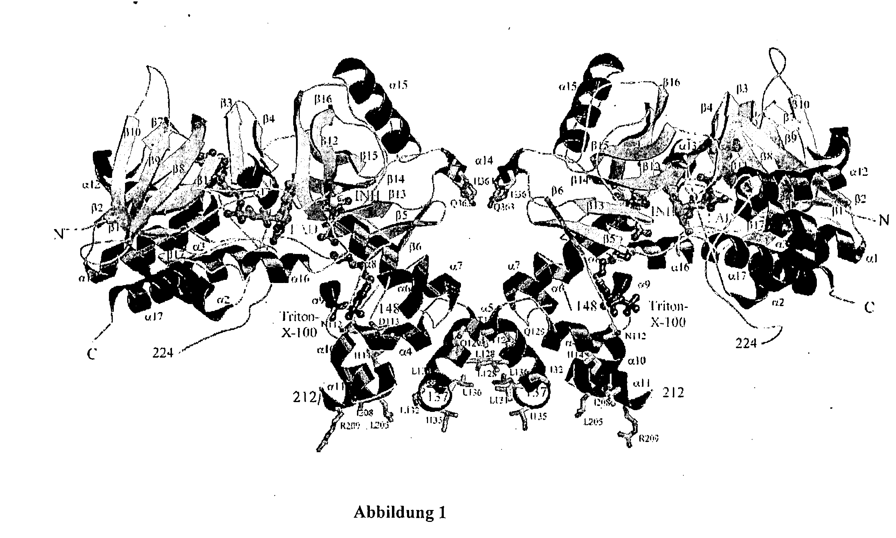

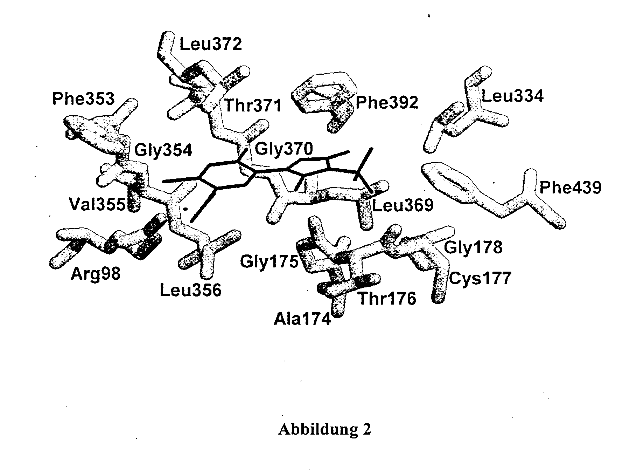

Das aktive Zentrum der PPO ist zwischen der FAD- und der Substratbindedomäne lokalisiert. Der Inhibitor bindet am aktiven Zentrum in einer tunnelförmigen Einstülpung in der Nähe des FAD-Kofaktors in einer gestreckten Konformation (Abb.1 und 2). Die Substratbindestelle neben dem FAD wird durch eine flache Einbuchtung gebildet, die von einer Vielzahl von aliphatischen und aromatischen Aminosäuren sowie Asn67 und Arg98 umgeben ist. Die Kristallstruktur impliziert ein Modell für die Substrat- und Produktbindung, in dem die negativ geladenen Propionylgruppen in Richtung der solvensexponierten Teile der Einbuchtung am aktiven Zentrum gerichtet sind. Arg98, das hochkonserviert in allen eukaryotischen PPOs ist, liefert die Gegenladung für die Carboxylgruppe des Inhibitors, und wahrscheinlich ebenso für die Propionatgruppe in Ring C von Protoporphyrinogen-IX. Der Pyrazolring des Inhibitors dient als Vorbild für die Bindung des Rings A in Protoporphyrinogen-IX und wird gebunden durch aromatische Wechselwirkung mit dem Rest Phe392, der konserviert in allen mitochondrialen PPOs aus Pflanzen ist. In Chloroplasten-Isoenzymen ist dieser Rest durch ein Tyrosin ersetzt. Der Phenylring des Inhibitors ahmt vermutlich die Bindung des Protoporphyrinogenrings B nach und ist zwischen den konservierten Resten Leu356 und Leu372 eingeklemmt. In dem beschriebenen Modell ist die Methylenbrücke (C20) zwischen den Ringen A und D des Protoporphyrinogen-IX in Richtung des reaktiven N5-Atoms des FAD-Kofaktors orientiert. Diese Orientierung impliziert, dass die Oxidation durch FAD zuerst an dieser Stelle stattfindet. Die weitere Oxidation sollte von dieser Position aus fortschreiten, da die schmale Einbuchtung am aktiven Zentrum die Substratbindung nur in dieser Position erlauben und eine Rotation des Substrats ausschliessen sollte. Durch eine Wasserstoffumordnung über Imin-Enamin-Tautomerisierung können alle Hydridabspaltungen über die C20-Position erfolgen. Das Atom C10 zwischen den Ringen A und C kann sich nicht nahe genug an das N5-Atom des FAD annähern, da sich an dieser Stelle die Vinylgruppe auf der C10-Seite des Molekül befindet. Das FAD wird während der Reaktion dreimal durch molekularen Sauerstoff zurück oxidiert, so dass 3 Wasserstoffperoxid-Moleküle entstehen. In unmittelbarer Nachbarschaft des Substrats befinden sich keine Reste, die als Base fungieren könnten. Ein plausibler Mechanismus der Deprotonierung könnte eine Reduktion von molekularem Sauerstoff durch ein negativ geladenes FADH⁻ umfassen, wobei ein Peroxidanion entsteht, das ein Proton vom Lösungsmittel abstrahiert. Das resultierende Hydroxidanion könnte dann als allgemeine Base fungieren.

### Beispiel 5:

### In vitro-Test zur Bestimmung der enzymatischen Aktivität einer PPO bei An- und Abwesenheit eines Liganden

Zur Überprüfung der enzymatischen Aktivität der PPO wurde zuerst eine Lösung von frisch reduziertem Protoporphyrinogen IX hergestellt. Dazu wurden 1.5 mg kommerziell erhältliches Protoporphyrin IX mit 8 ml 10 mM KOH und 2ml Ethanol abs. versetzt. Diese Lösung wurde mit Argon entgast und zu 1.5 g unter Schutzgas abgewogenem 5%igem Natrium-Amalgam zugegeben. Nach 15 minütigem Stehen bei Raumtemperatur wurde mit 40%iger Phosphorsäure auf einen pH-Wert von 7.0 titriert. 5ml der resultierenden Lösung wurden durch einen Sterilfiter (Porengröße 0.45 µm, Durchmesser 1 cm) zu 25 ml Pufferlösung gegeben, die 100 mM K₂HPO₄, 100 mM KH₂PO₄, 1 mM EDTA und 0.3 % (v/v) TWEEN 80 bei einem pH-Wert von 7.2 enthielt. Zum Starten der enzymatischen Reaktion wurden je 250 µl der resultierenden Reaktionslösung mit 50 µl Enzymlösung (10 µg/ml in 5mM Tris/HCl, 50 mM NaCl, pH8.0) versetzt. Der Reaktionsverlauf wurde durch Beobachtung der Fluoreszenz nach 0, 5, 15 und 30 Minuten mit einem Fluoreszenz-Spektrometer bei einer Anregungsswellenlänge von 410 nm und einer Emissionswellenlänge von 635 nm verfolgt. Zur Korrektur des Effekts der unspezifischen Oxidation des Substrats durch Luftsauerstoff wurde parallel in gleicher Weise eine Probe gemessen, die kein Protein enthielt. Die aus dieser Messung resultierende Aktivität wurde von der Gesamtaktivität abgezogen, um die spezifische Aktivität zu erhalten.

### Beispiel 6:

### In vivo-Test zur Bestimmung der herbiziden Aktivität eines Liganden (Post-emergence-Test)

| | |
|---|---|
| Lösungsmittel | 5 Gewichtsteile Aceton |
| Emulgator | 1 Gewichtsteil Alkylarylpolyglykolether |

Zur Herstellung einer eine Kandidatenverbindung enthaltenden Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, dass die jeweils gewünschten Wirkstoffmengen pro Flächeneinheit ausgebracht werden. Die Konzentration der Spritzbrühe wird so gewählt, dass in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle. Es bedeuten dann: 0 % = keine Wirkung (wie unbehandelte Kontrolle), 100 % = totale Vernichtung.

**Tabelle 1:**

| **Kristallographische Daten und Verfeinerungsstatistik (Details siehe Beispiel 2)** | |
|---|---|
| **Datenaufnahme:** | |
| Auflösung (Å; letzte Schale) | 20-2.9 (3.0-2.9) |
| Raumgruppe | C222₁ |
| Zellkonstanten (Å) | 119.1/147.3/127.0 |
| Mittlere Redundanz | 3.8 (3.8) |
| R_{sym} (%; letzte Schale)* | 7.9 (52.8) |
| Vollständigkeit (%;letzte Schale) | 100.0 (99.3) |
| Mittlere I/sigma (%;letzte Schale) | 11.6 (2.8) |
| Verfeinerung: | |
| Anzahl Proteinatome | 6962 |
| Anzahl Solvenspositionen | 143 |
| Anzahl Ligandenatome | 192 |
| Mittlere quadratische Abweichung der Bindungslängen (Å) | 0.008 |
| Mittlere quadratische Abweichung der Valenzwinkel (°) | 1.3 |
| R_{cryst} (R_{free}) (%)§ | 22.7(29.3) |
| Werte in Klammern entsprechen der letzten Auflösungsschale | |

| | |
|---|---|
| *R_{sym} (I) = Σₕₖₗ Σᵢ \| I_{hkl, i} - < Iₕₖₗ > \| / Σₕₖₗ Σᵢ \| I_{hkl, i}\| wobei < Iₕₖₗ > die mittlere Intensität der multiplen I_{hkl,i} Beobachtungen von symmetrieäquivalenten Reflexen ist. | |
| Mittlere quadratische Abweichungen bezogen auf Idealwerte. §R_{cryst} = Σₕₖₗ \| F_{obs} - F_{calc} \| / Σₕₖₗ \| F_{obs} wobei F_{obs} bzw. F_{calc} die beobachteten und berechneten Strukturamplituden sind. R_{free} ist R_{cryst}, allerdings berechnet mit den 10% der Daten, die bei der Verfeinerung nicht berücksichtigt wurden. | |

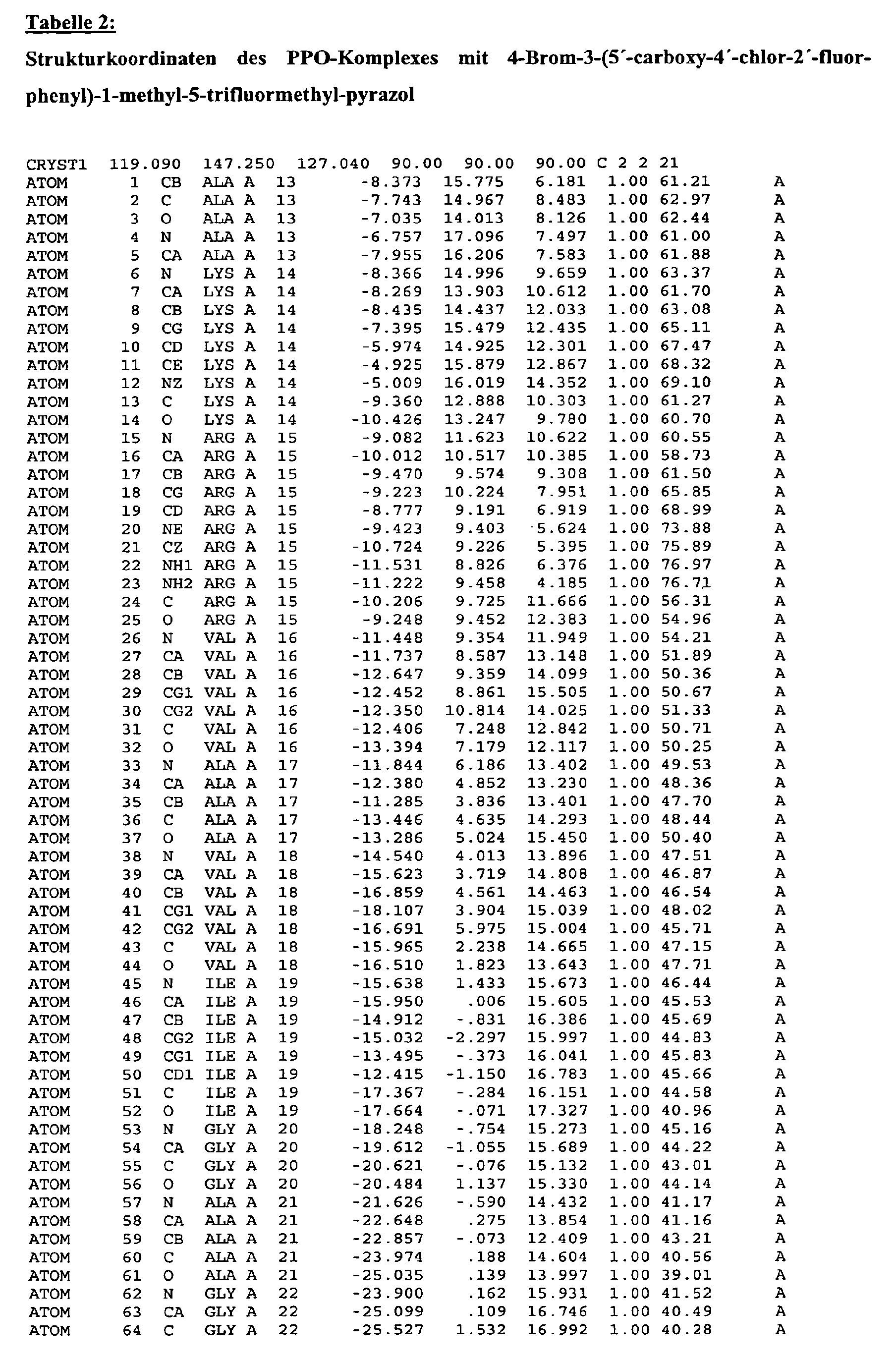

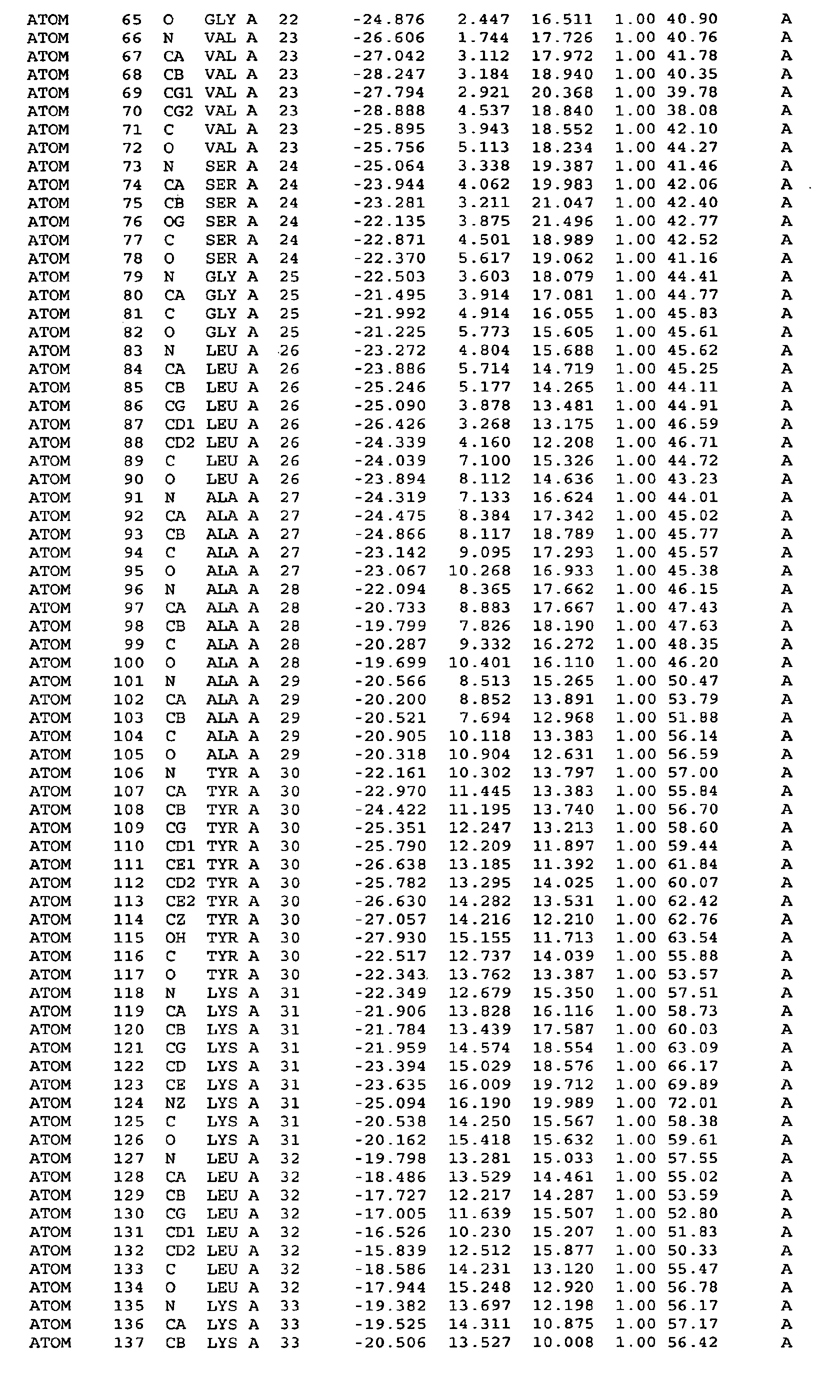

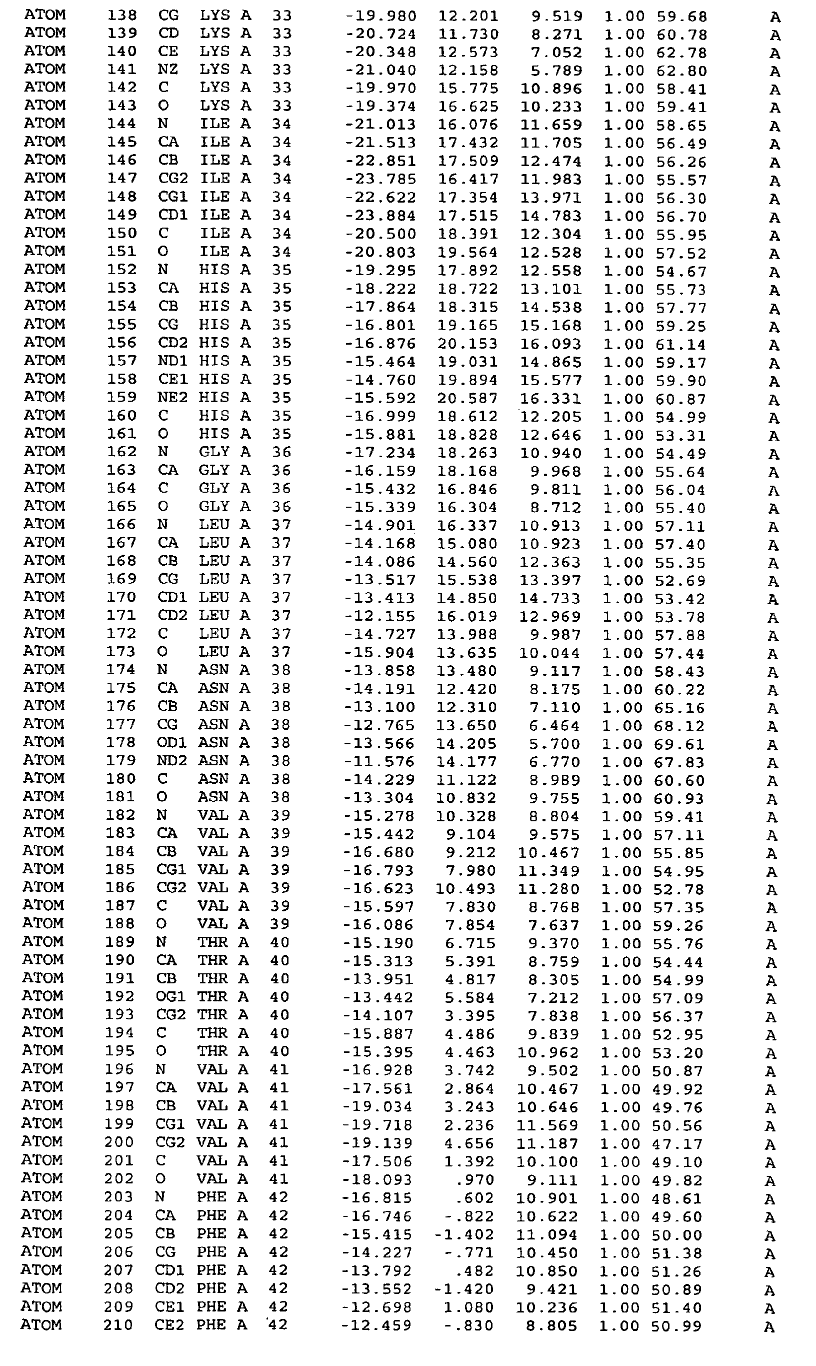

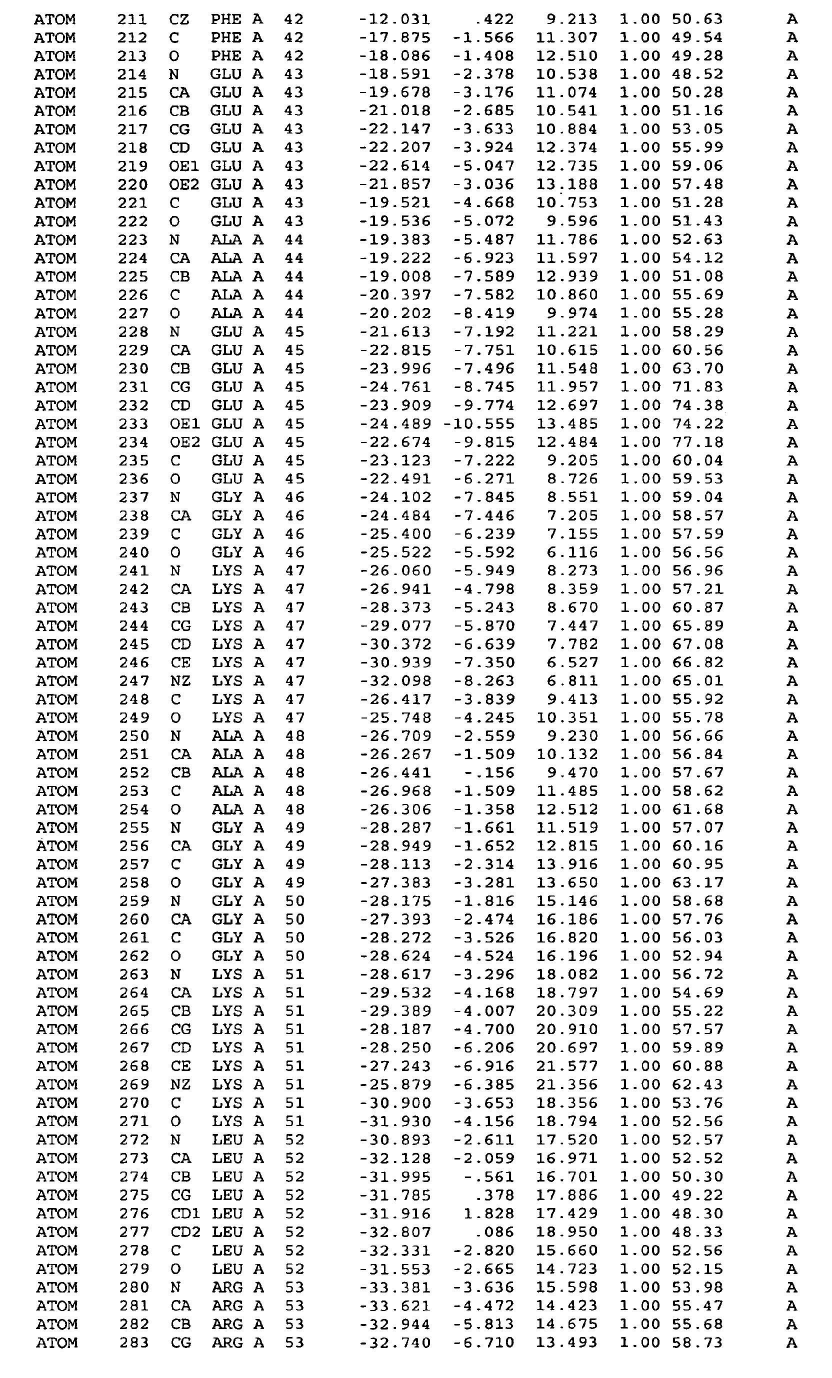

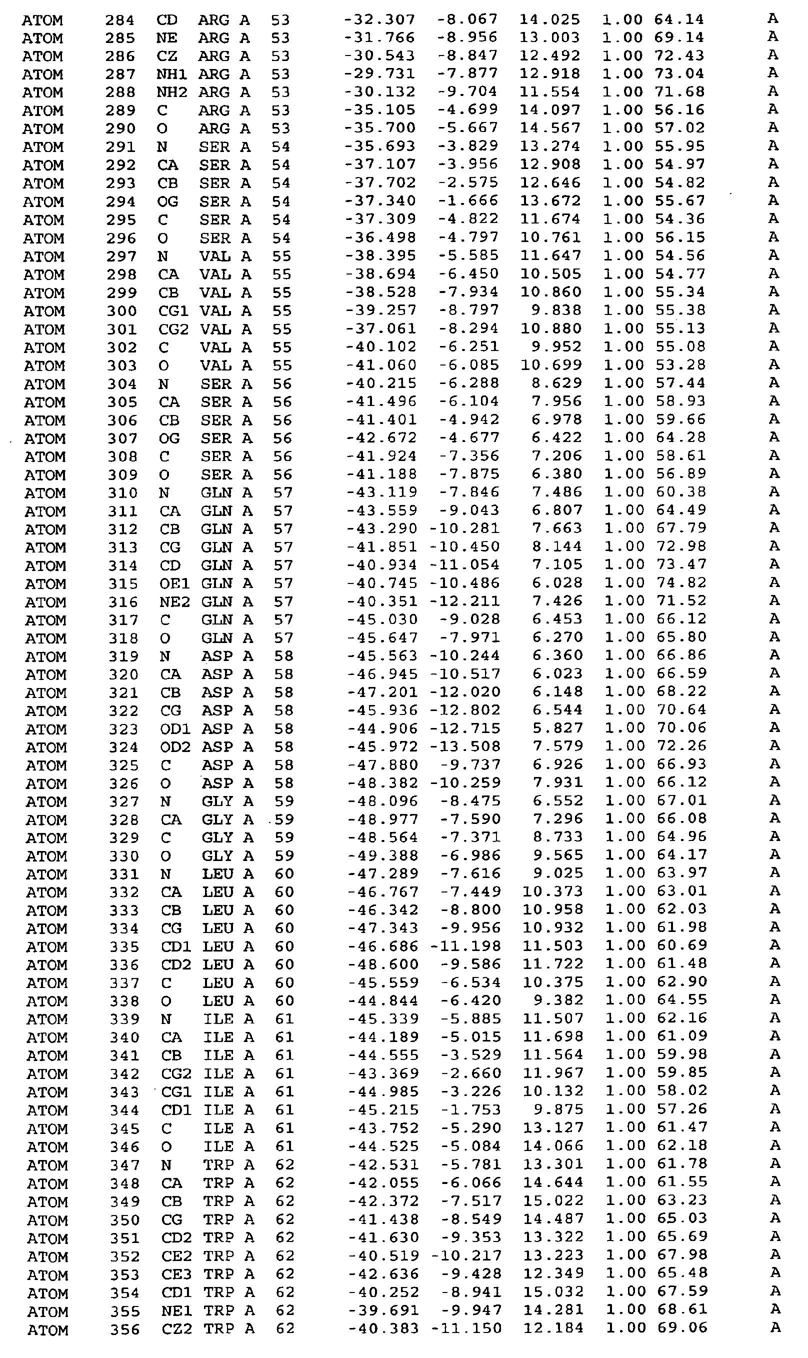

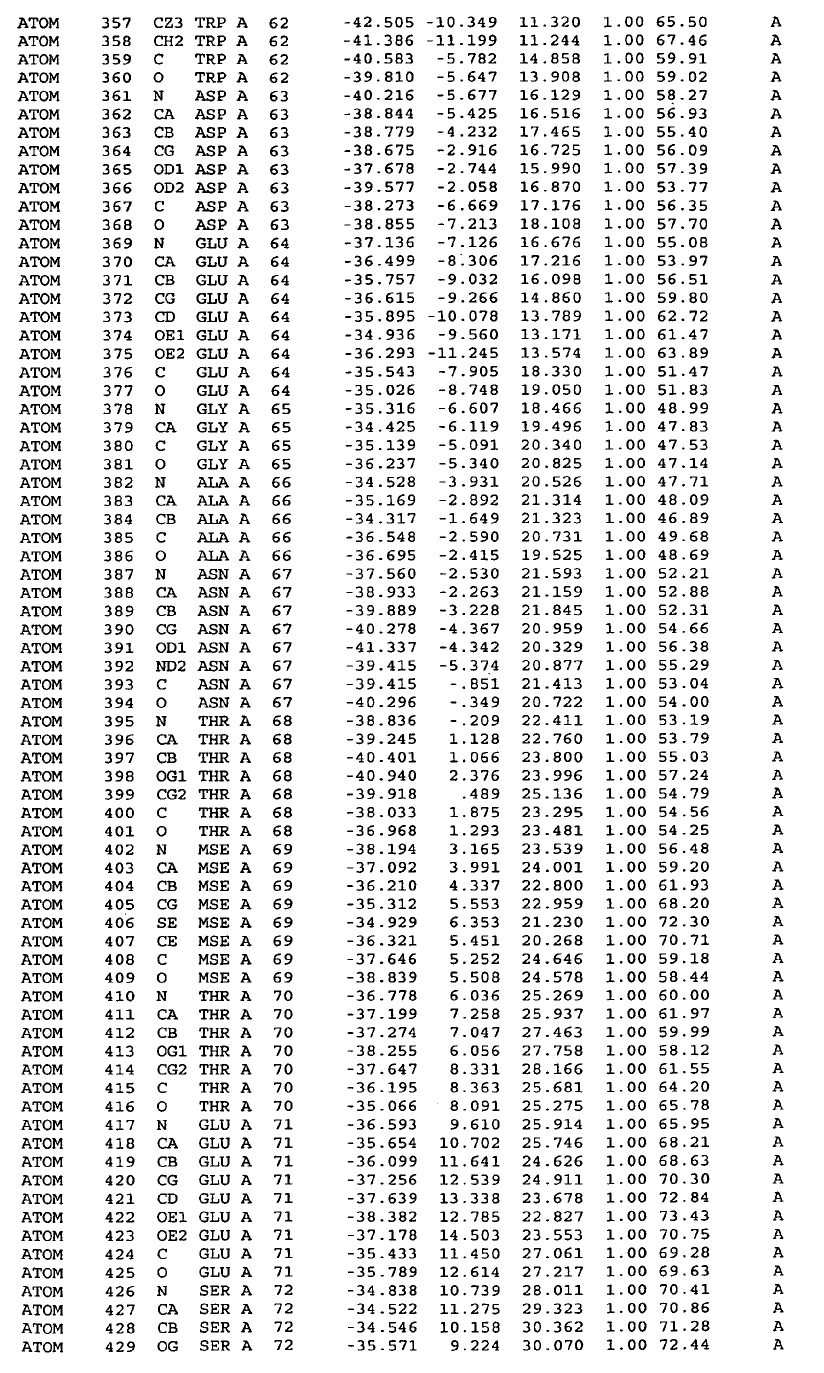

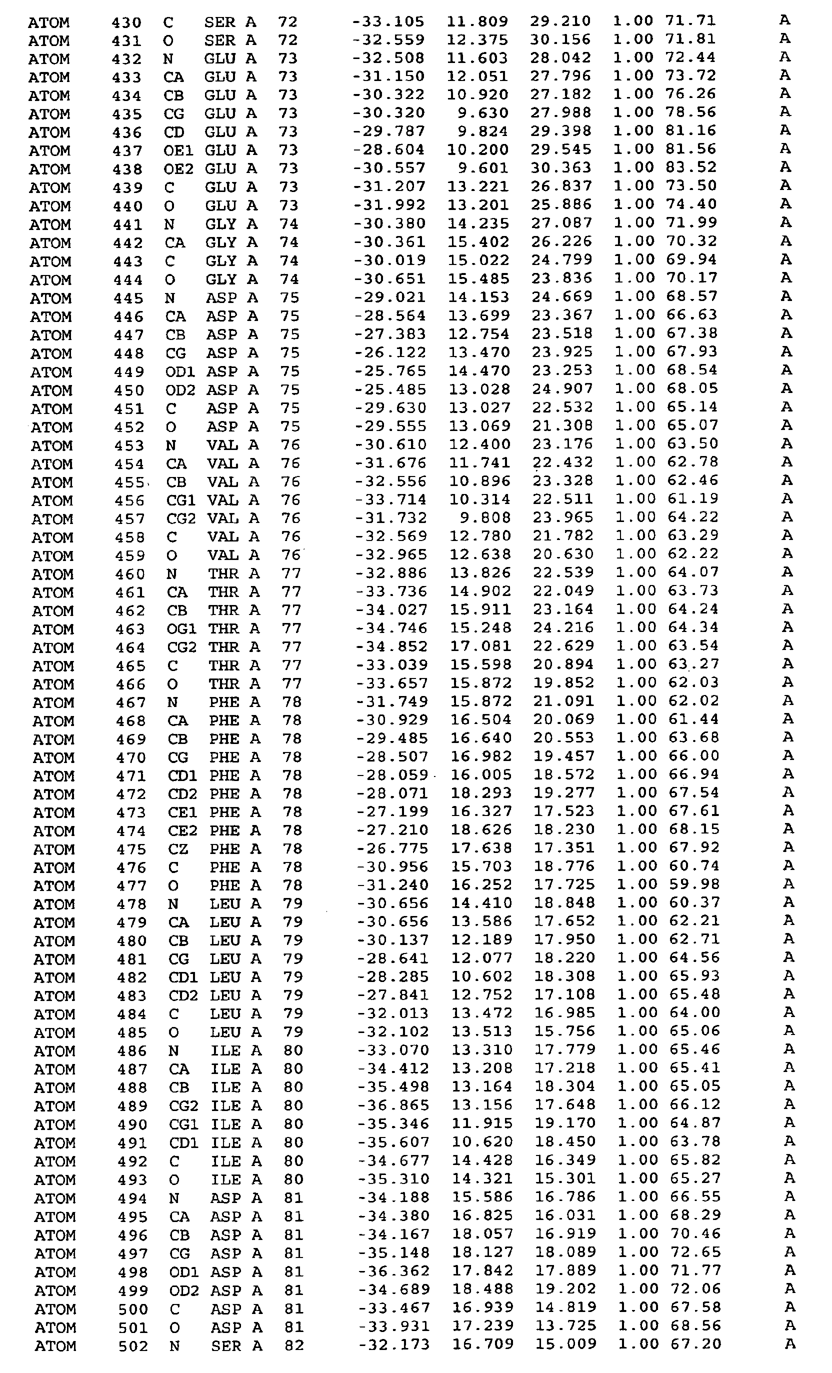

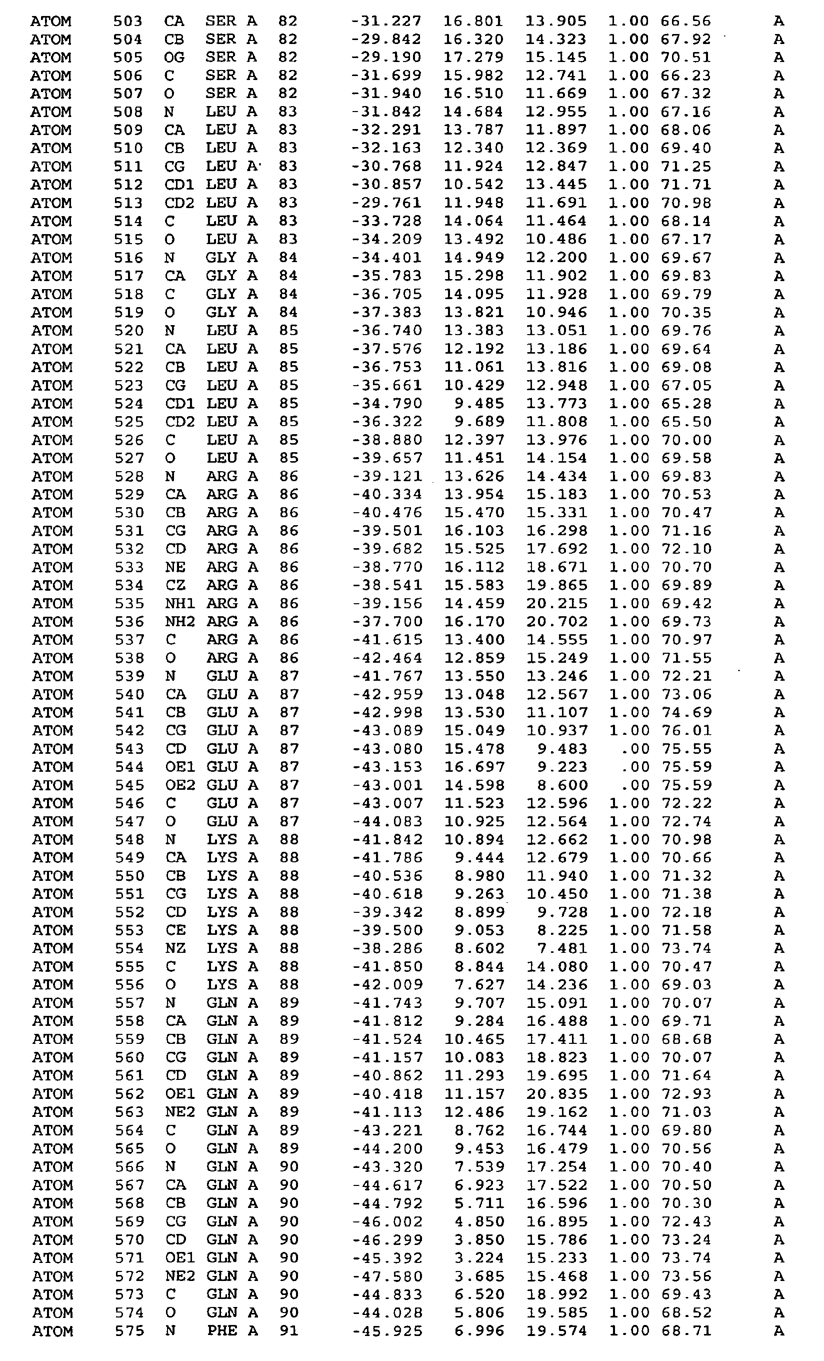

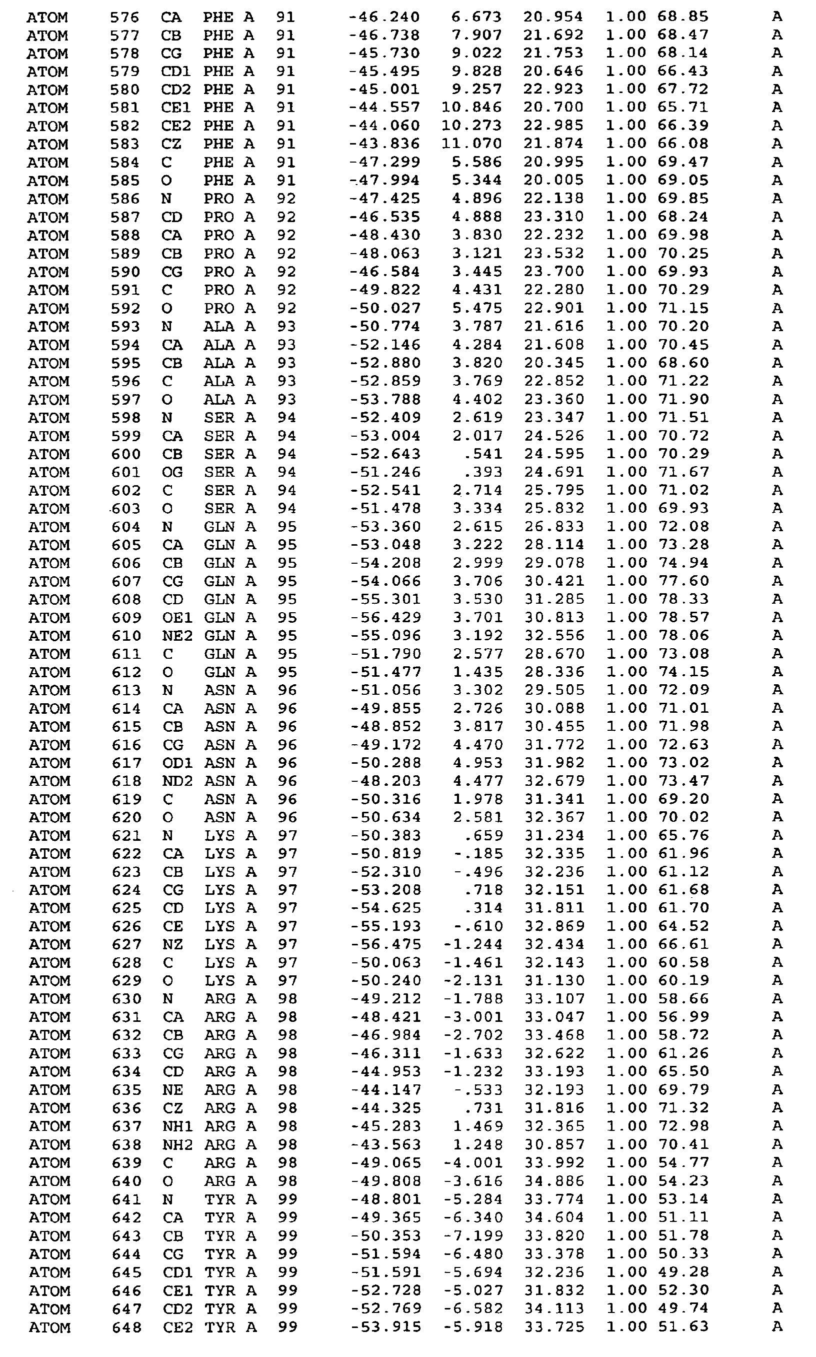

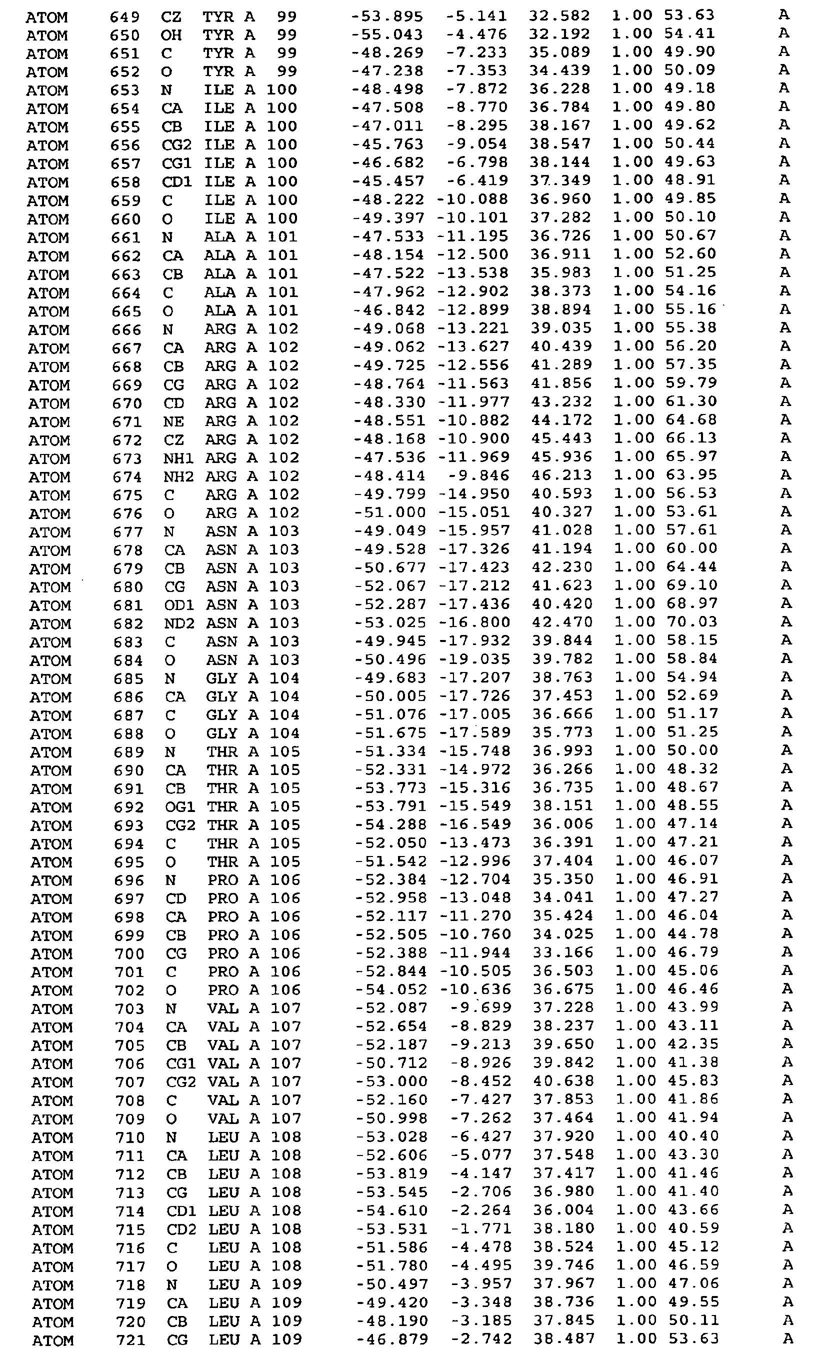

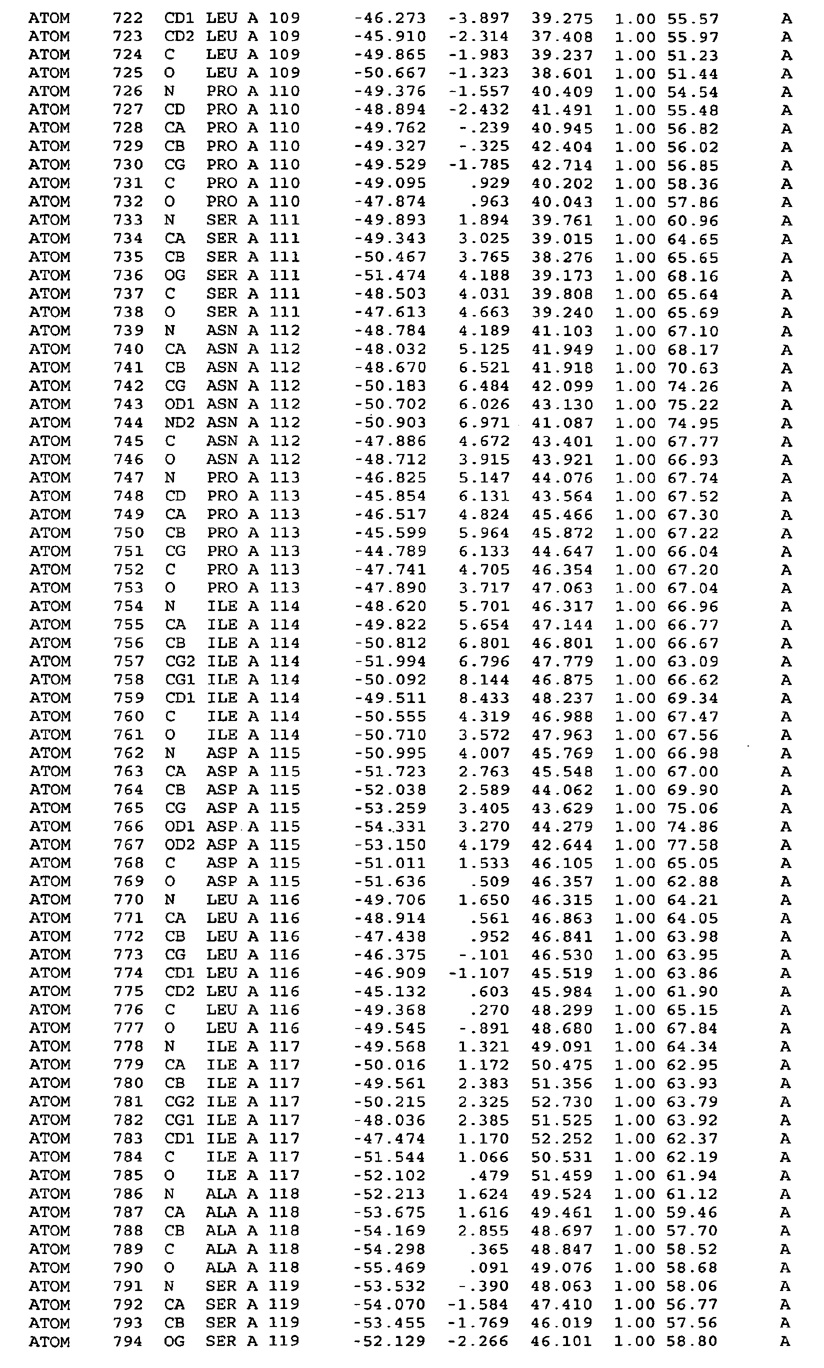

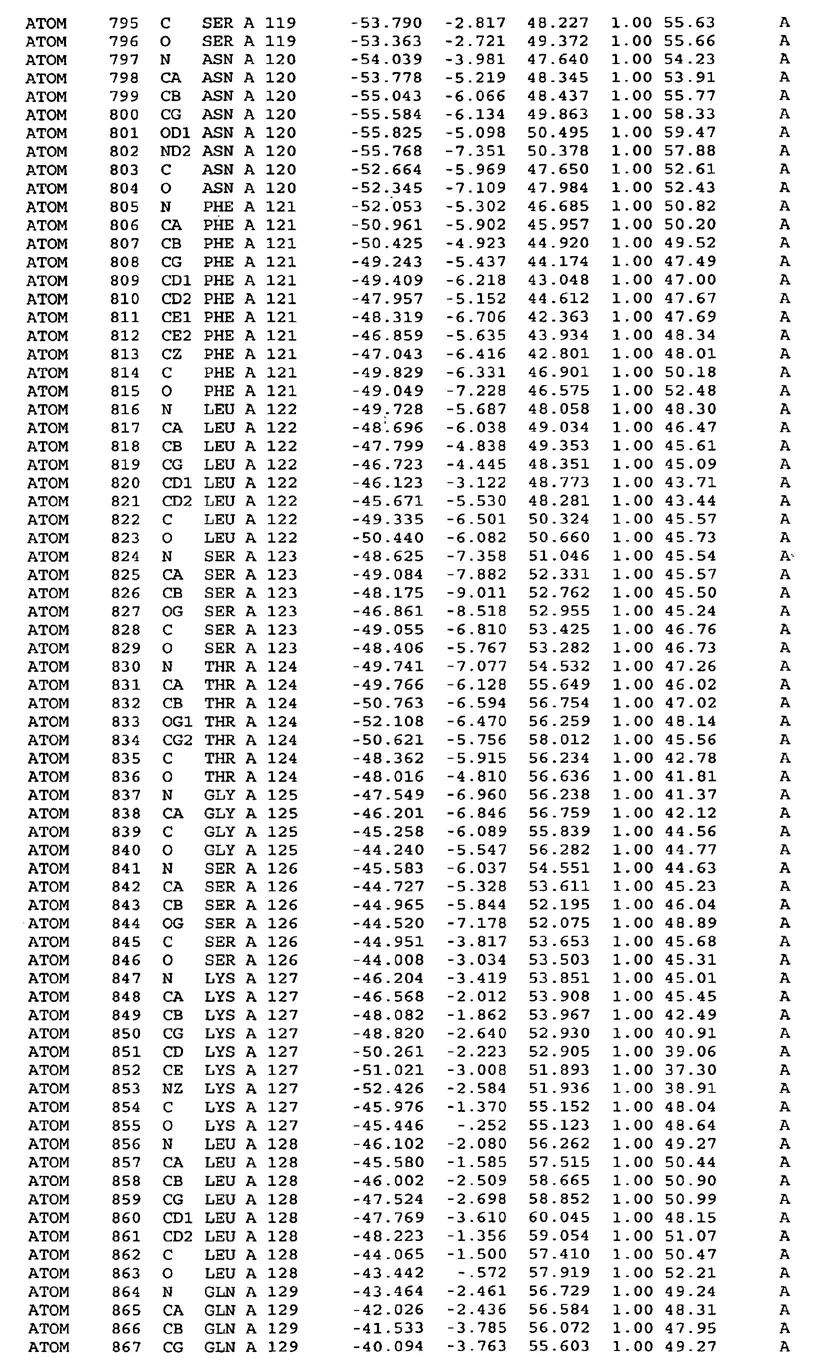

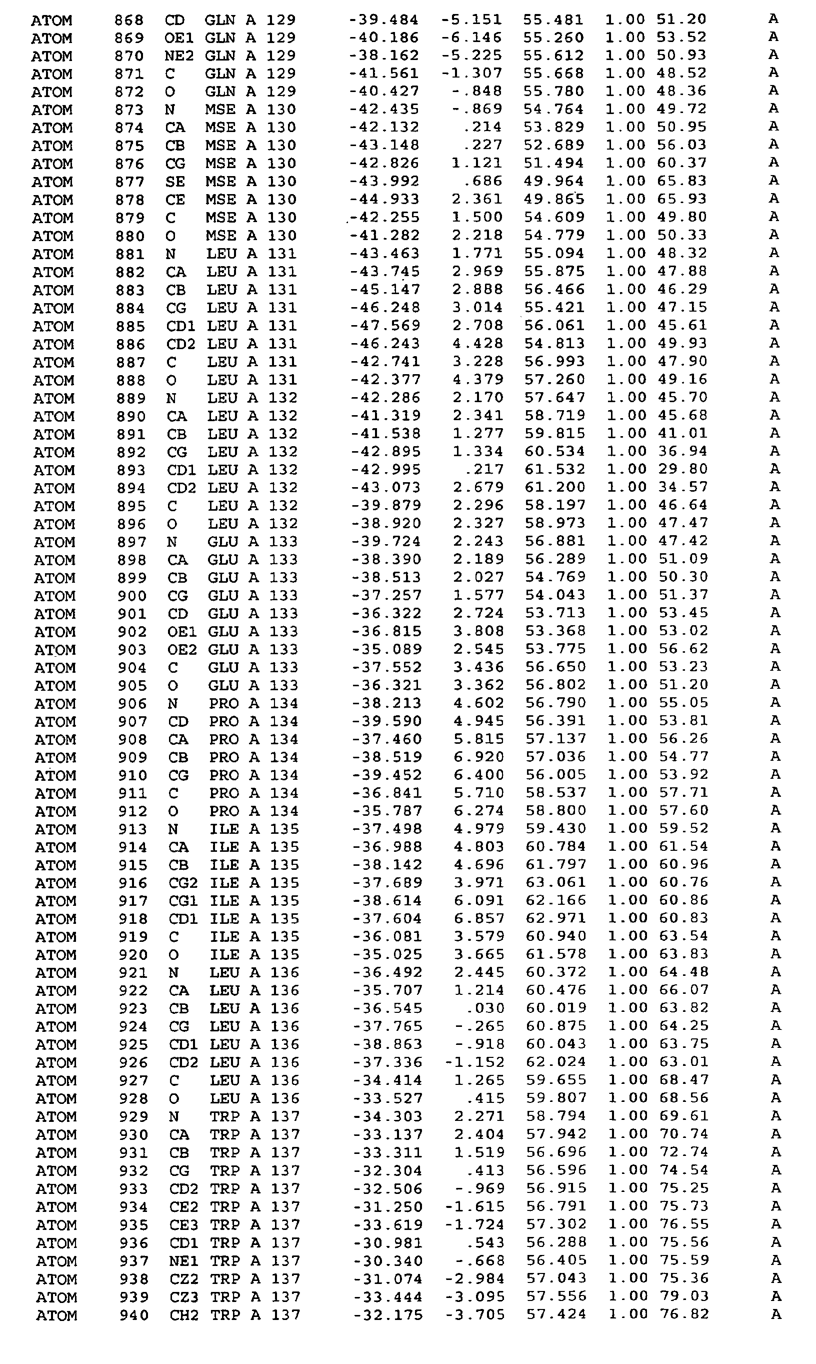

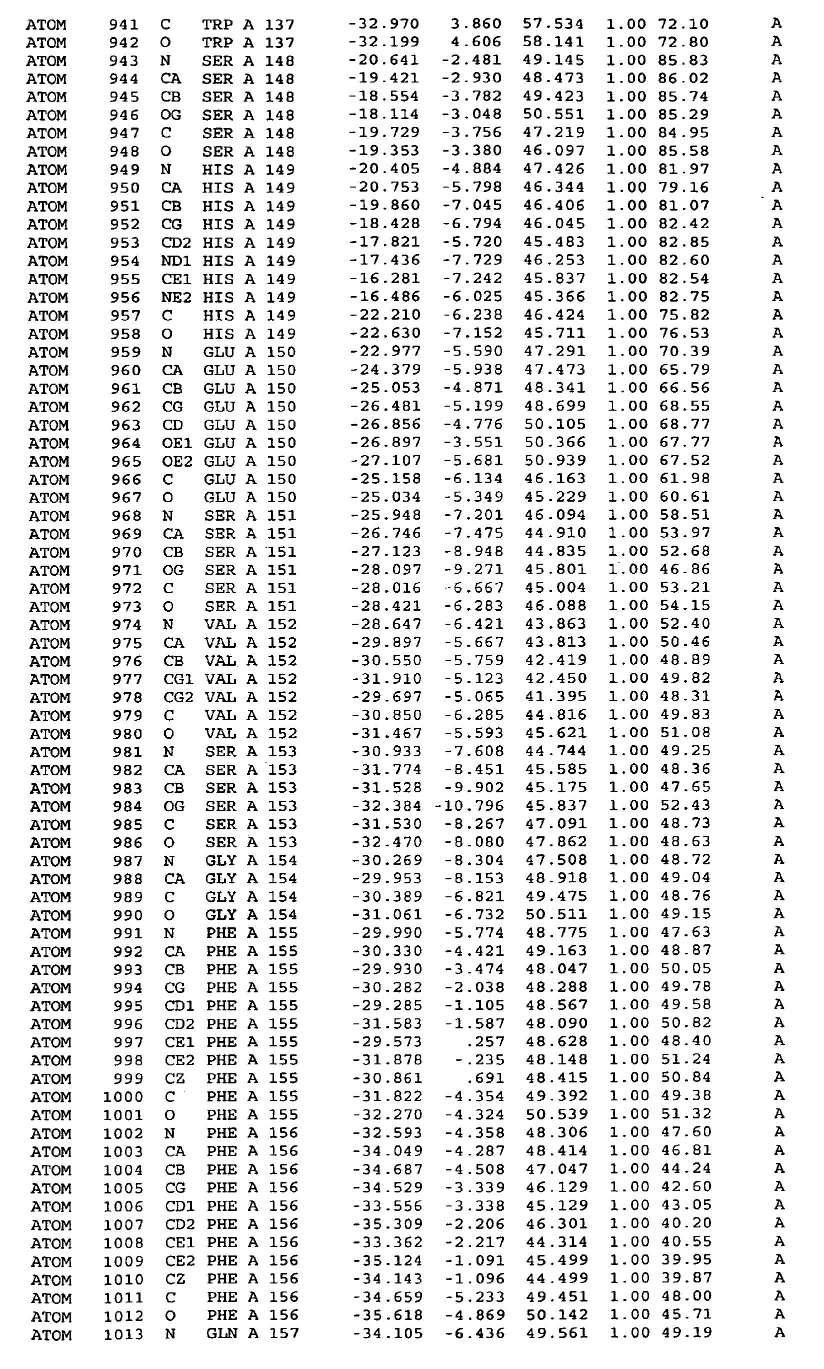

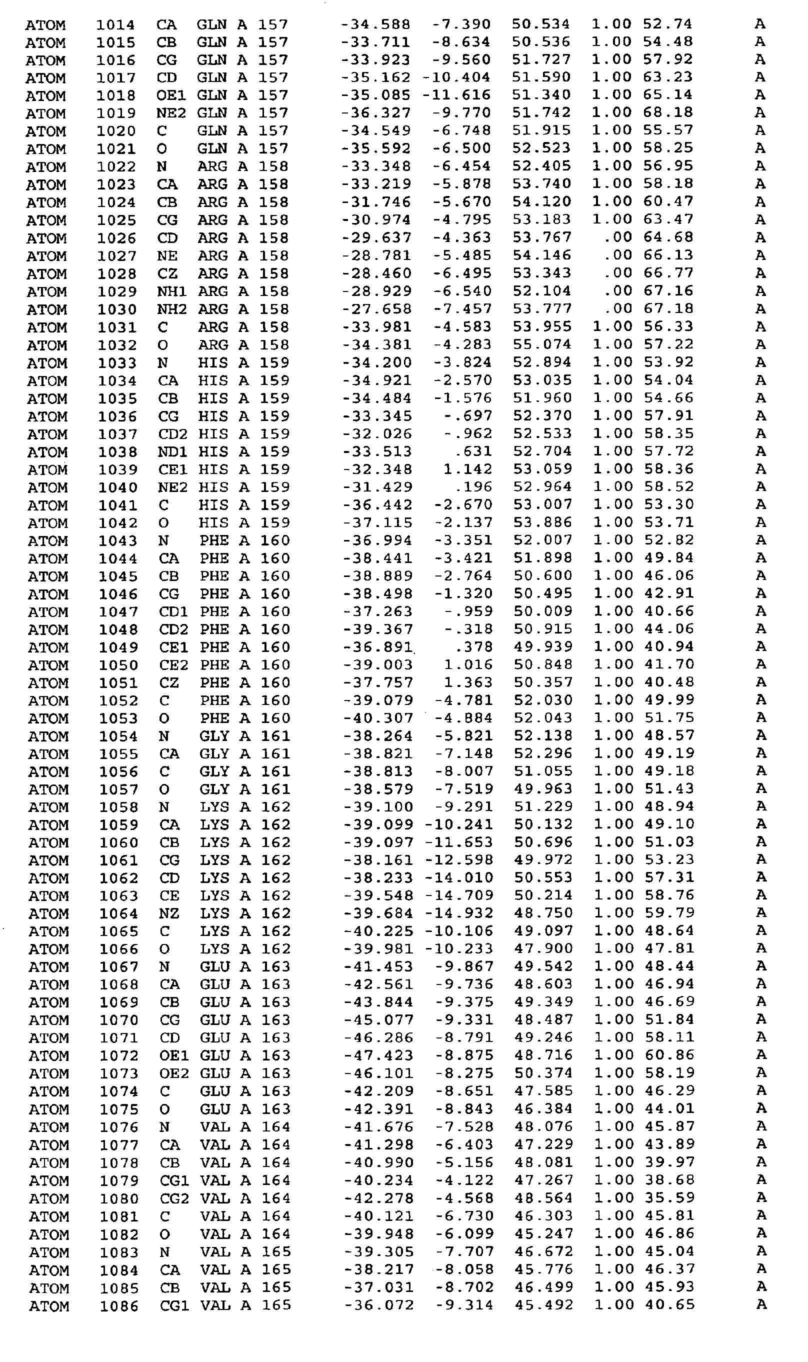

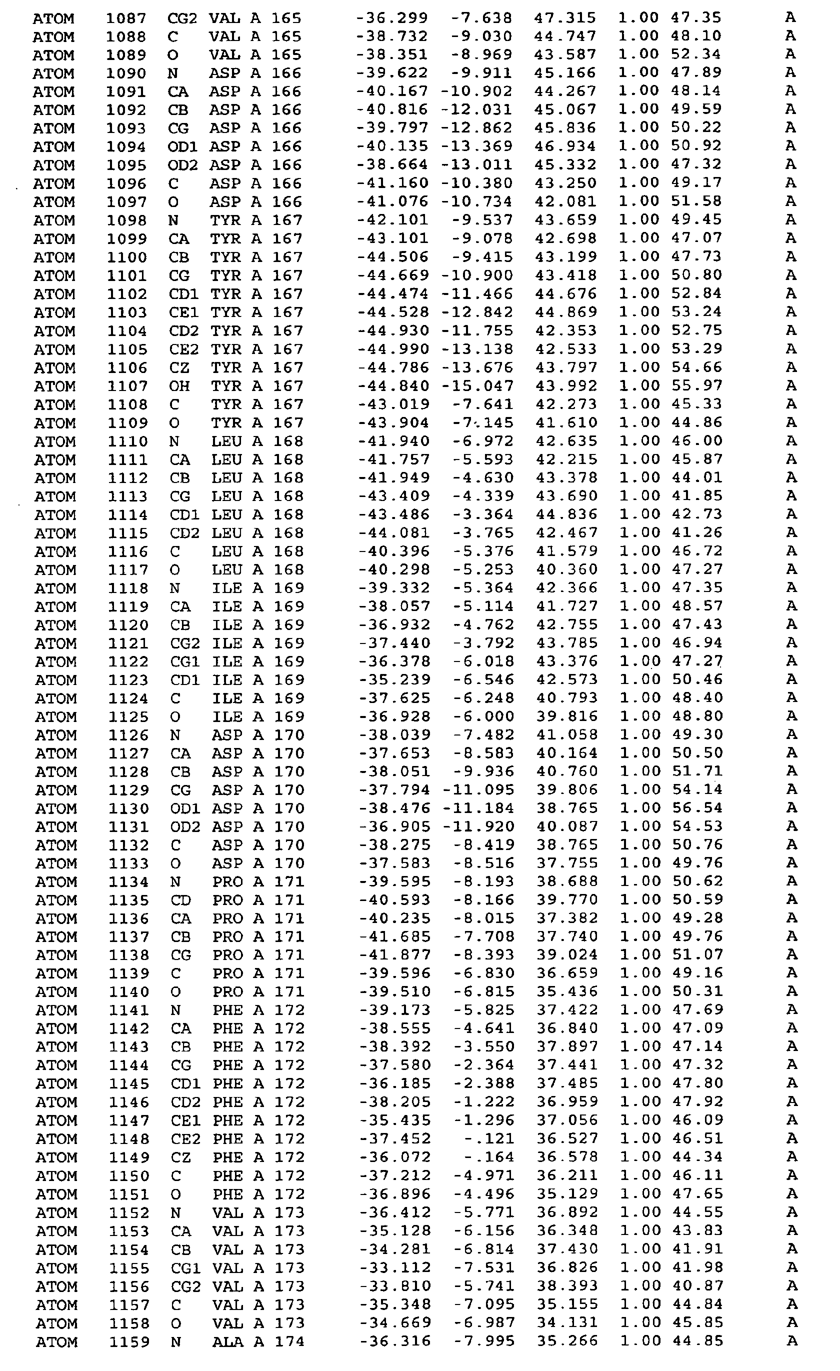

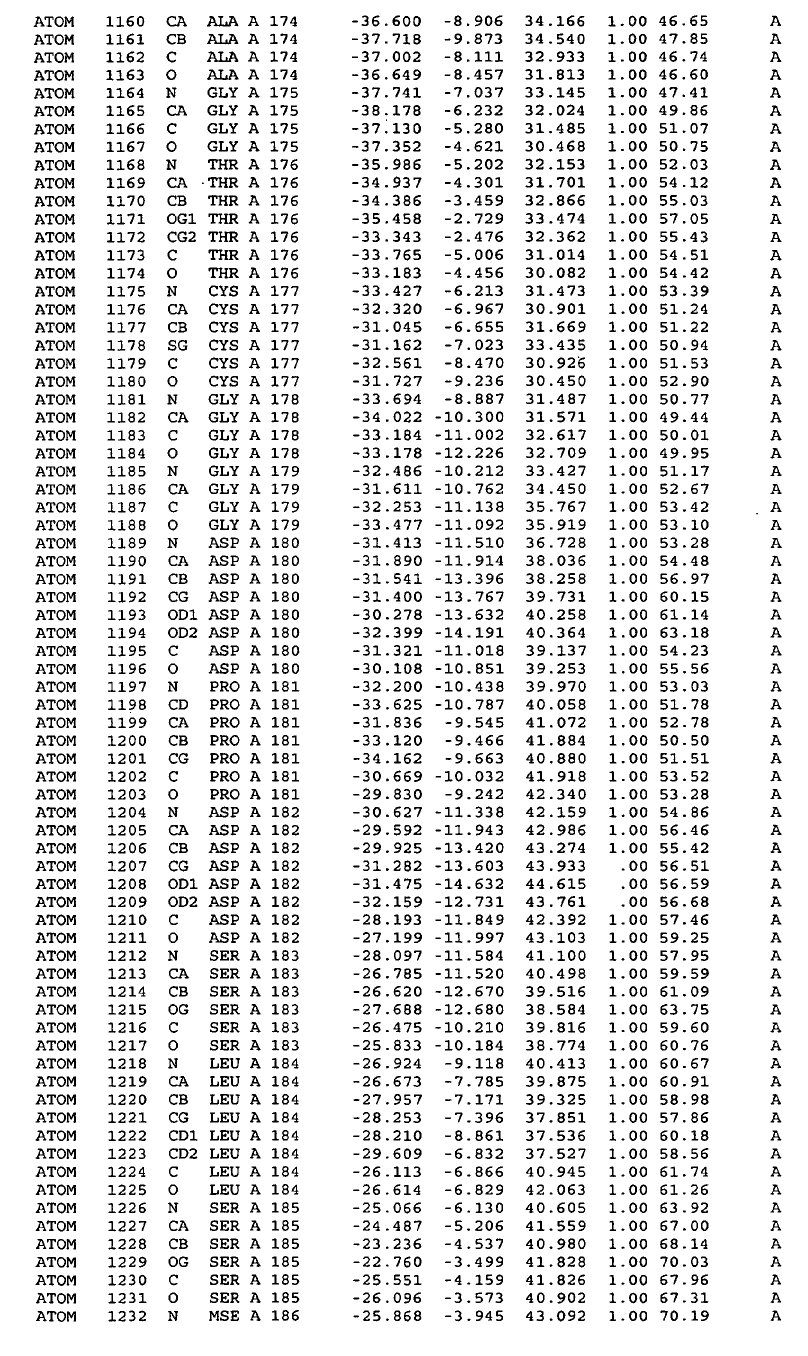

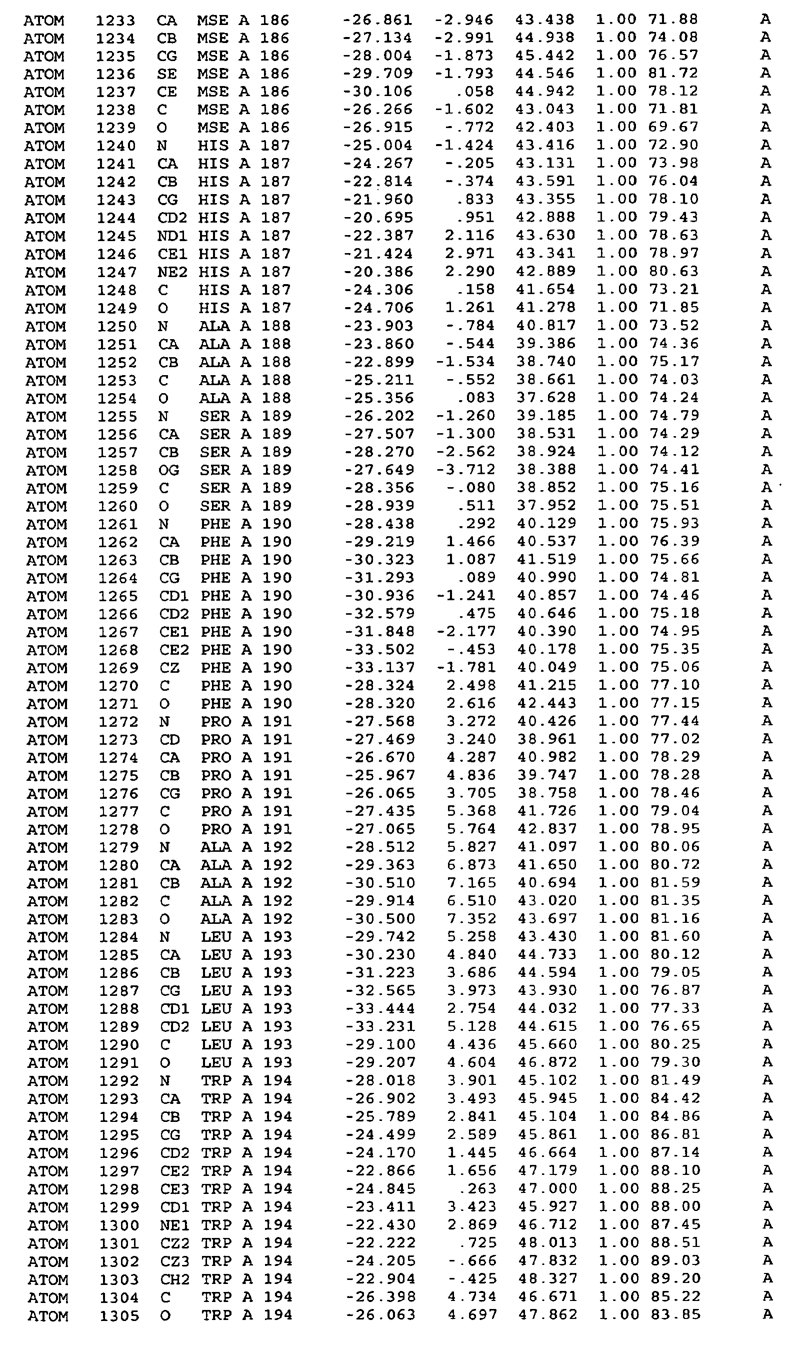

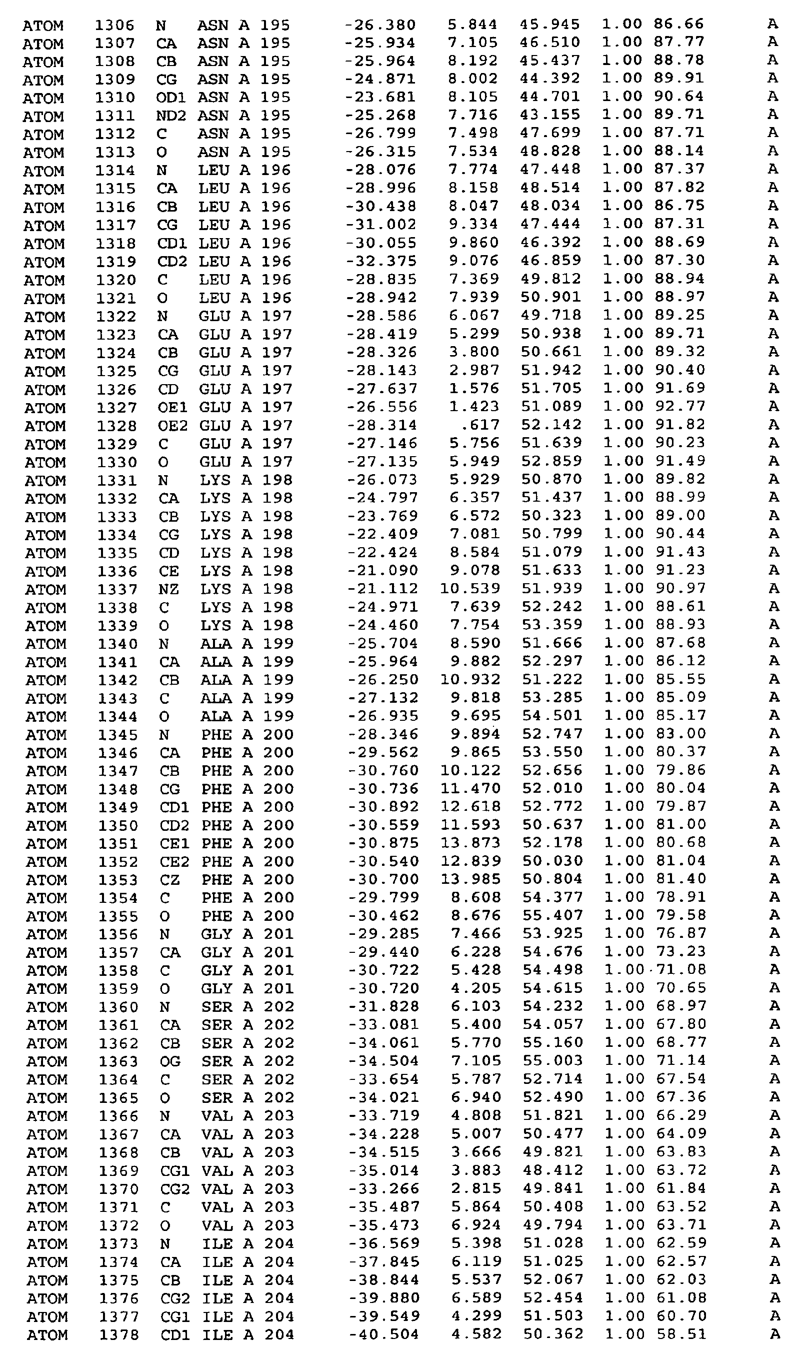

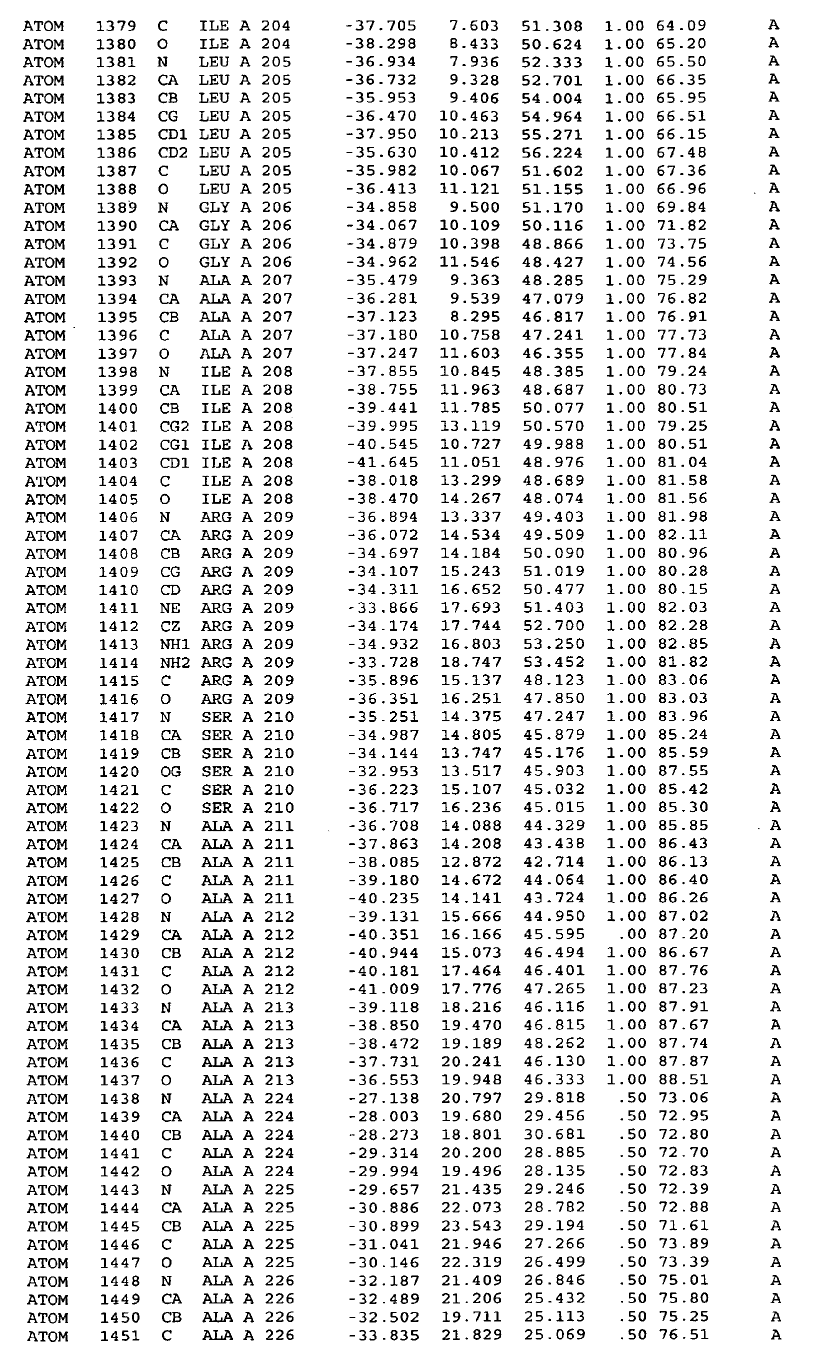

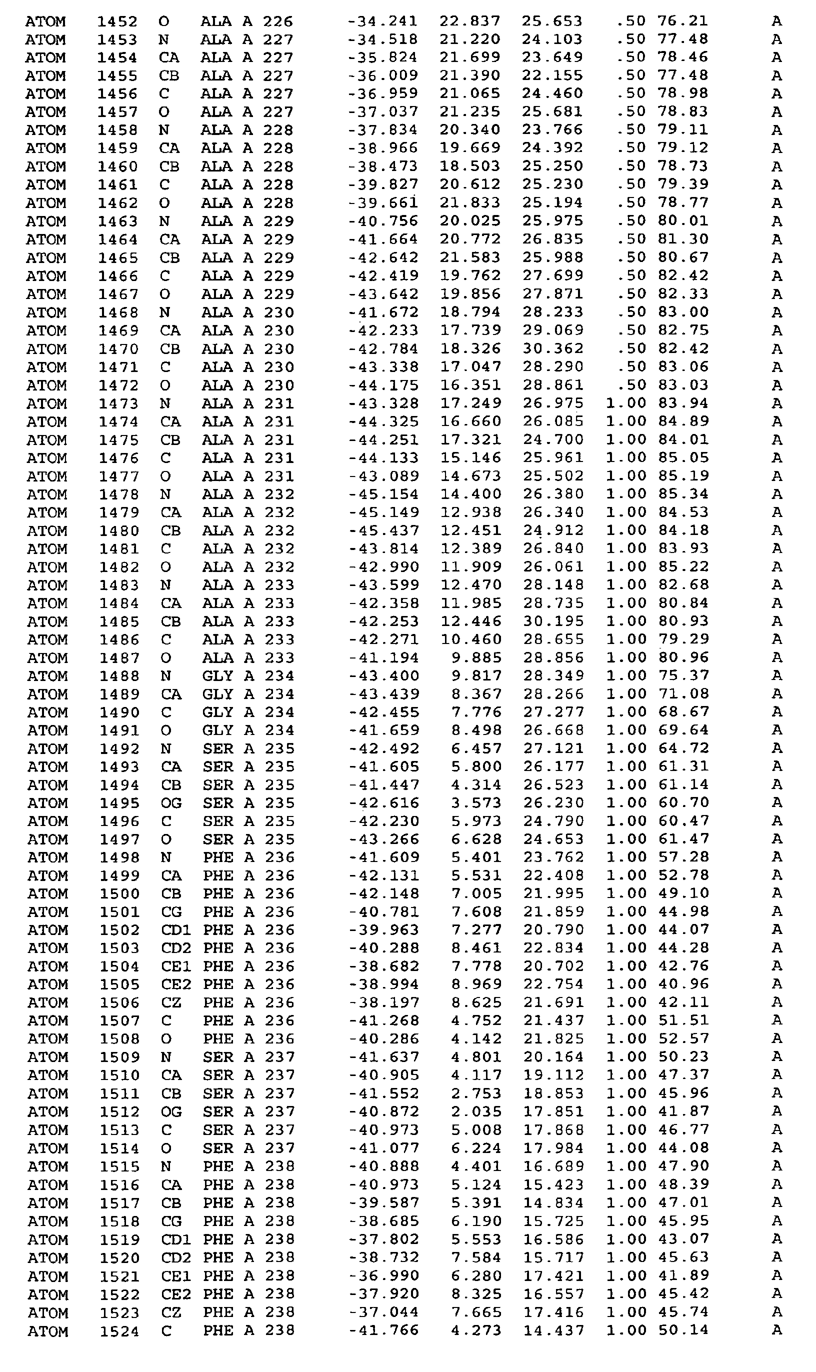

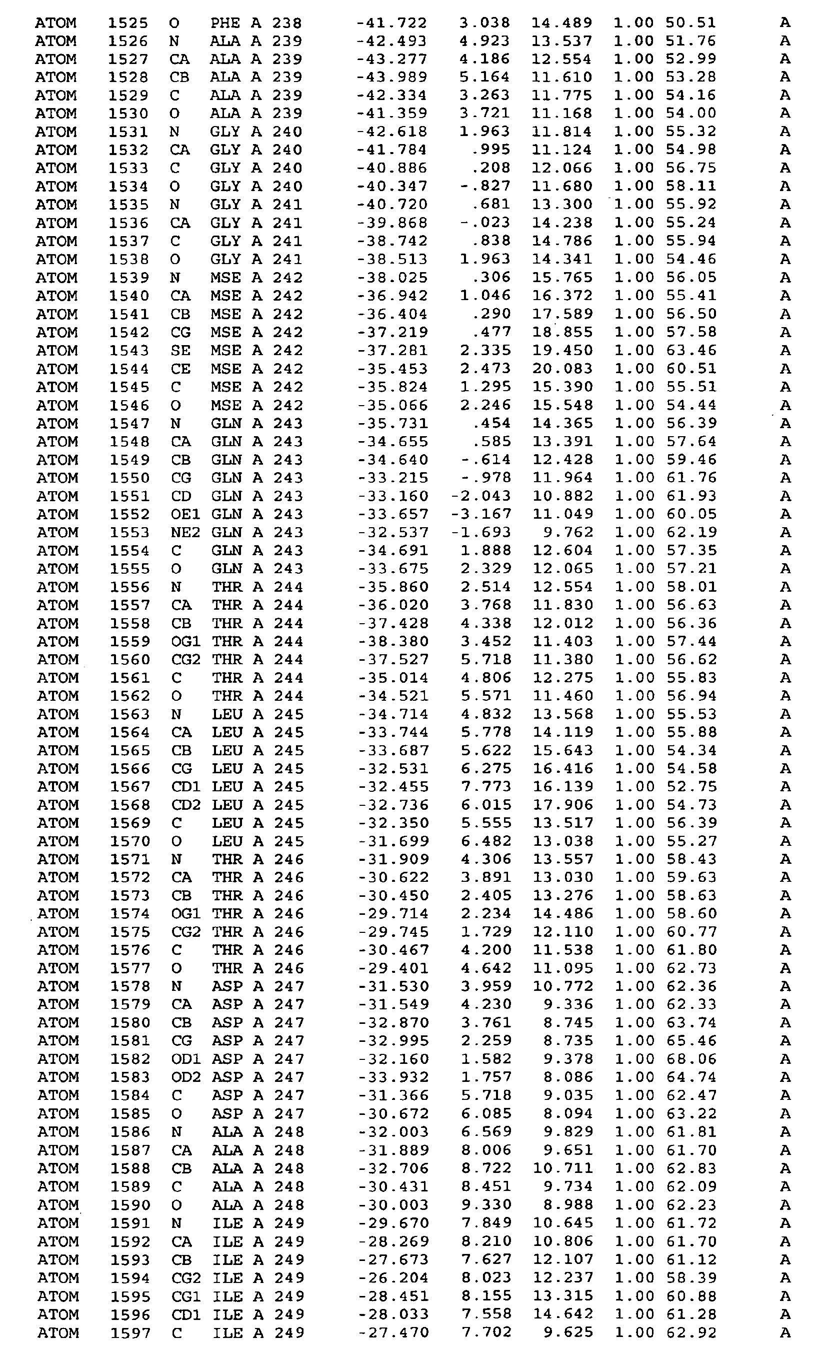

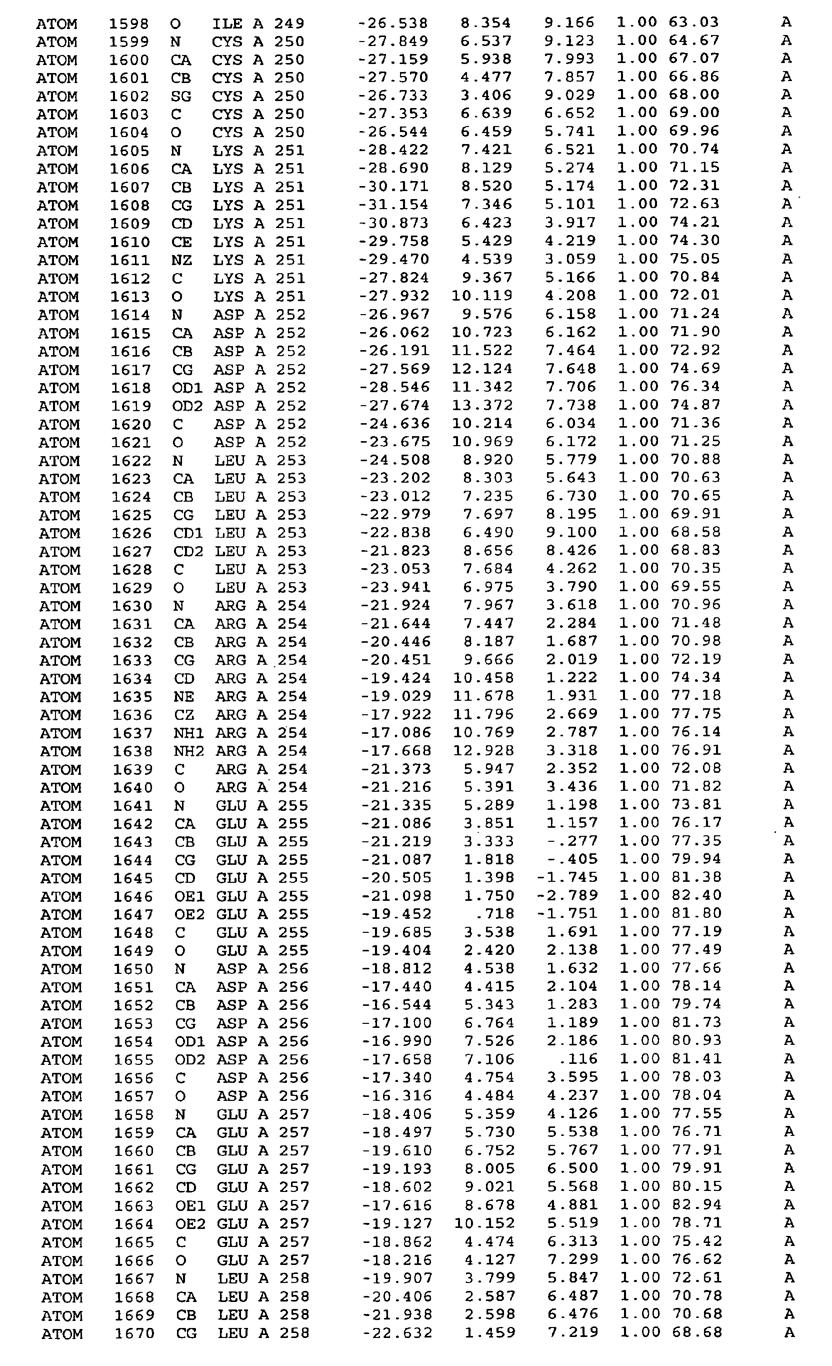

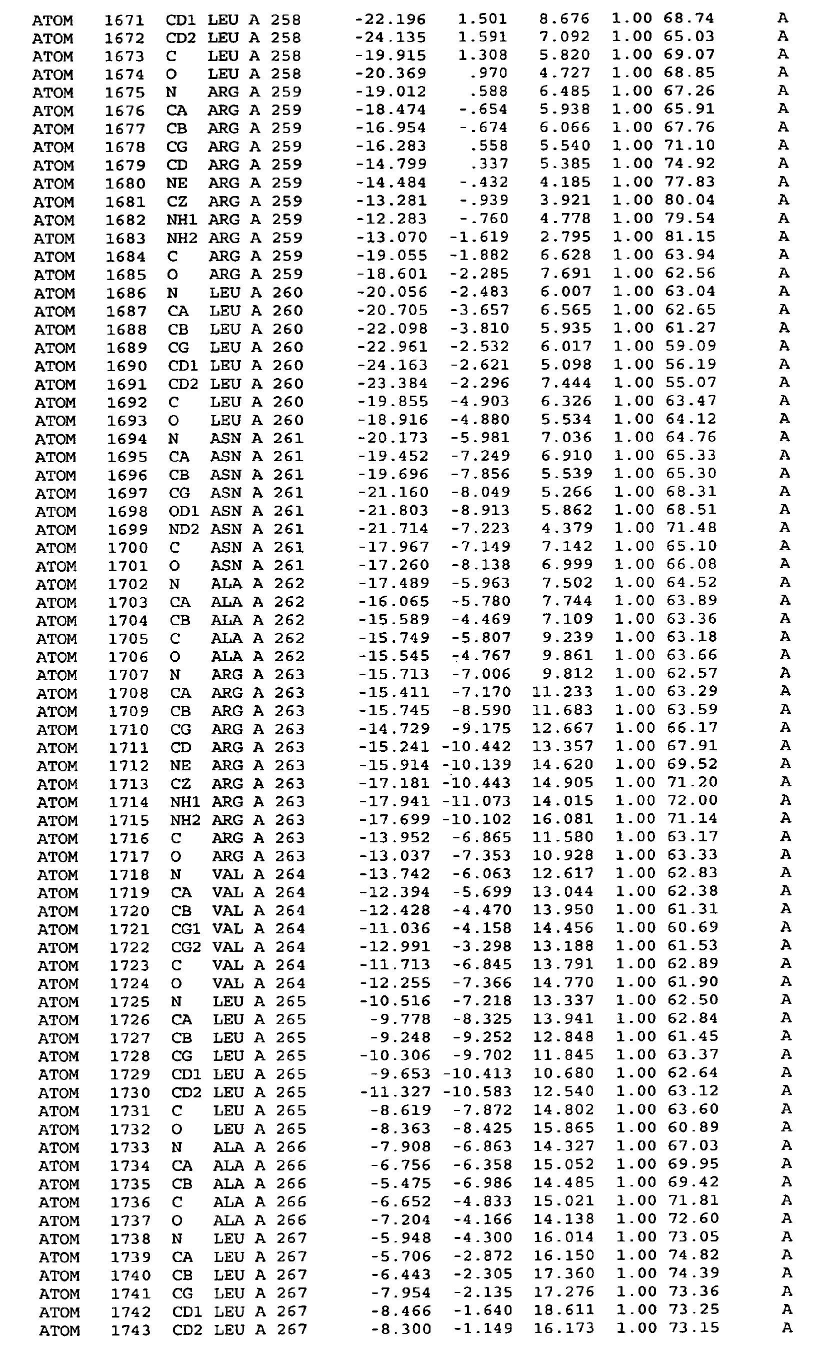

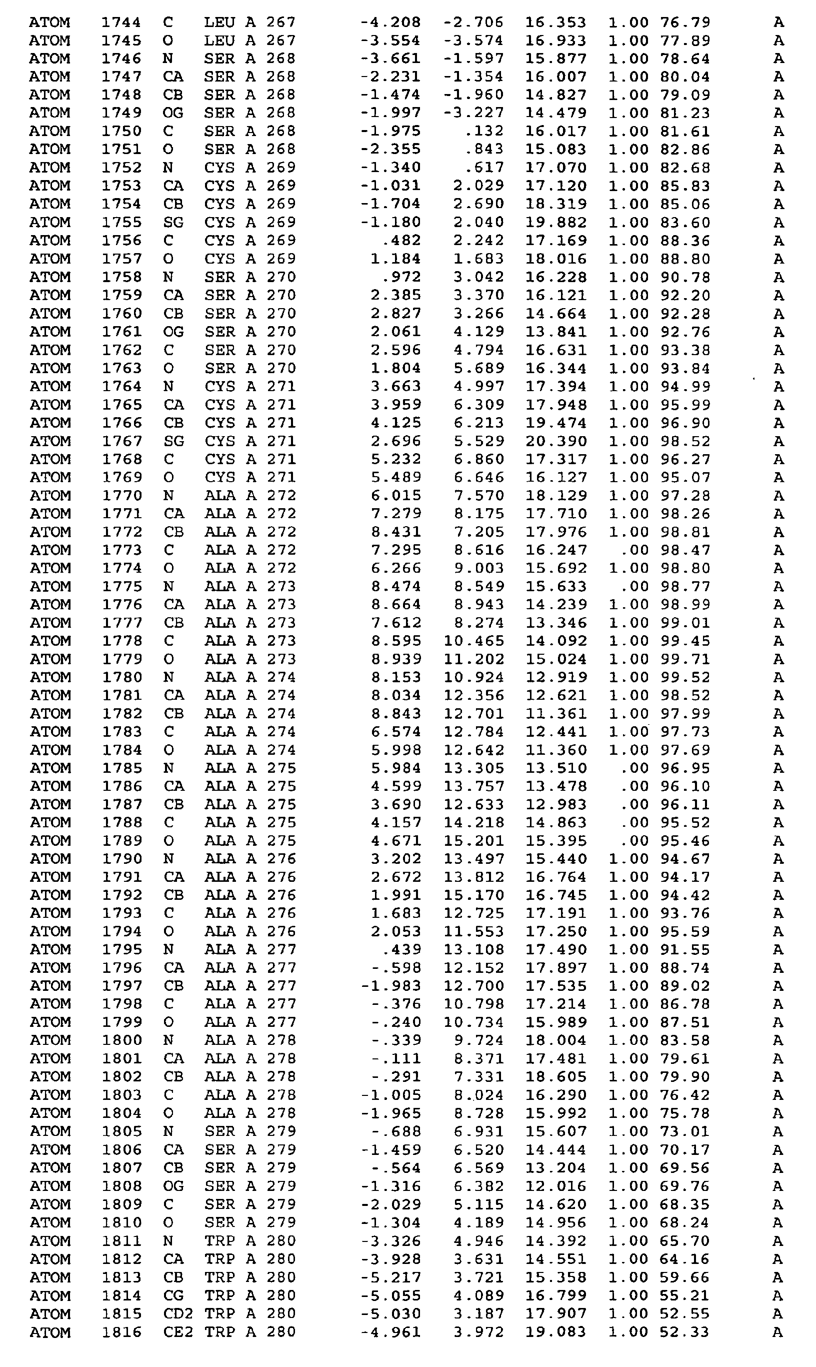

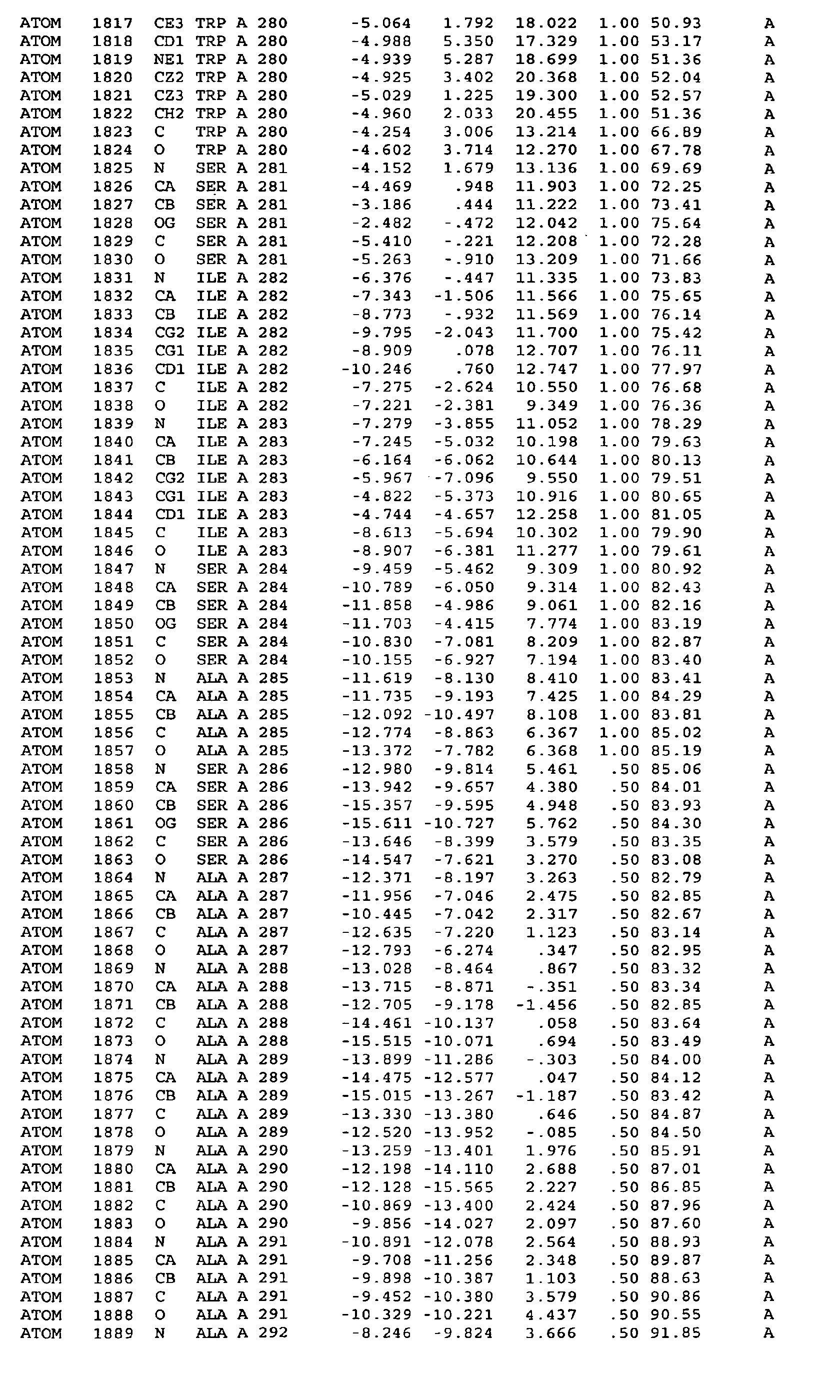

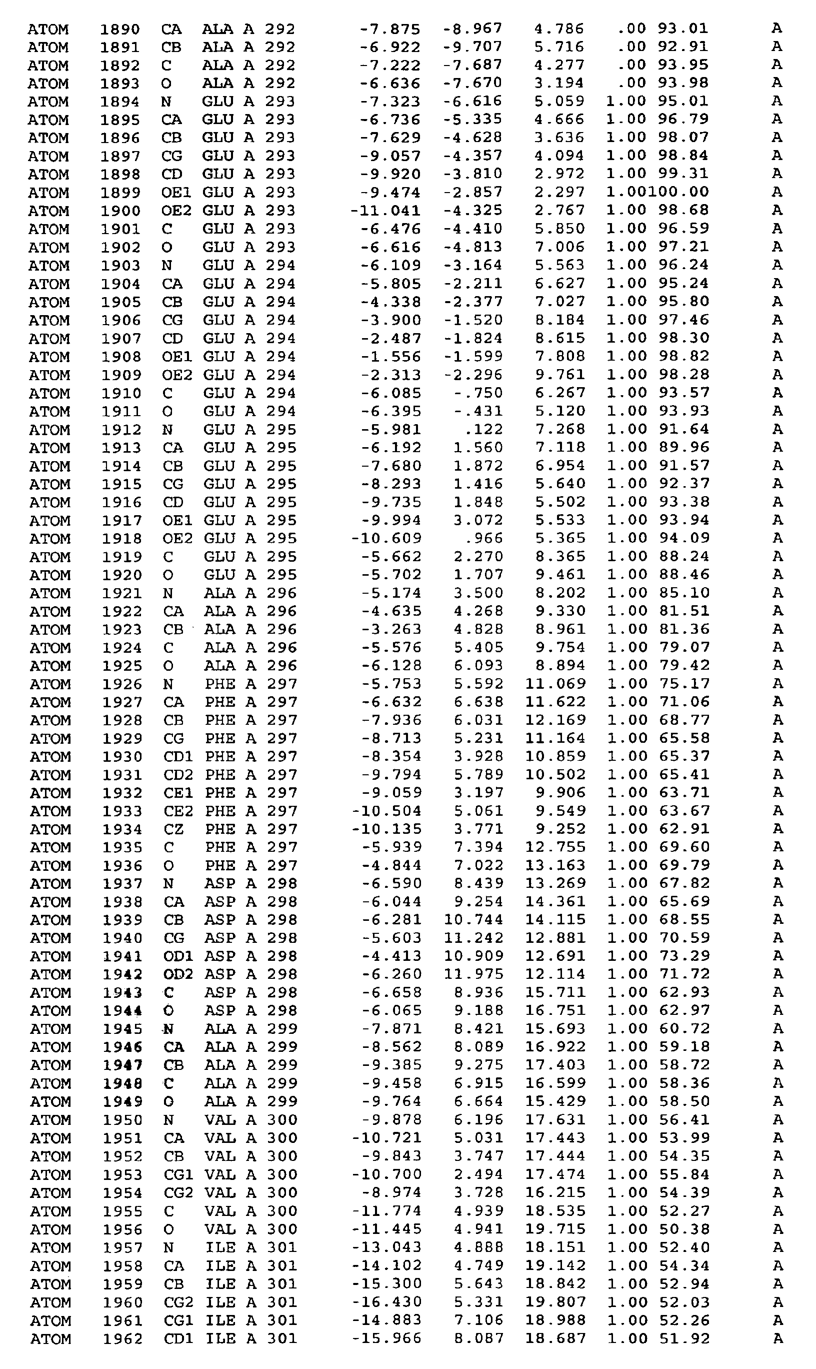

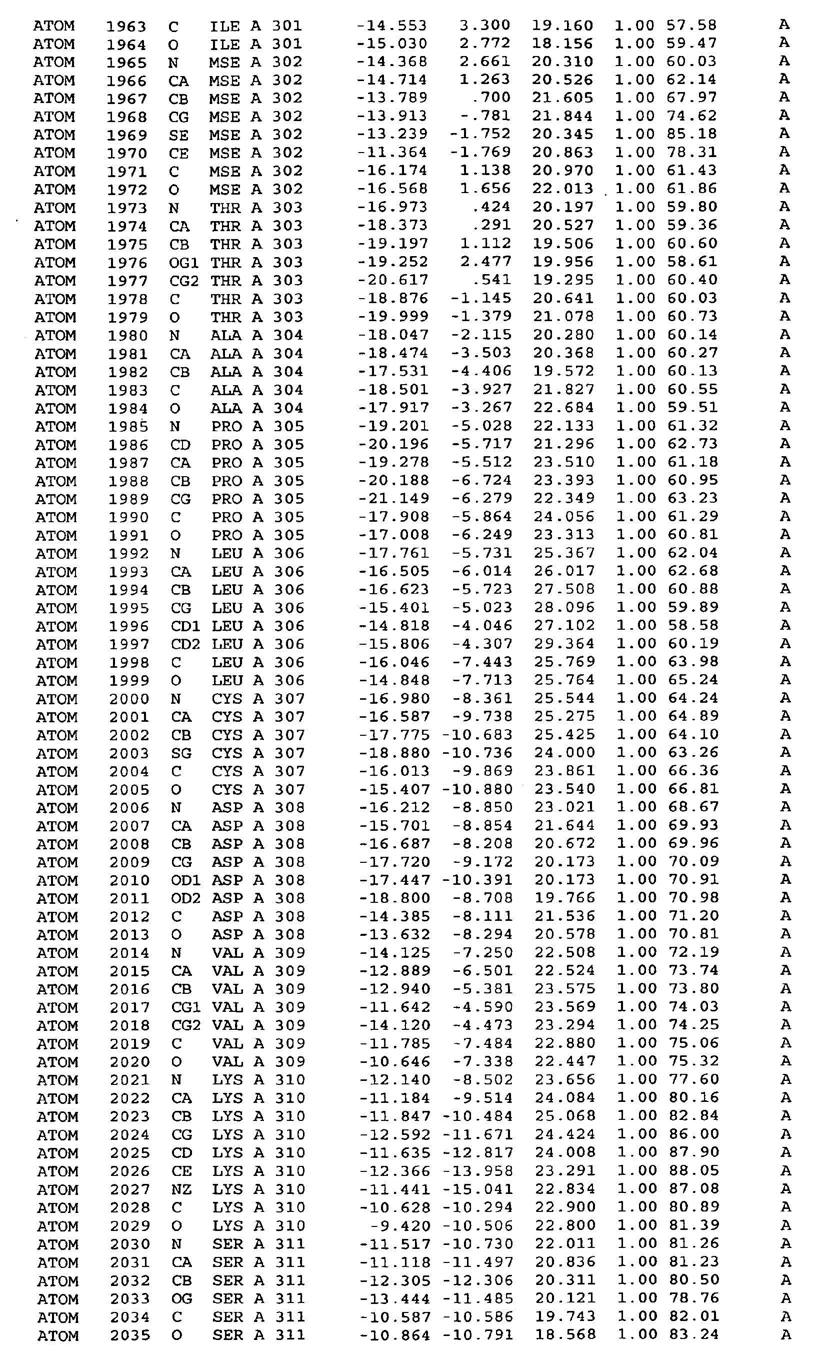

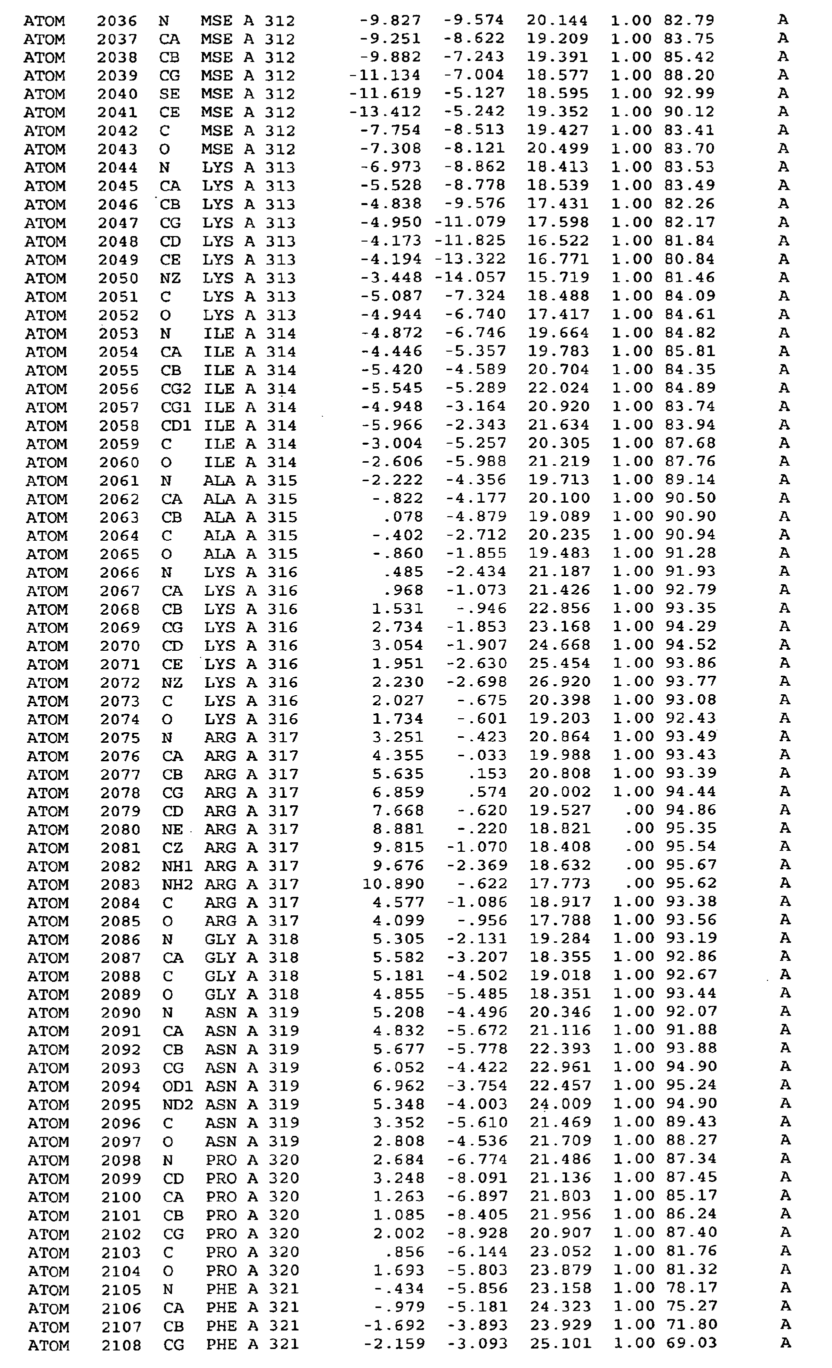

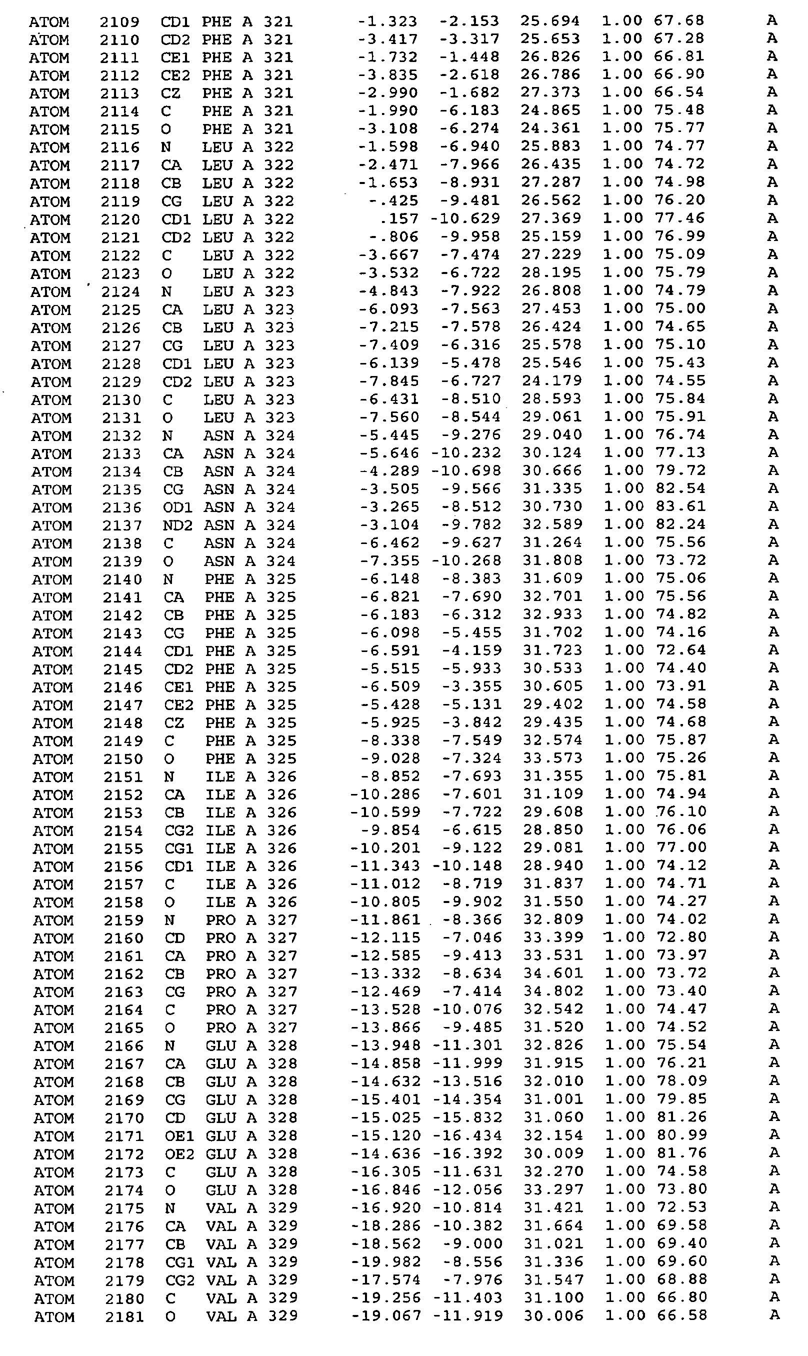

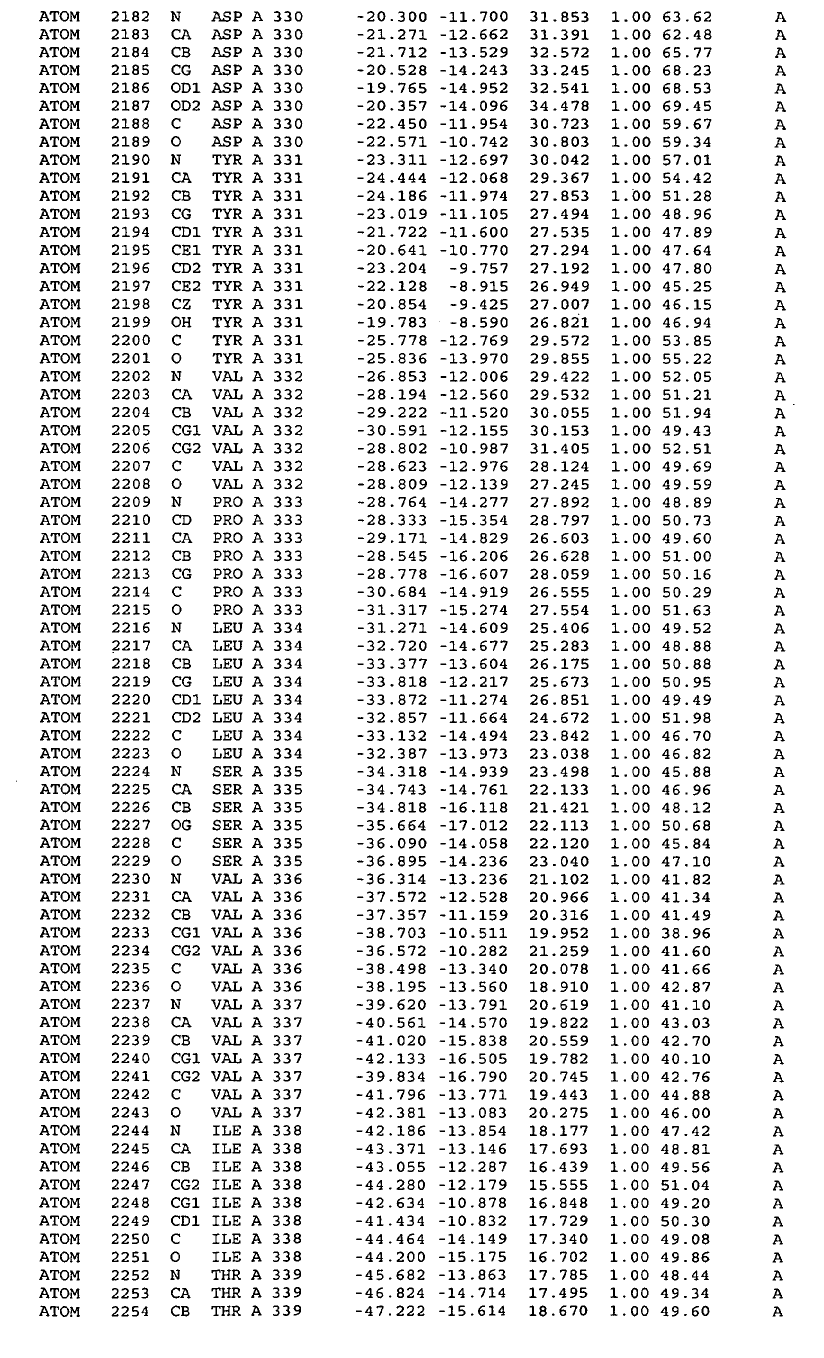

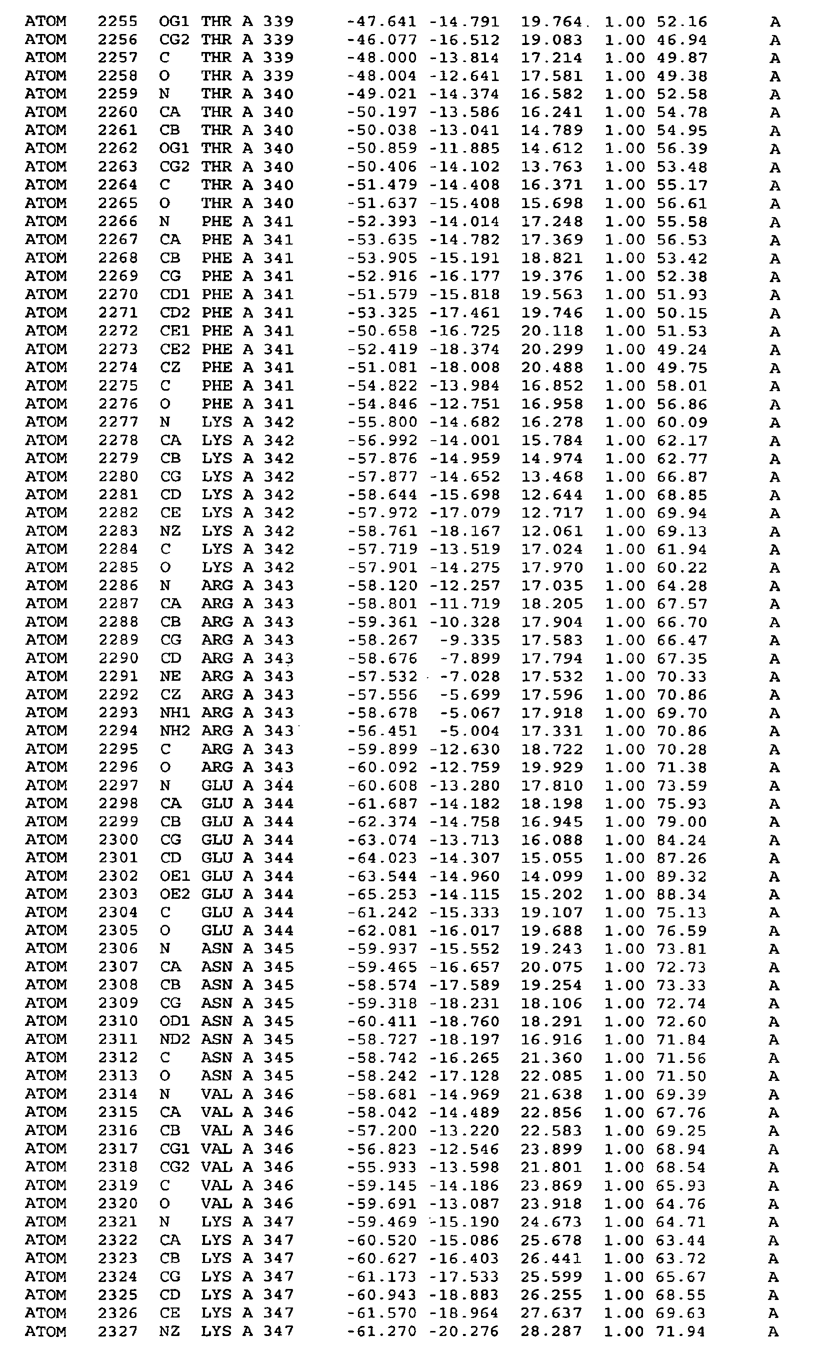

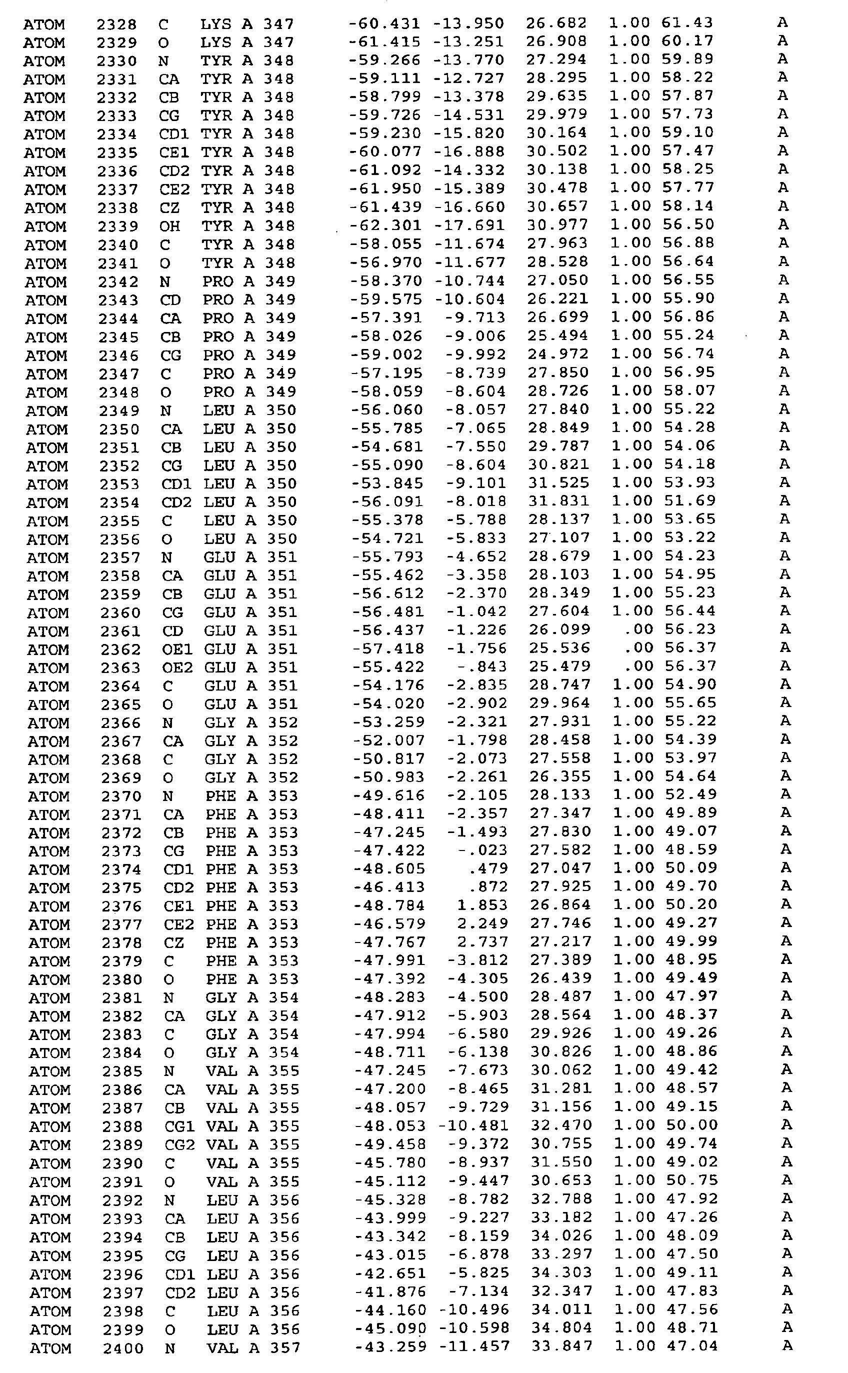

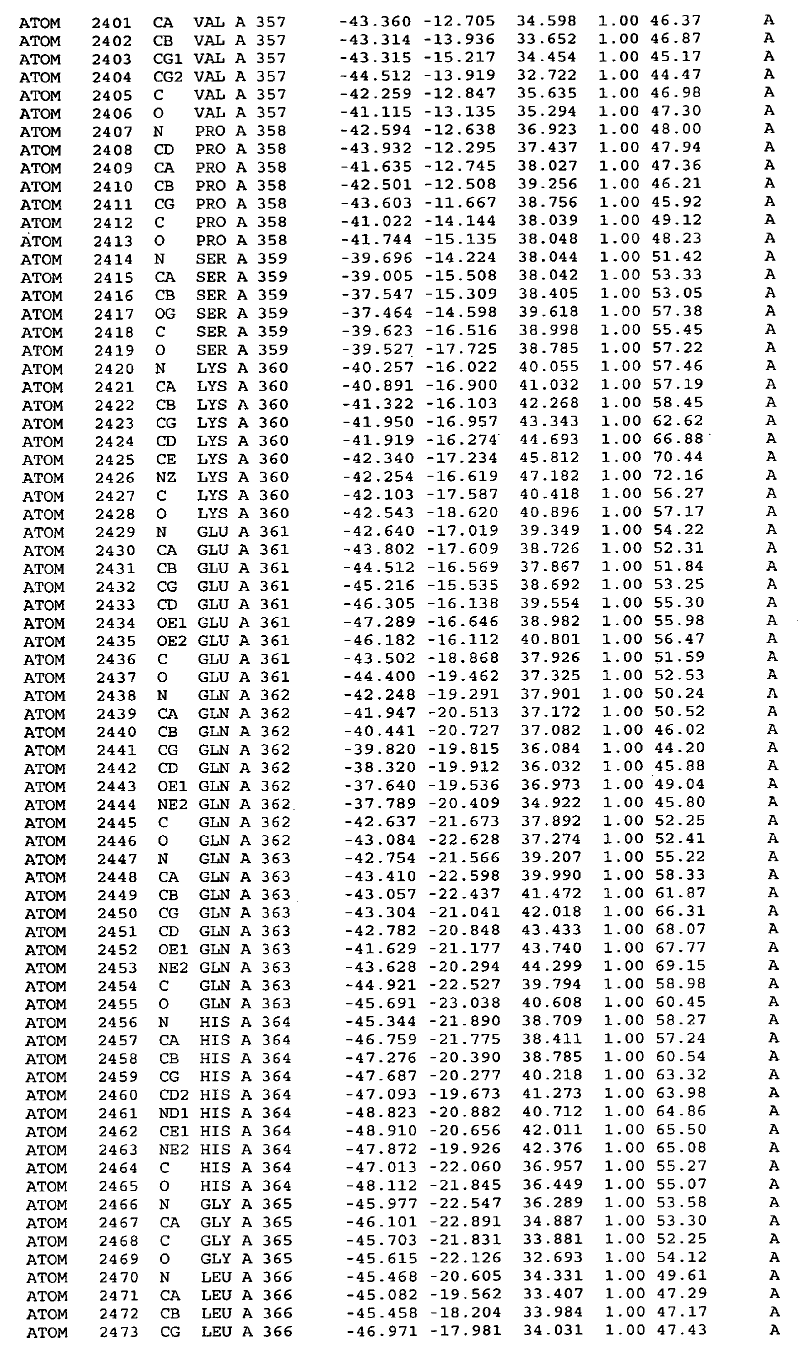

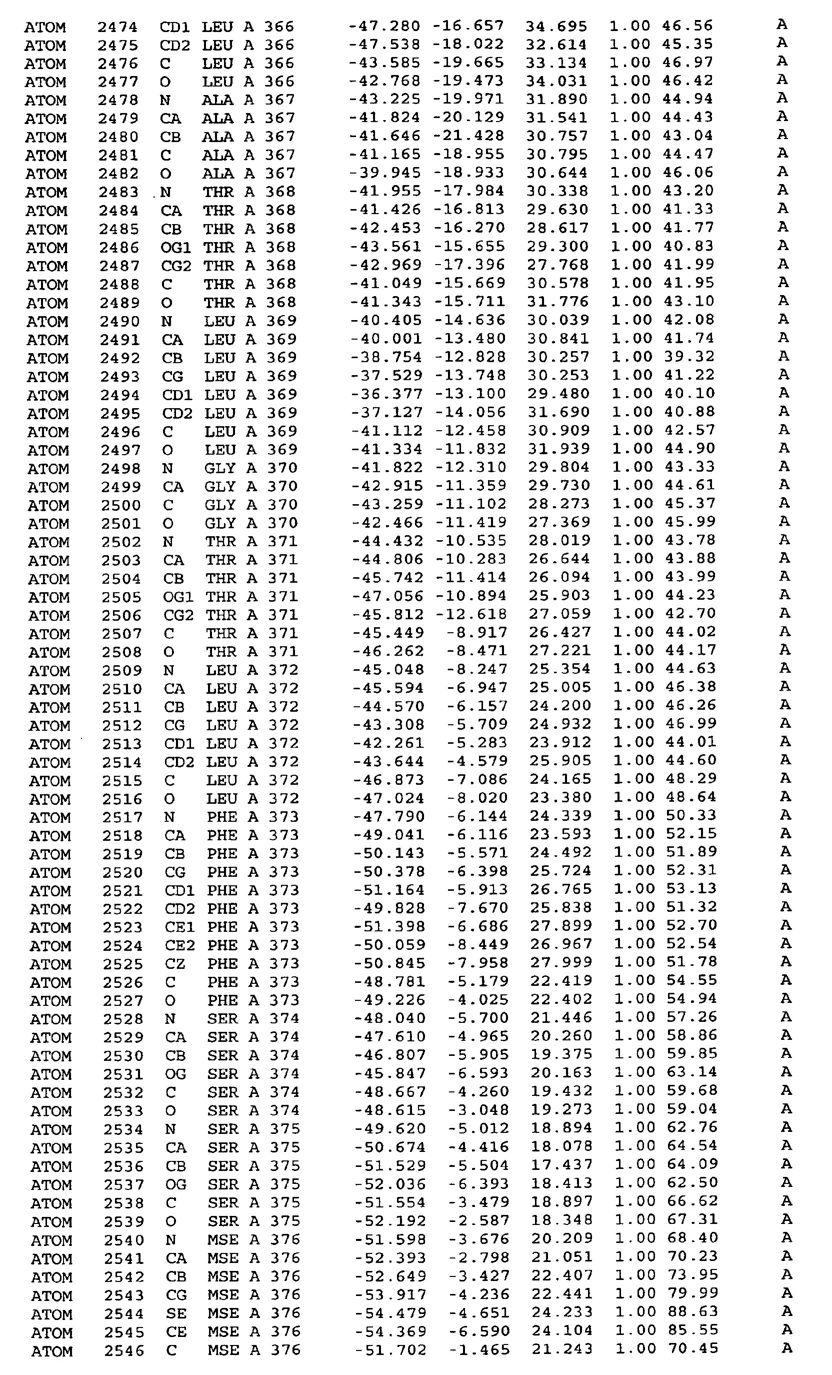

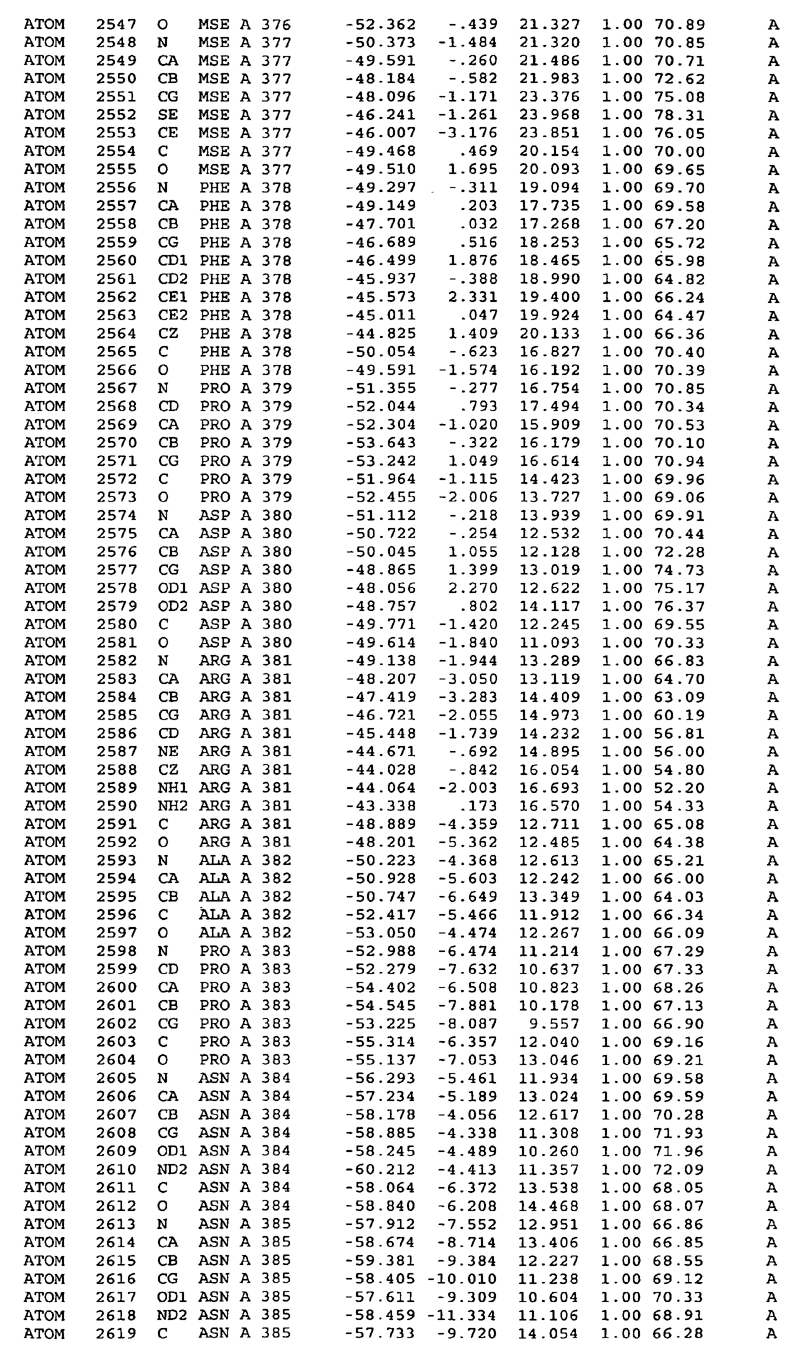

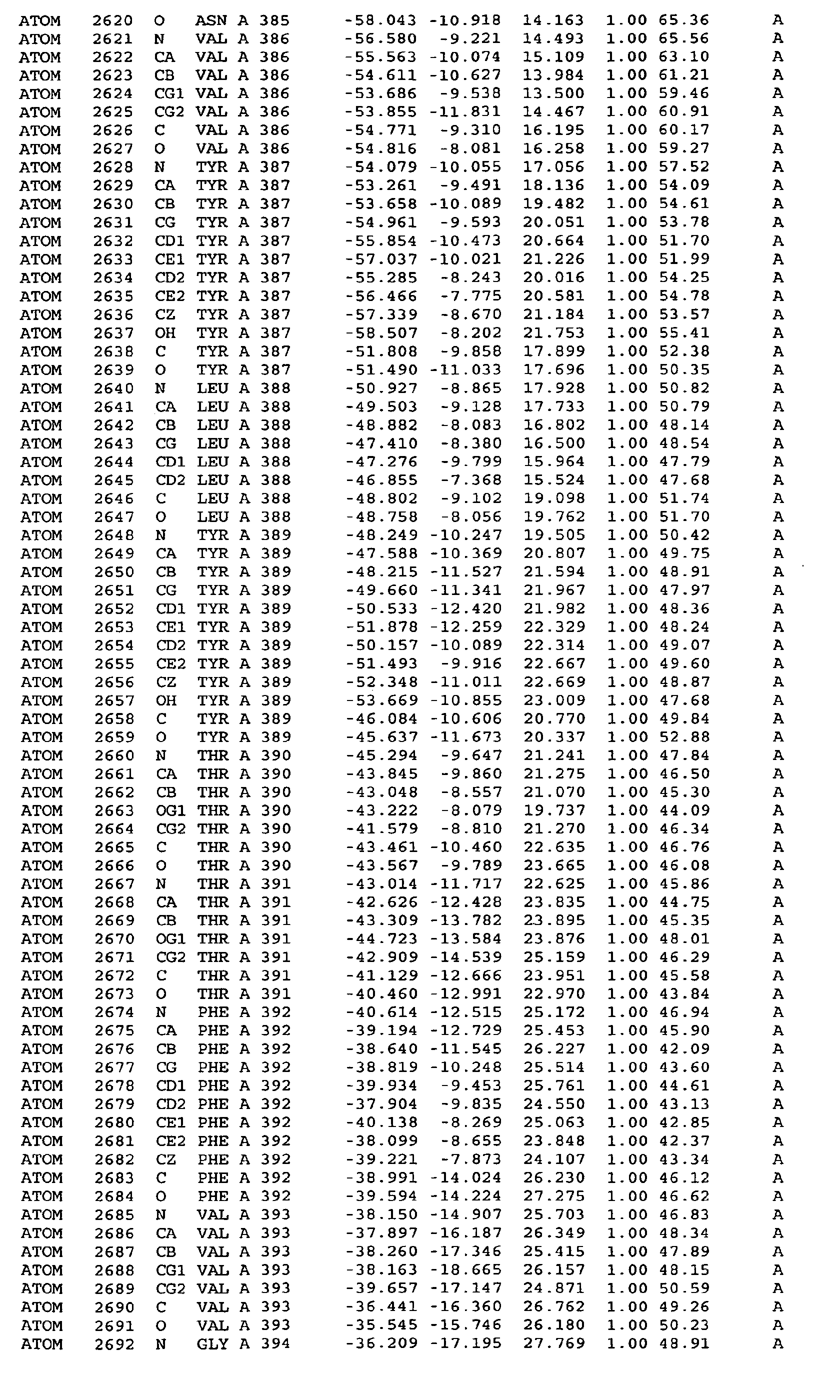

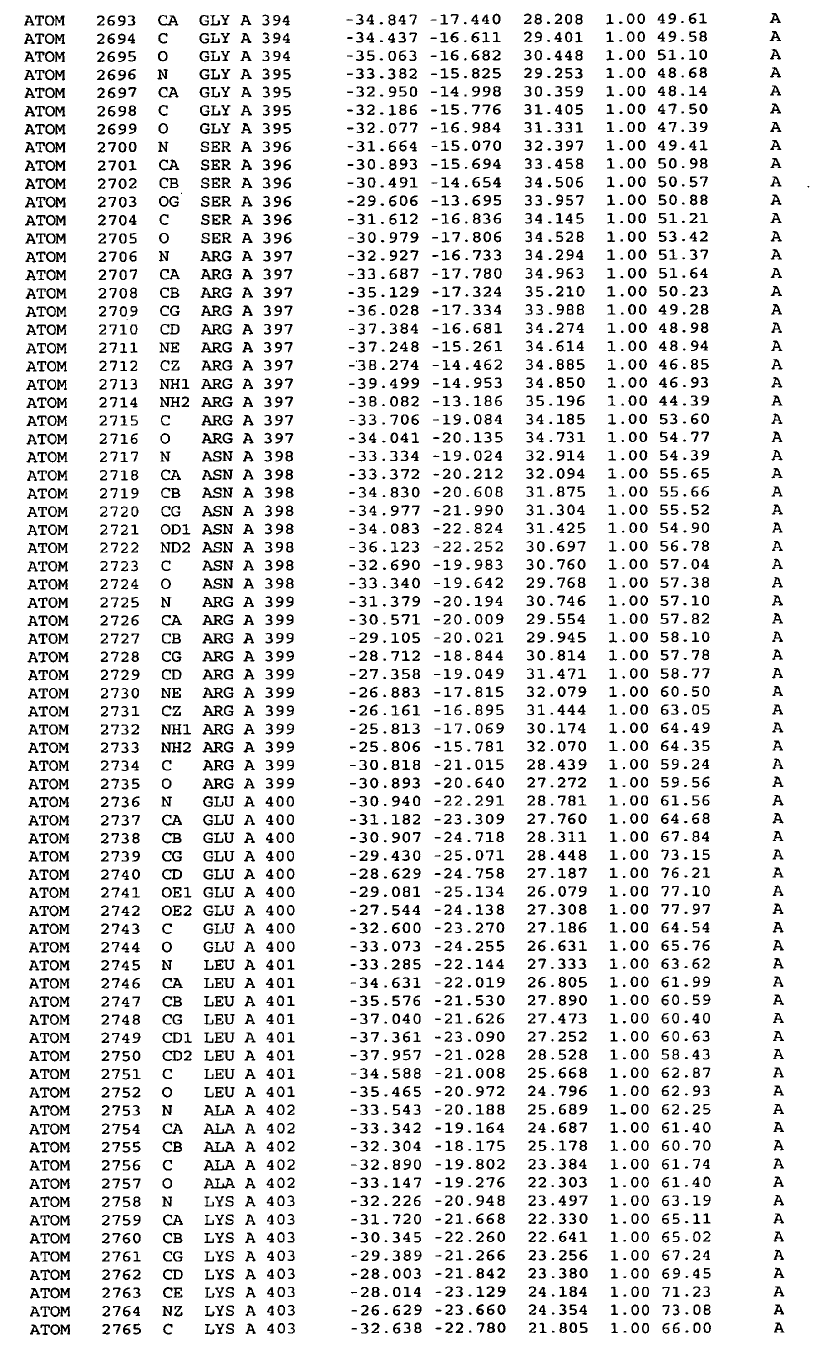

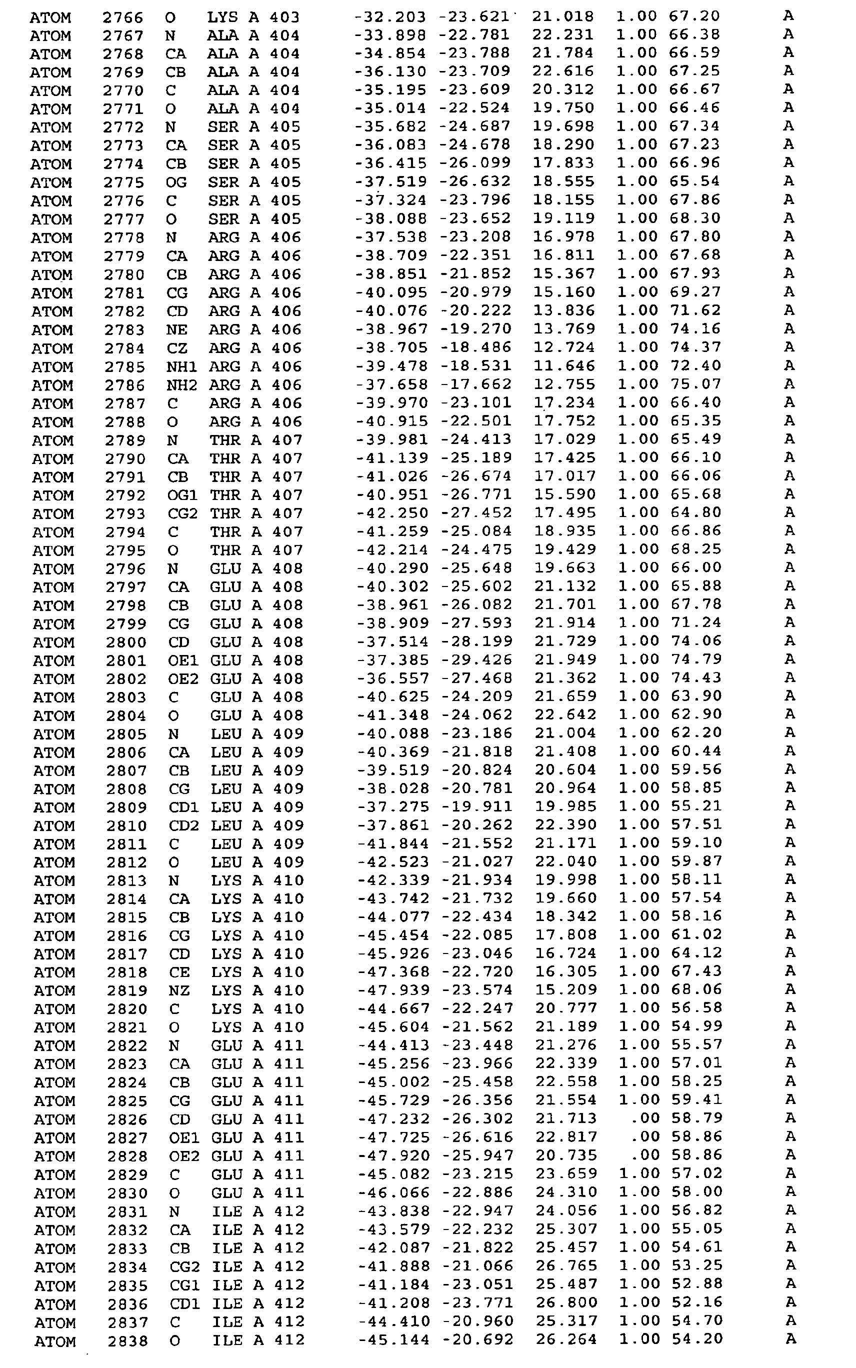

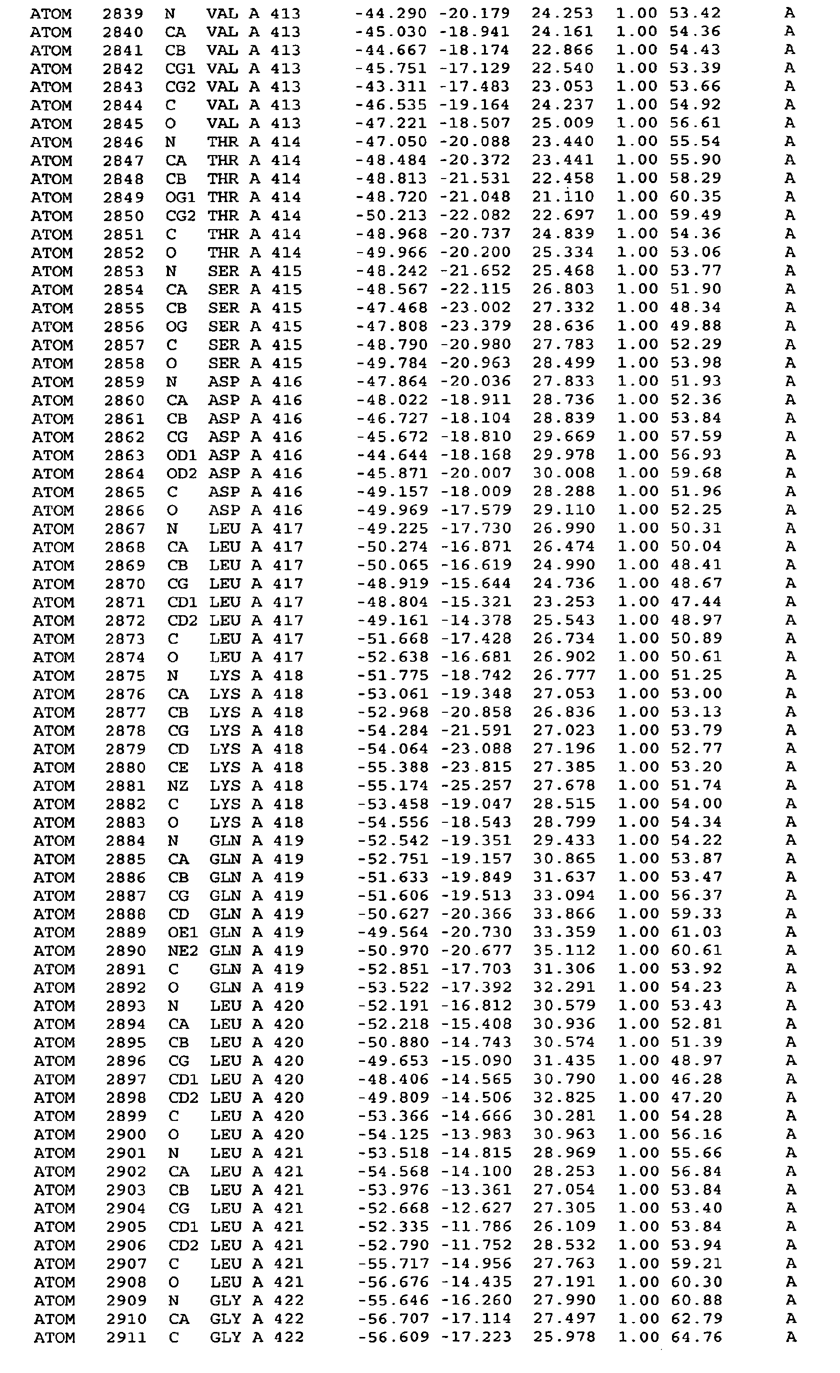

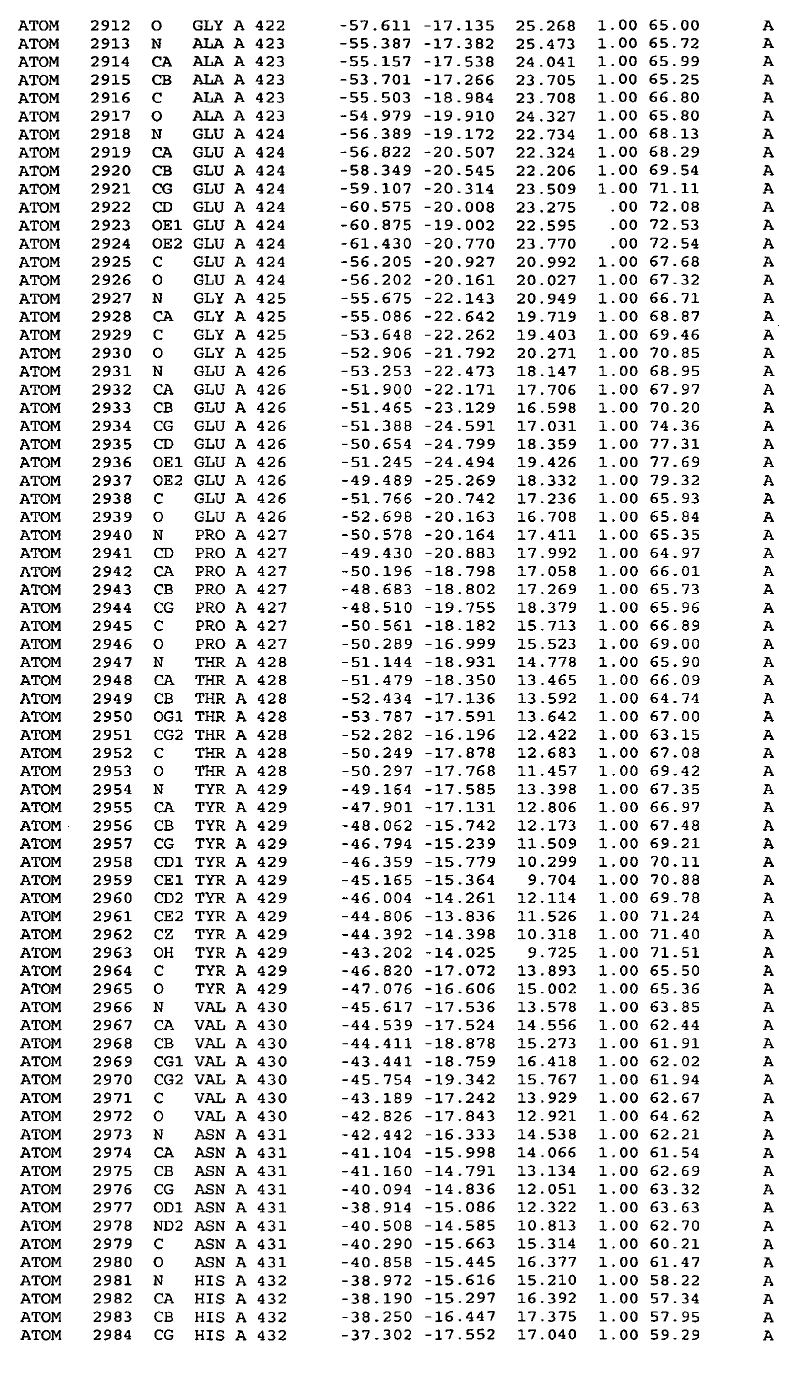

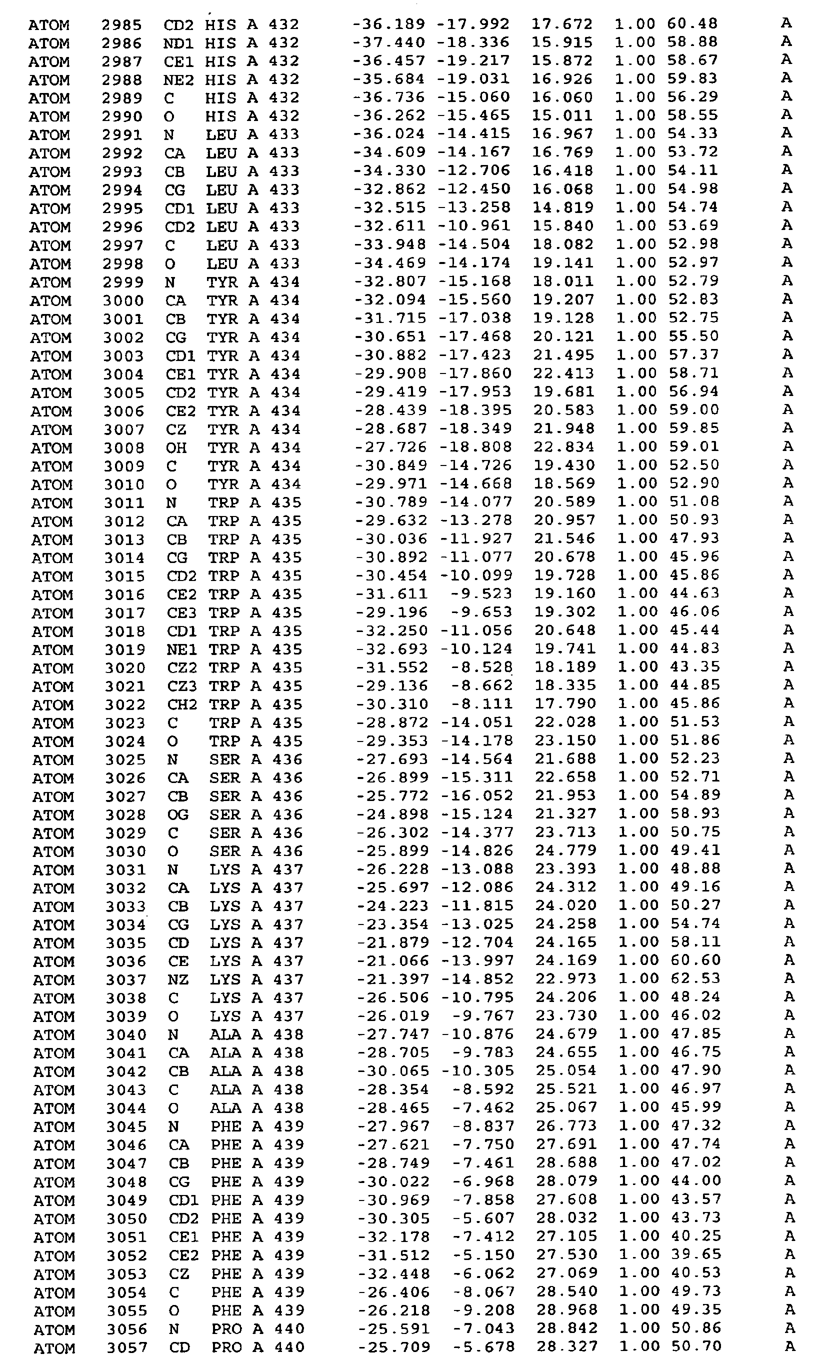

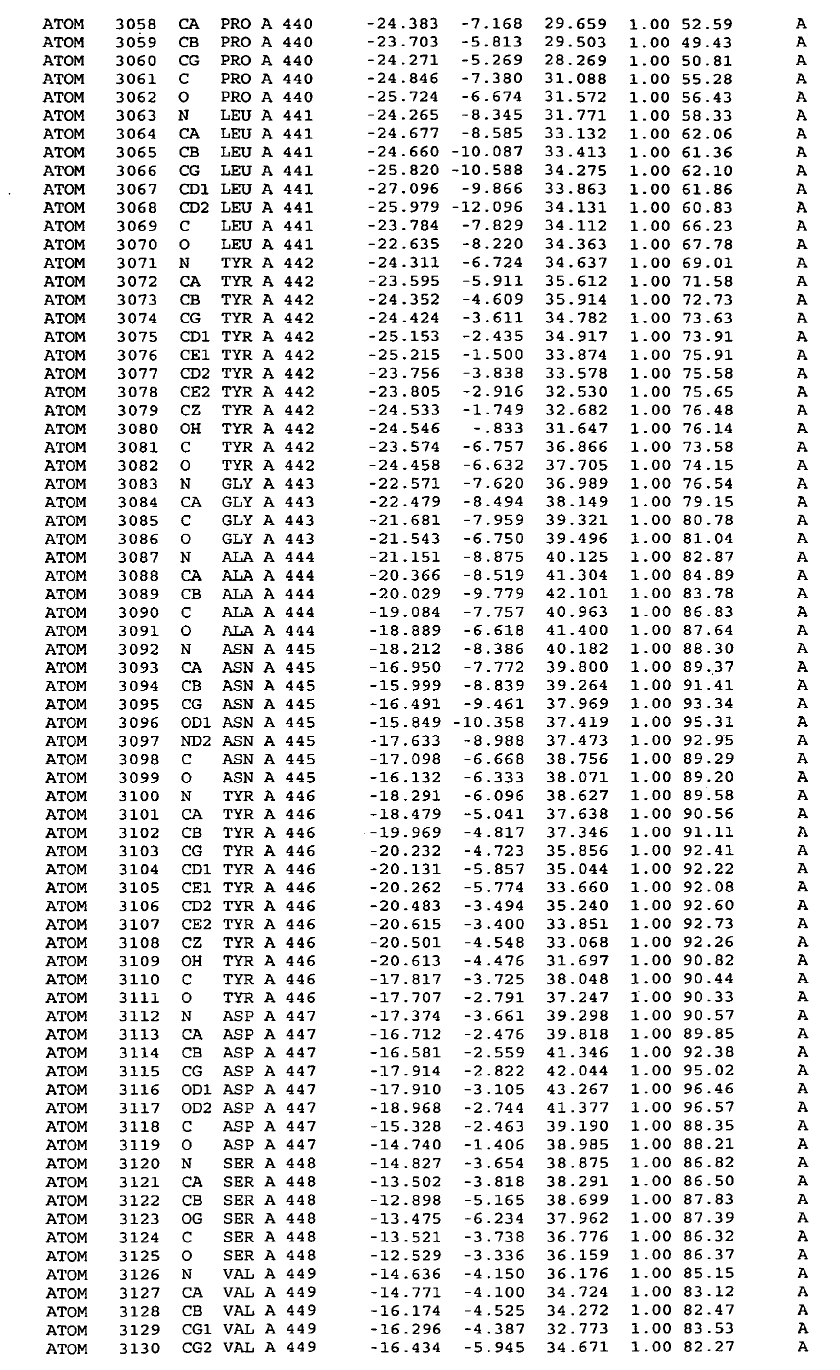

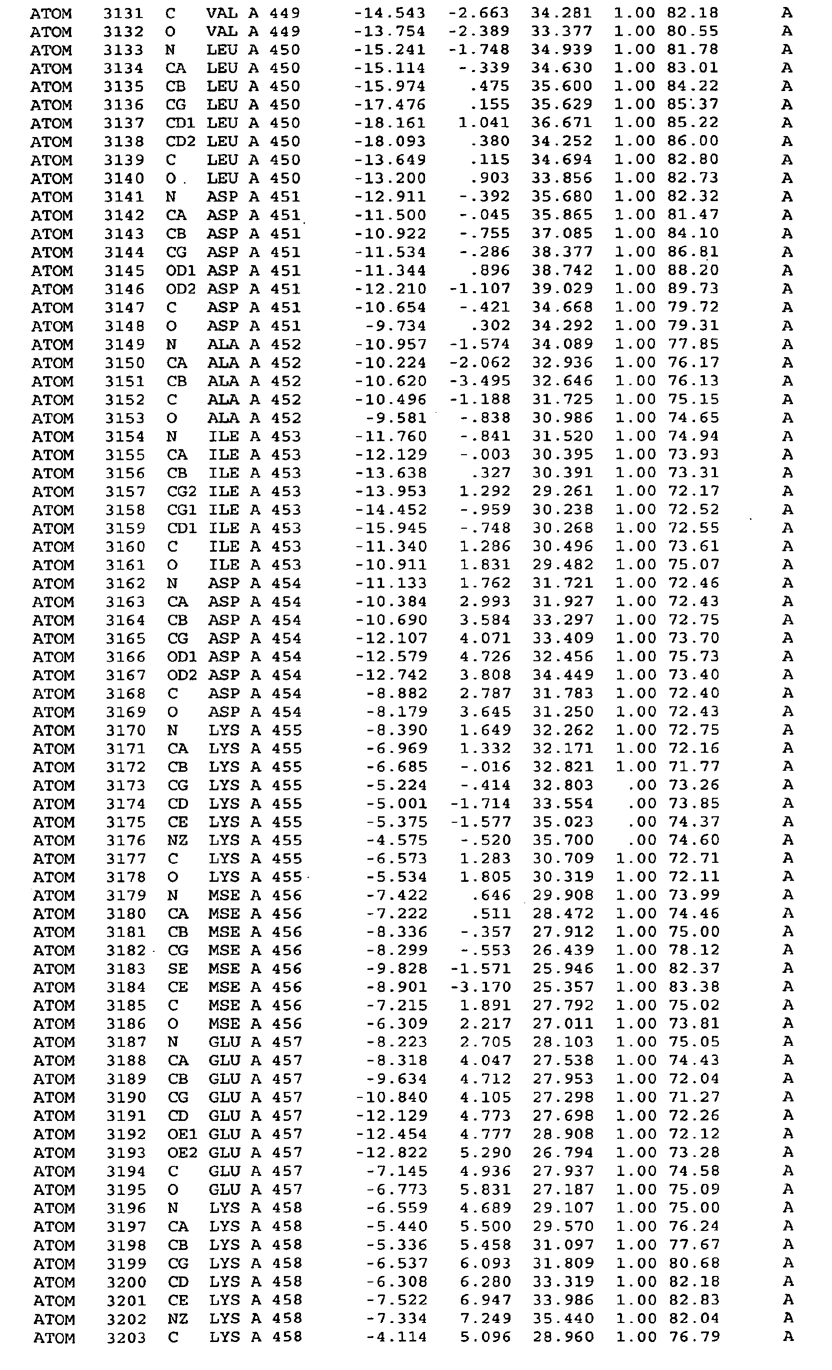

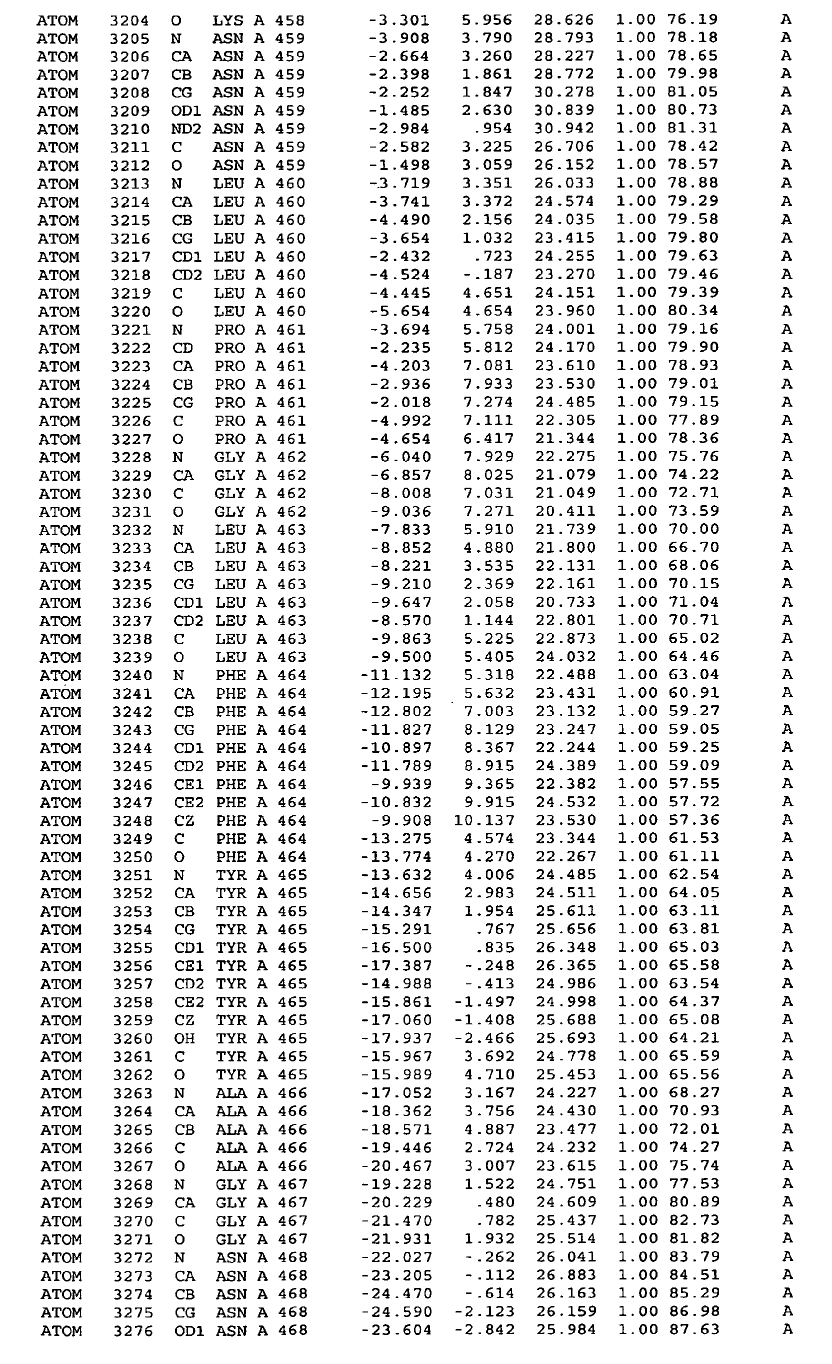

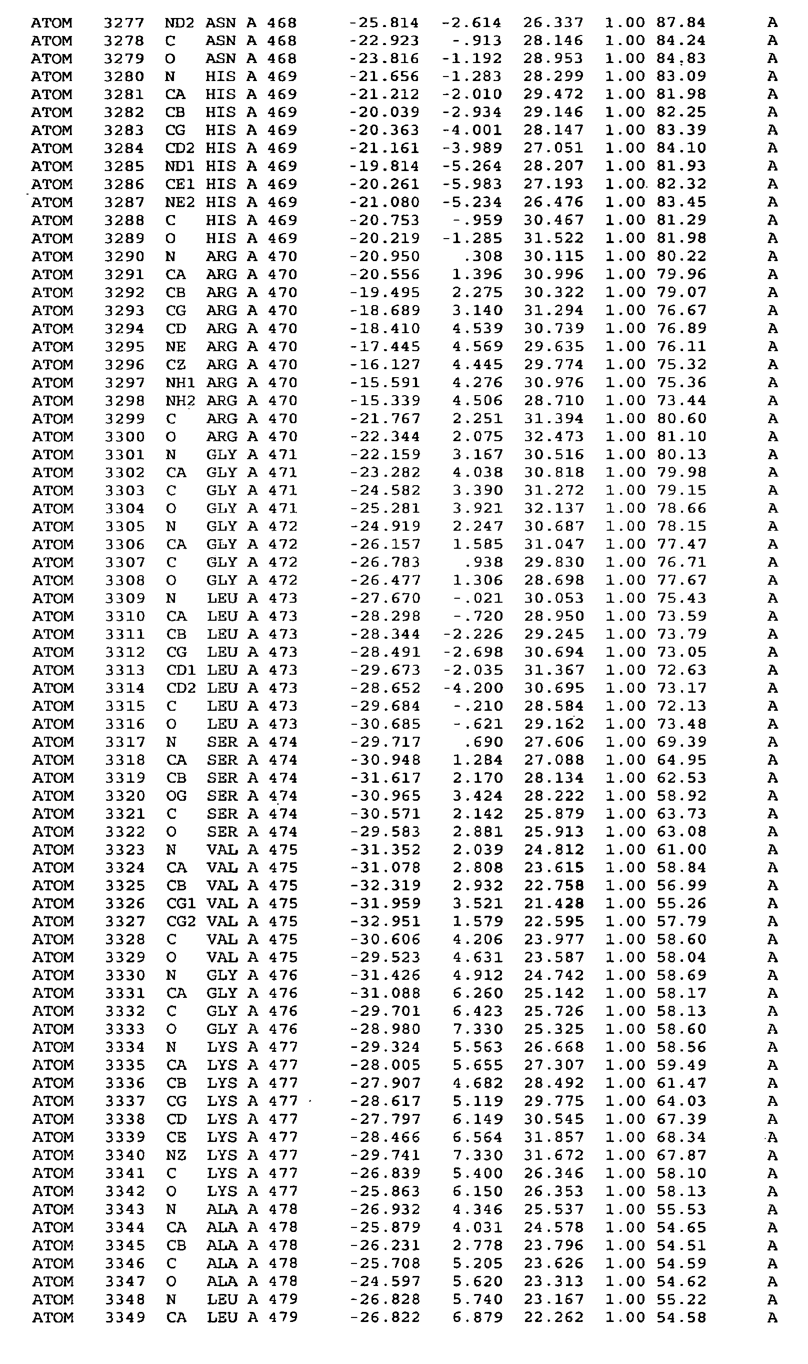

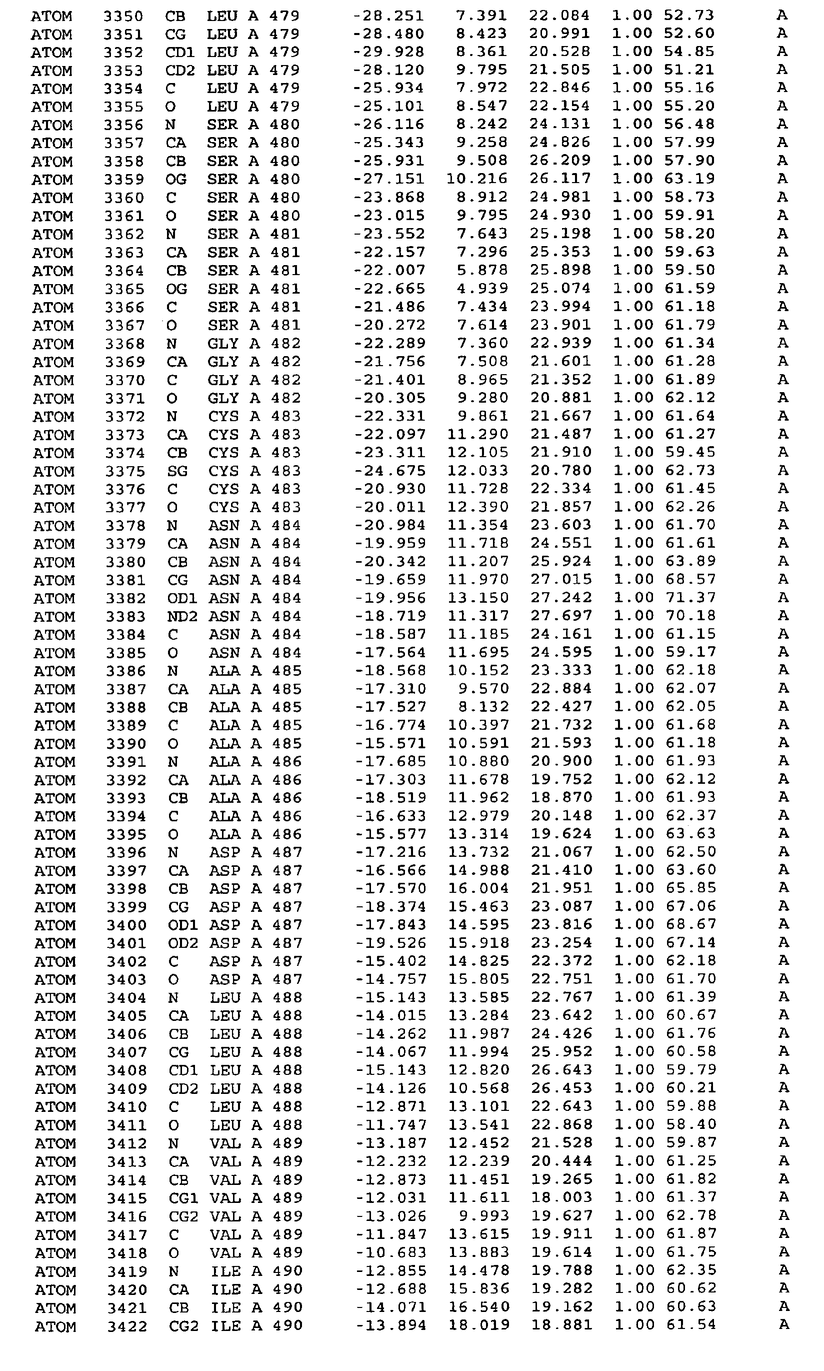

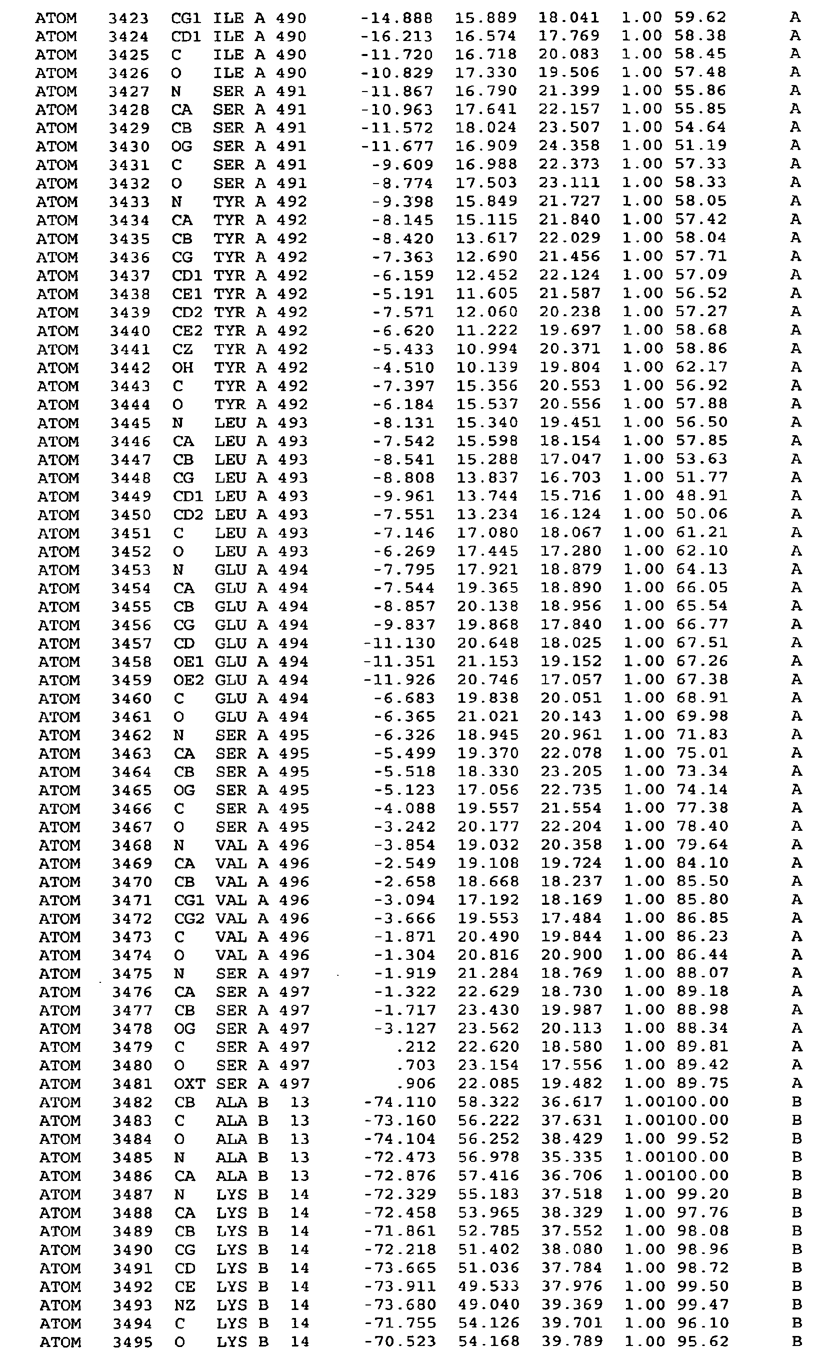

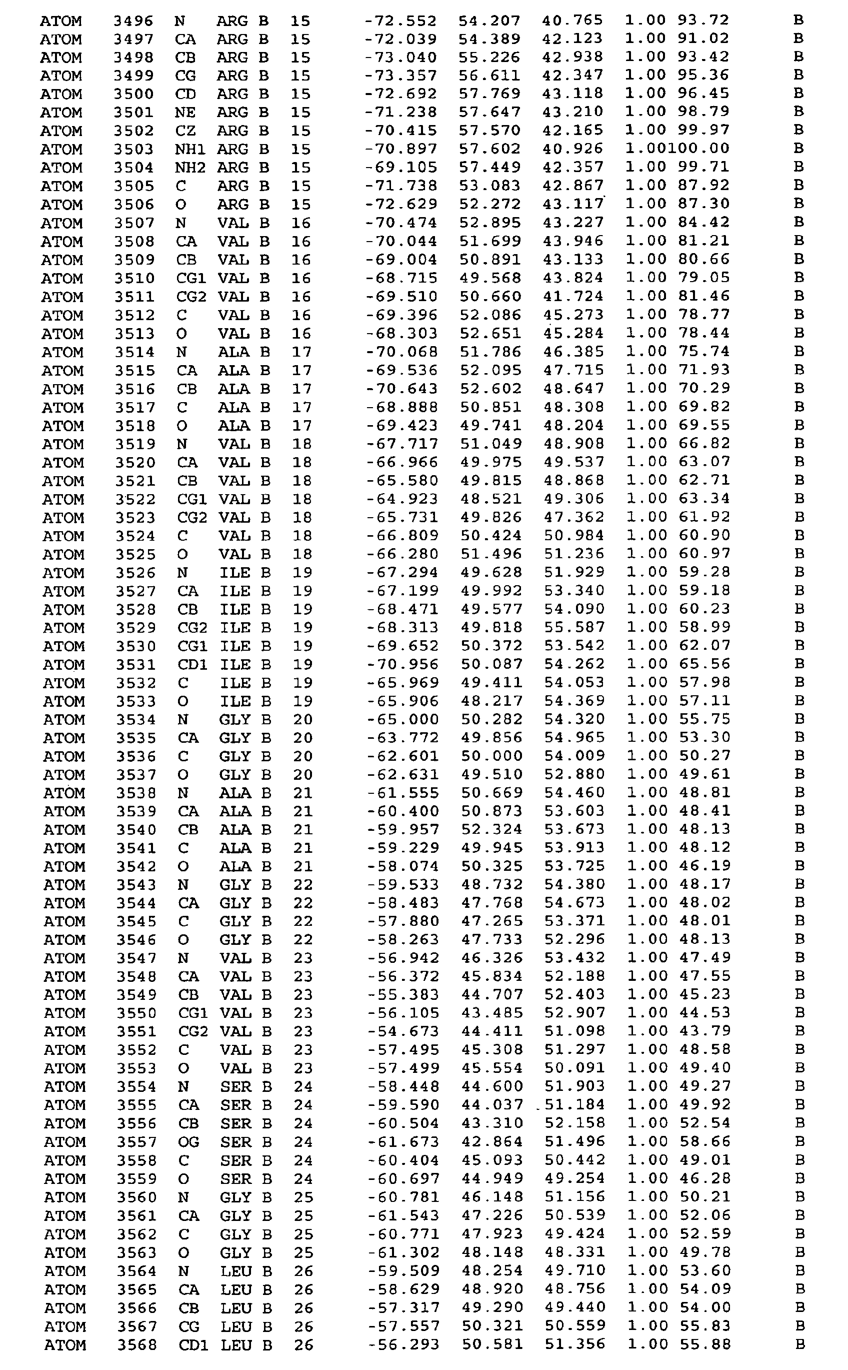

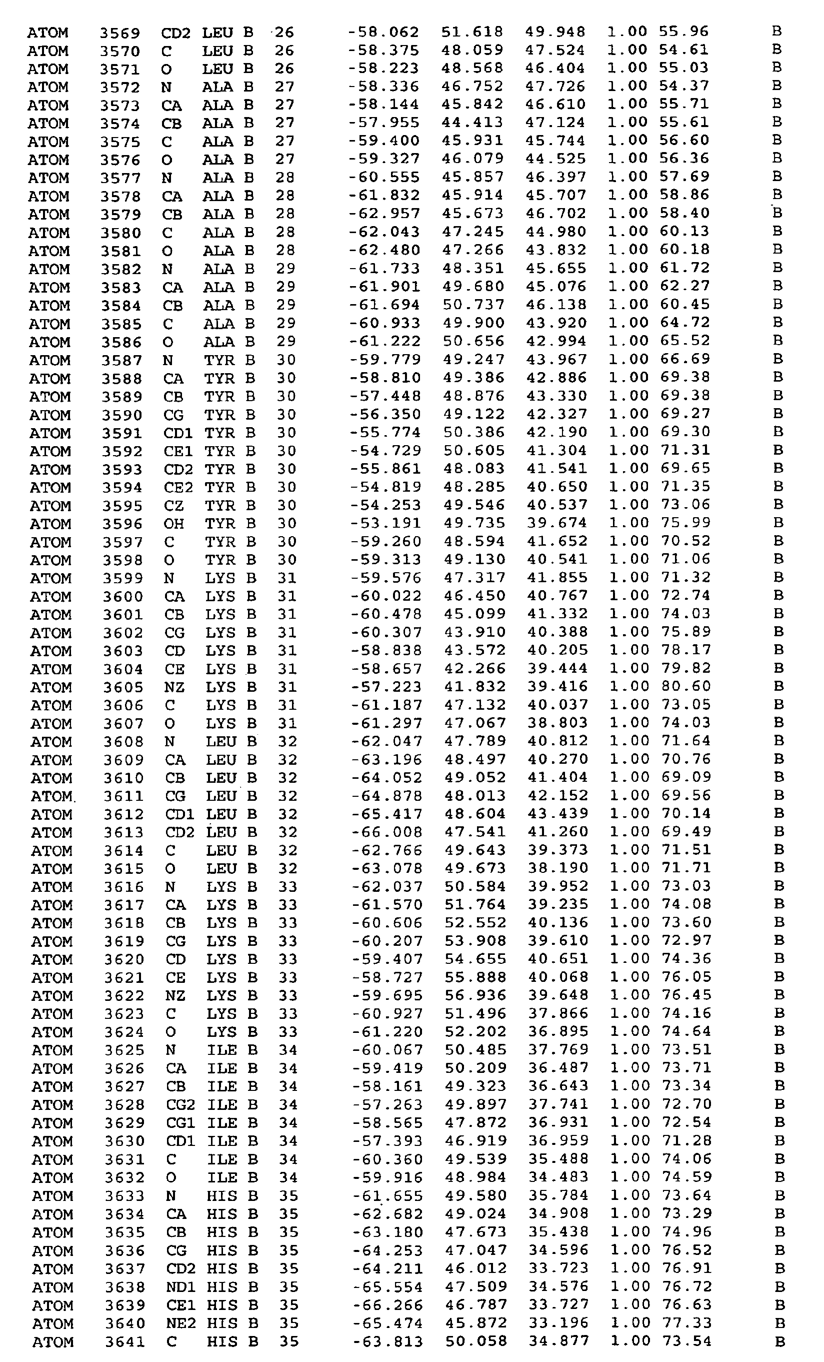

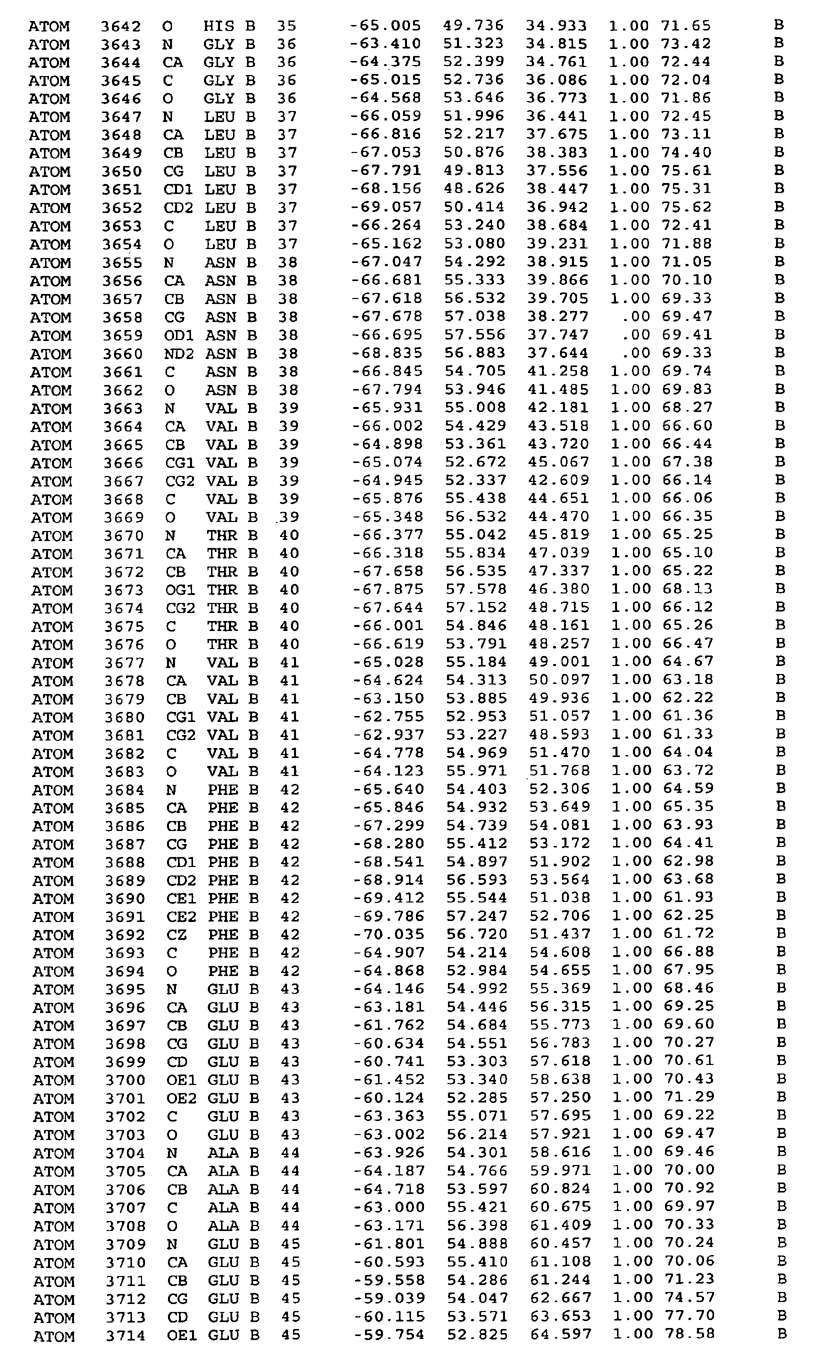

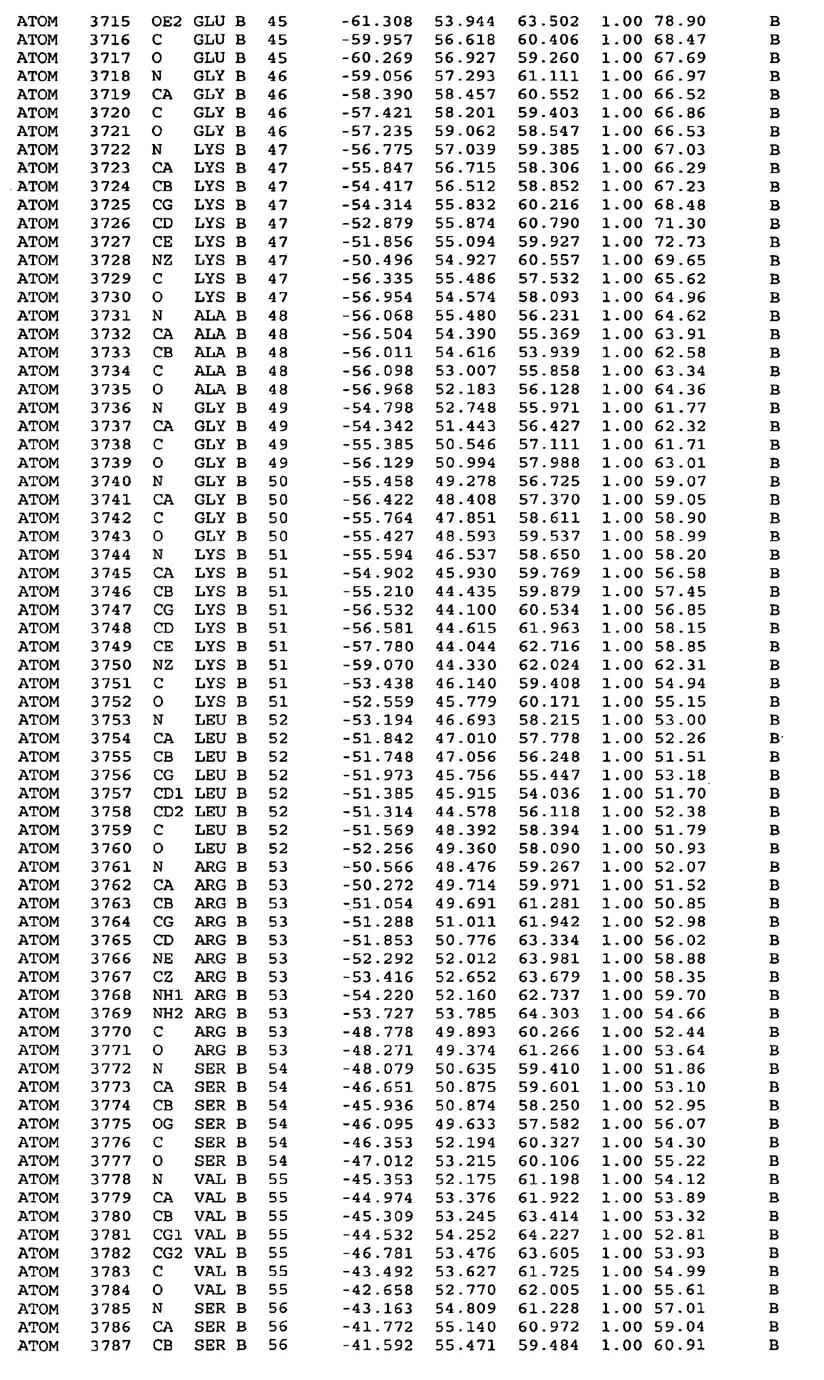

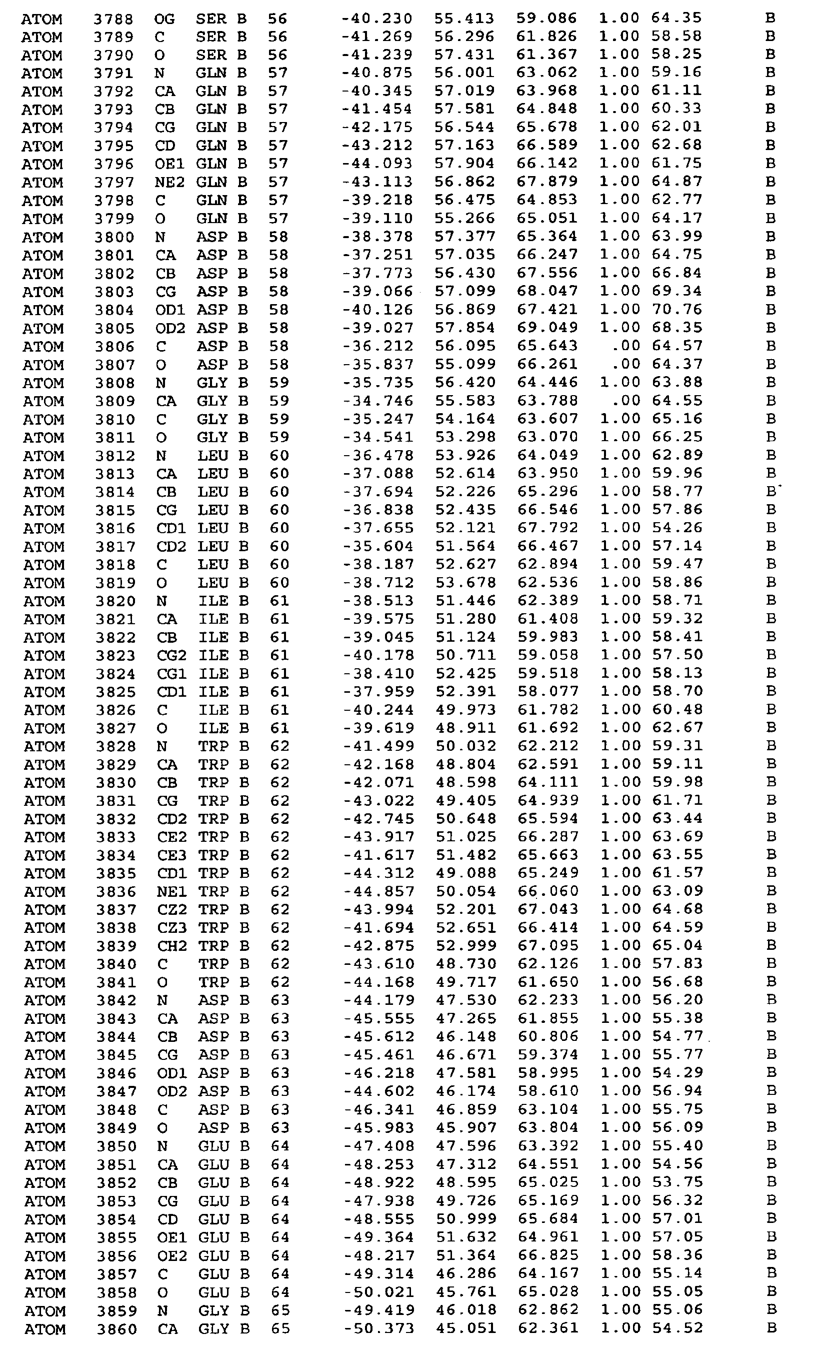

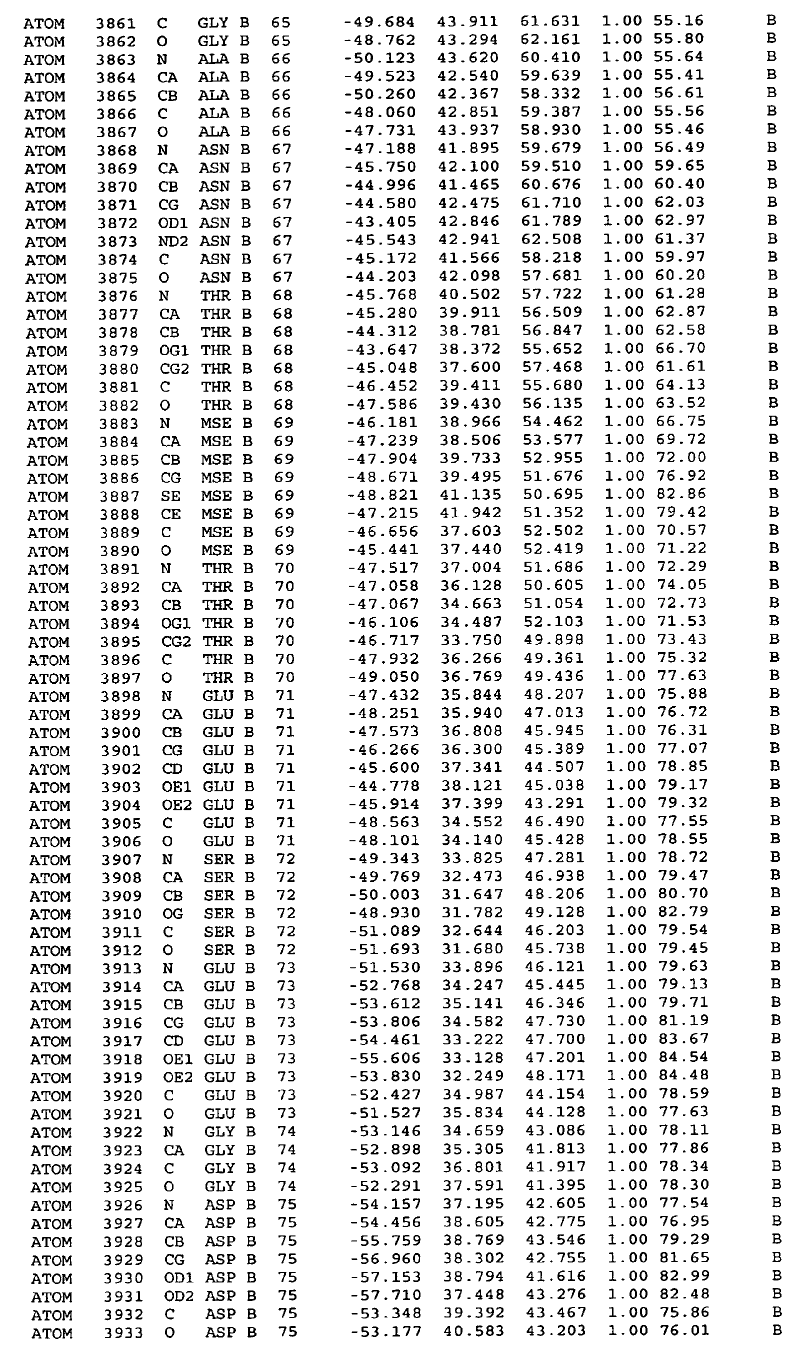

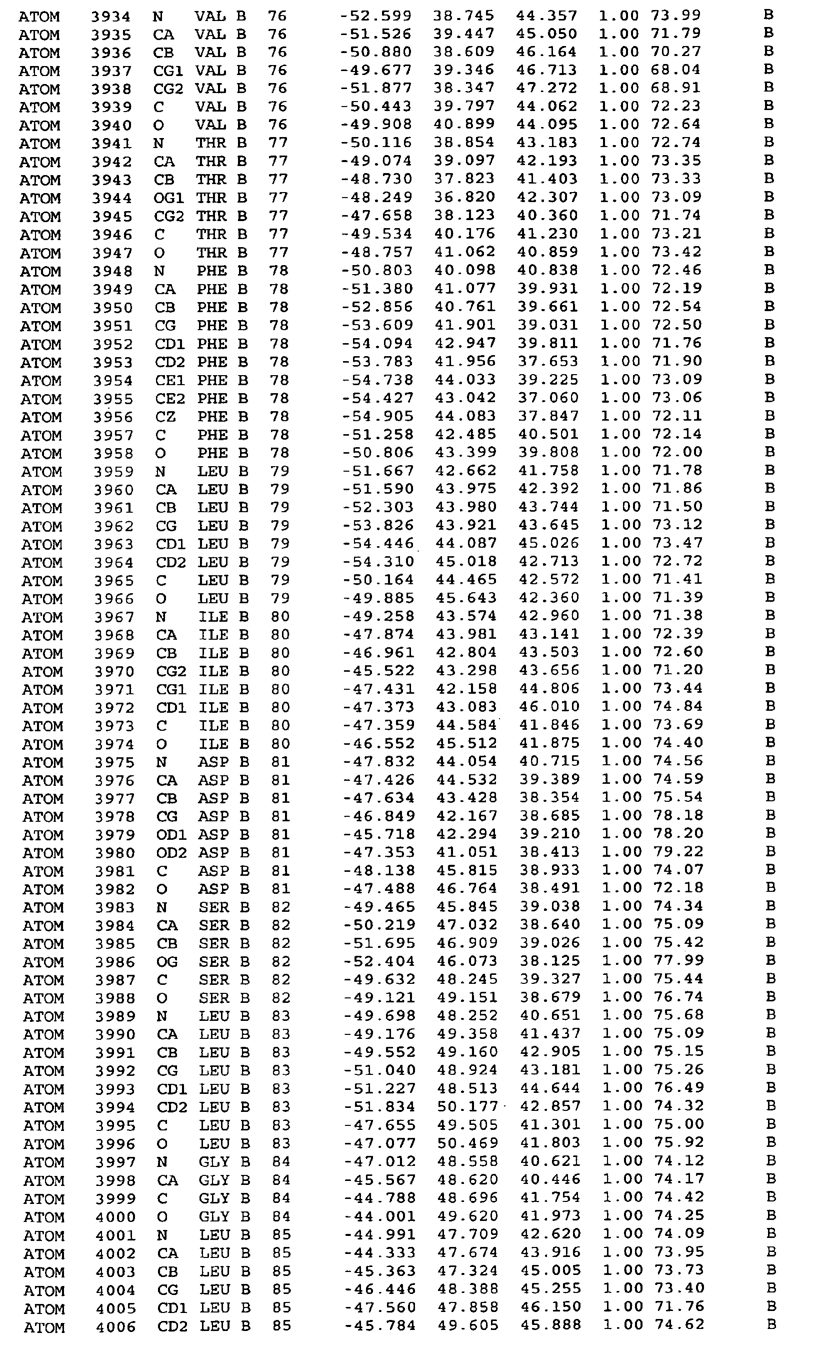

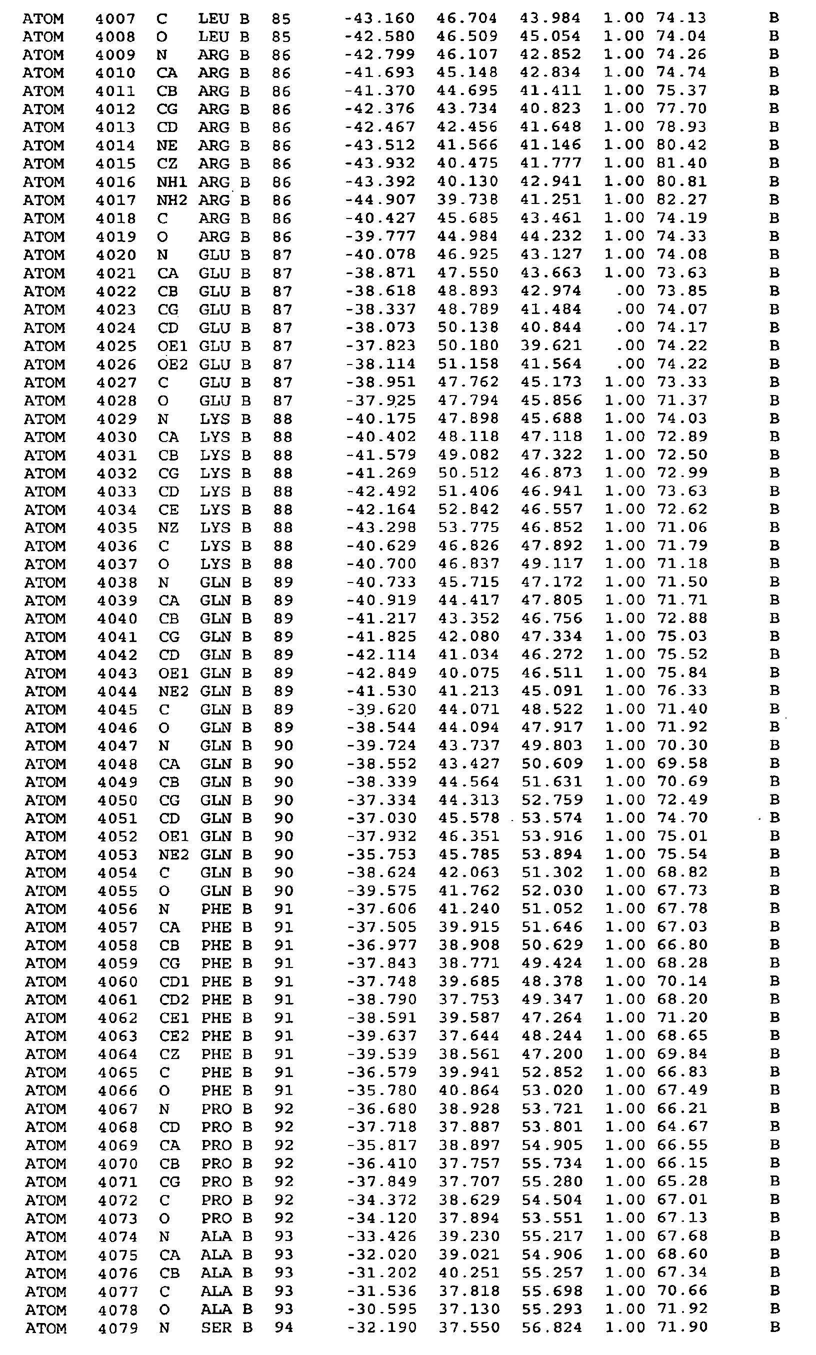

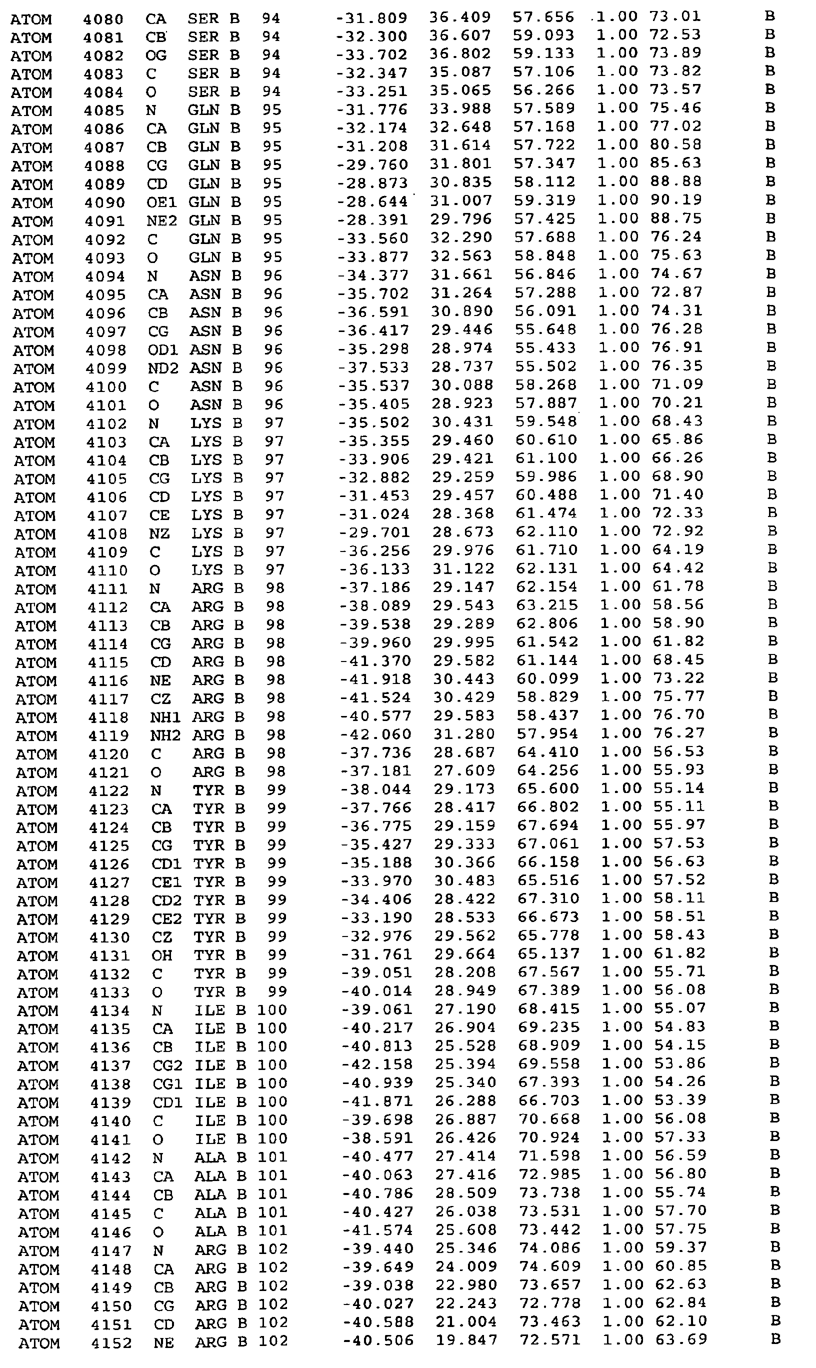

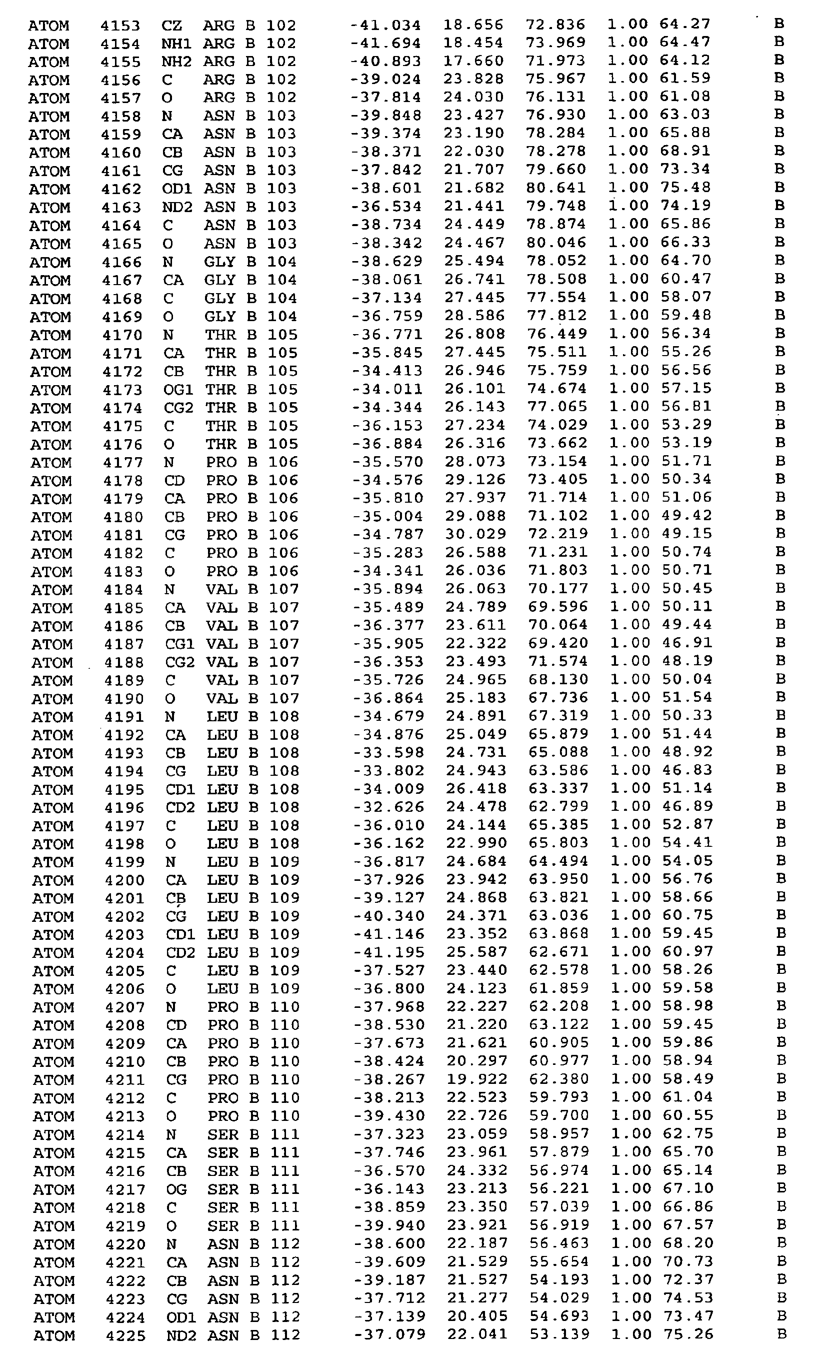

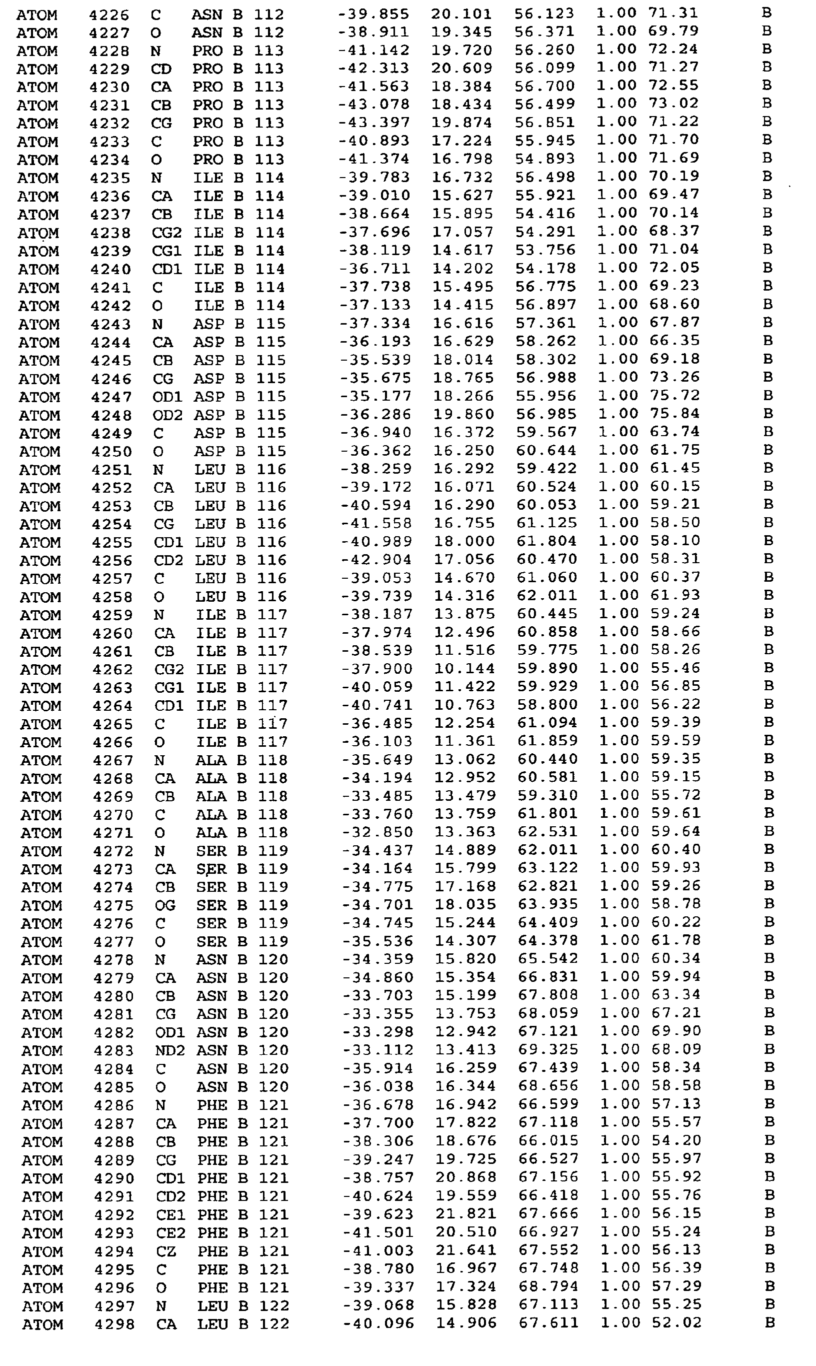

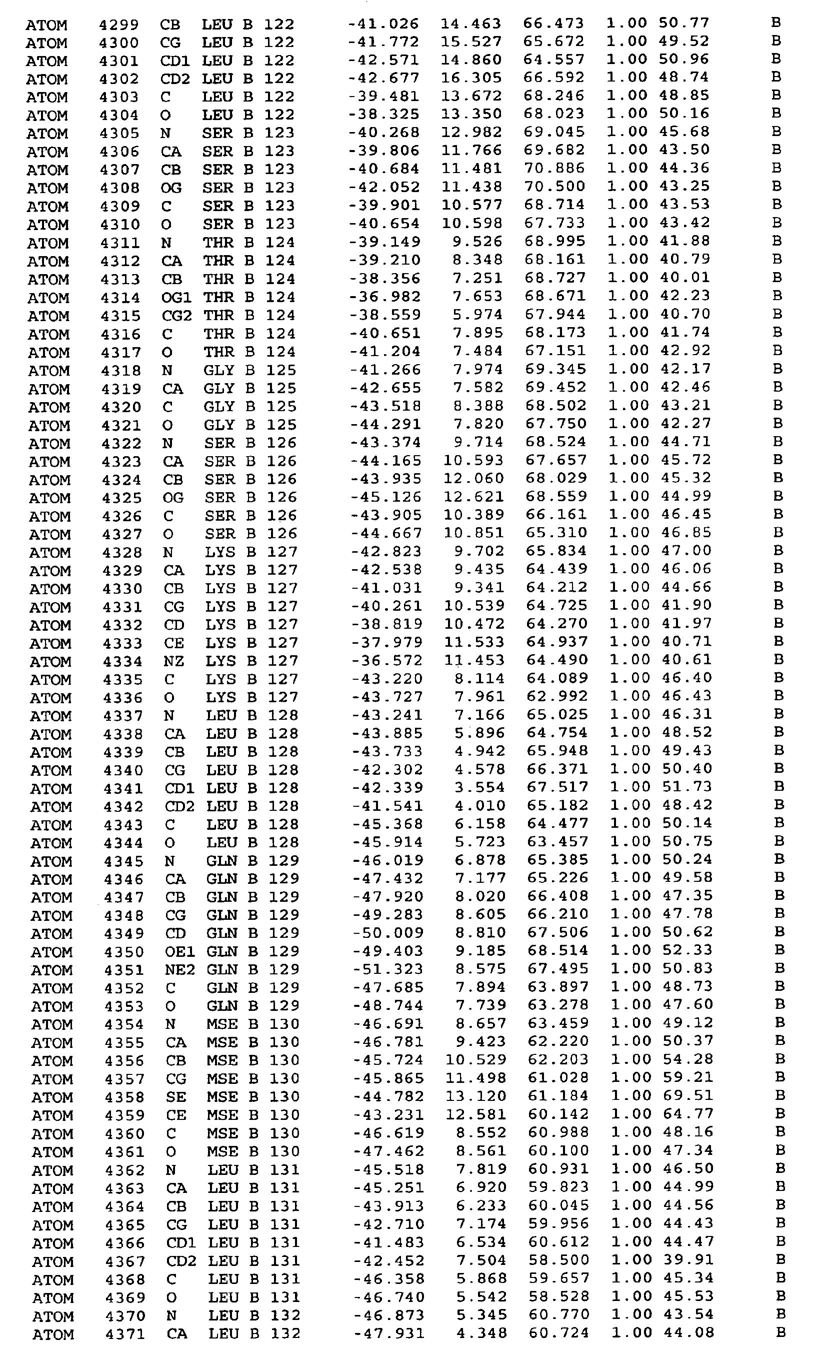

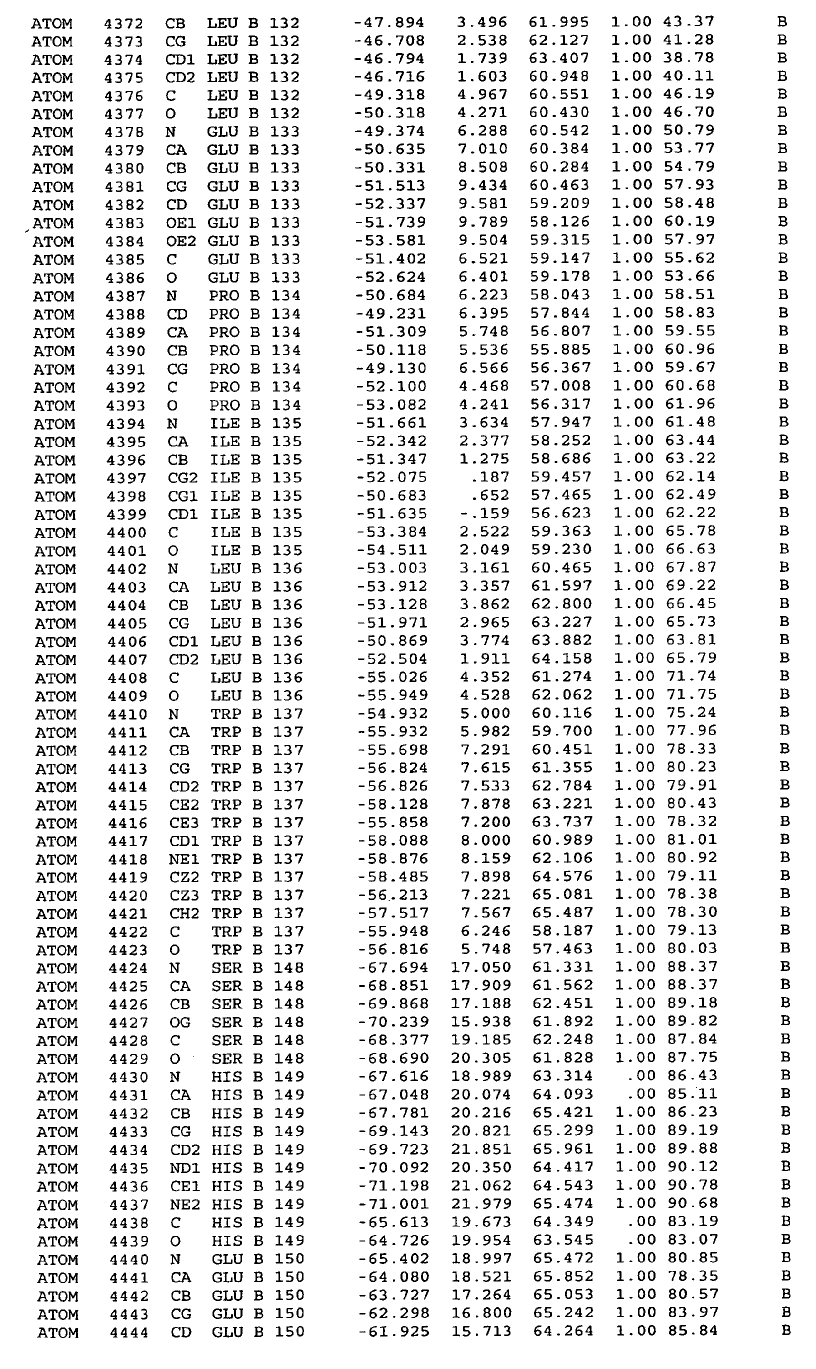

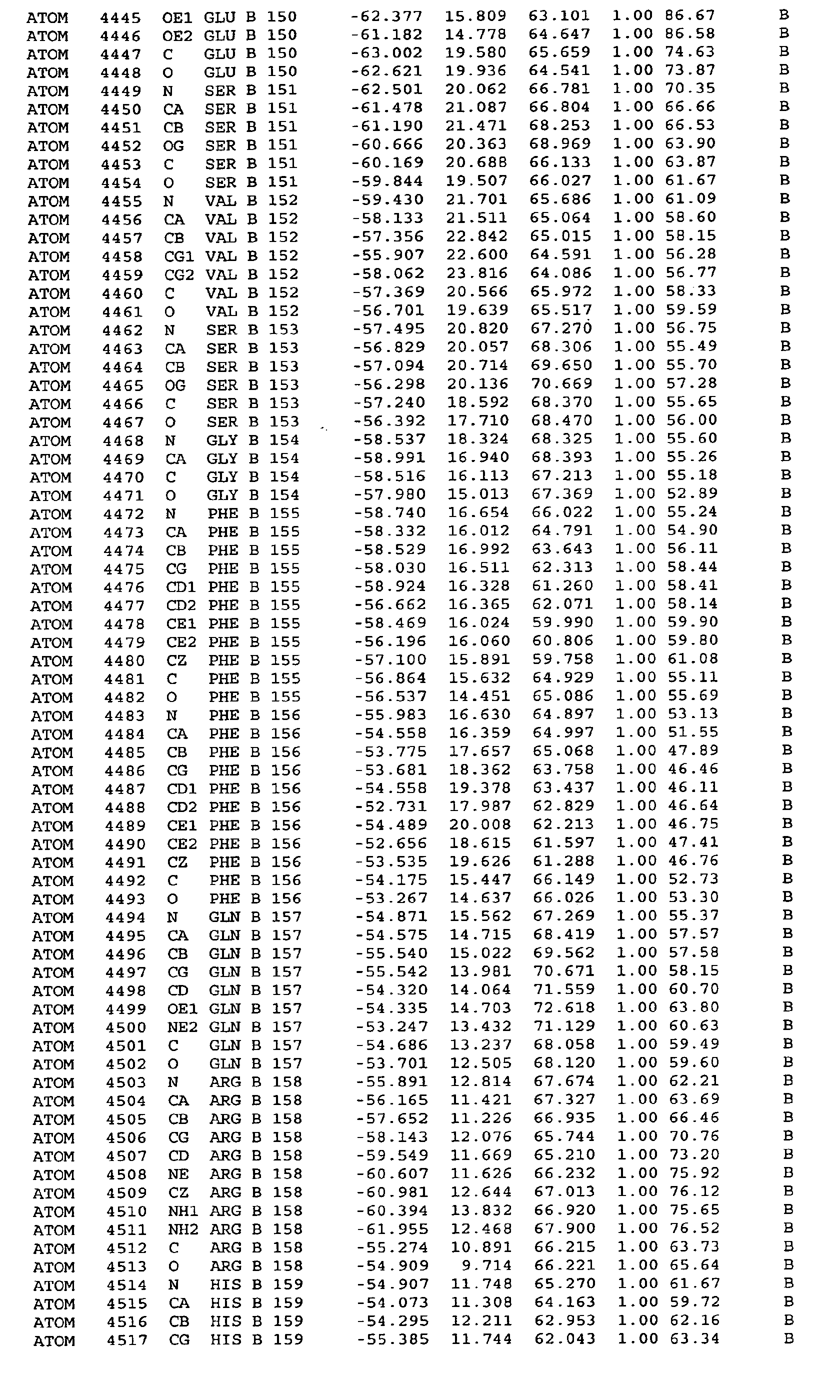

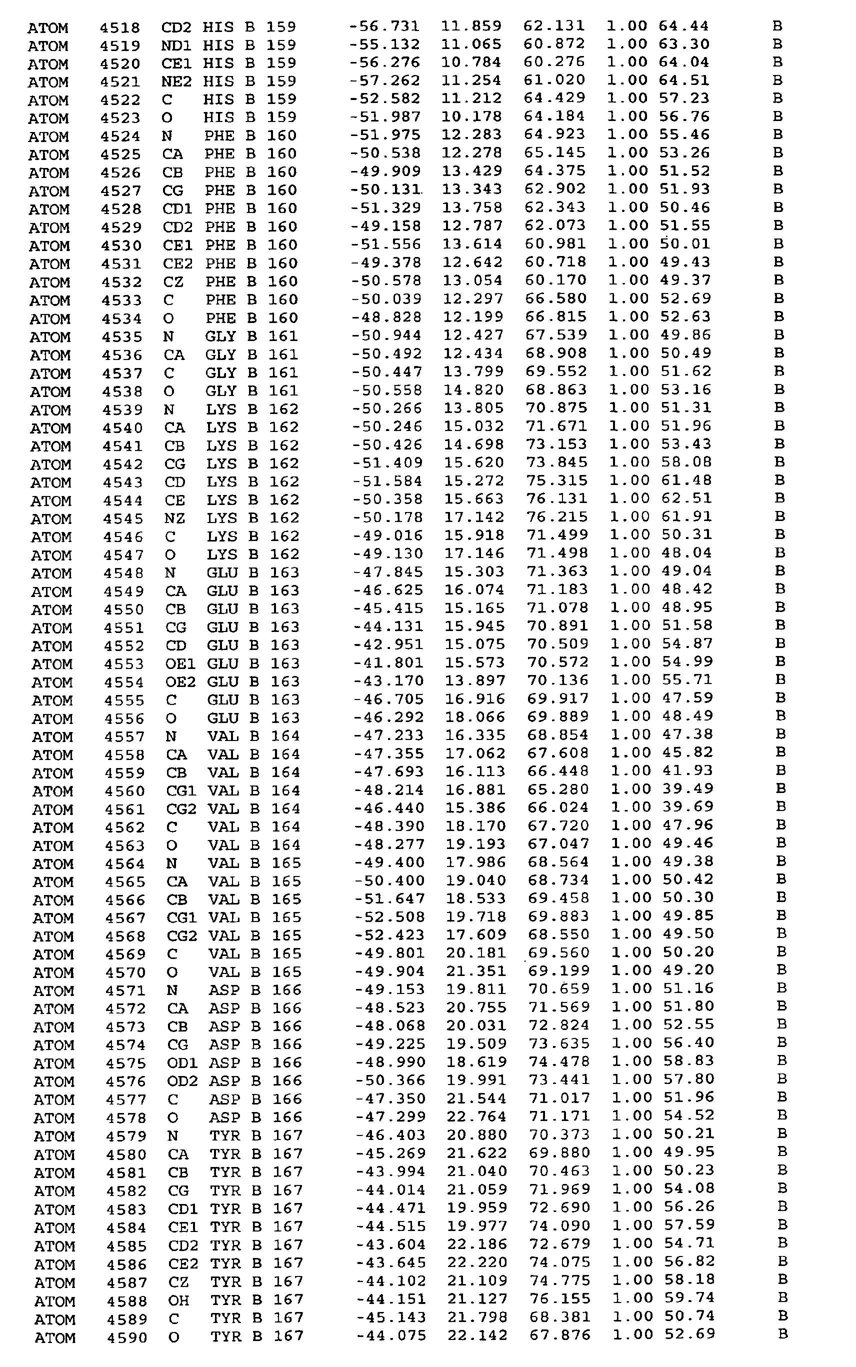

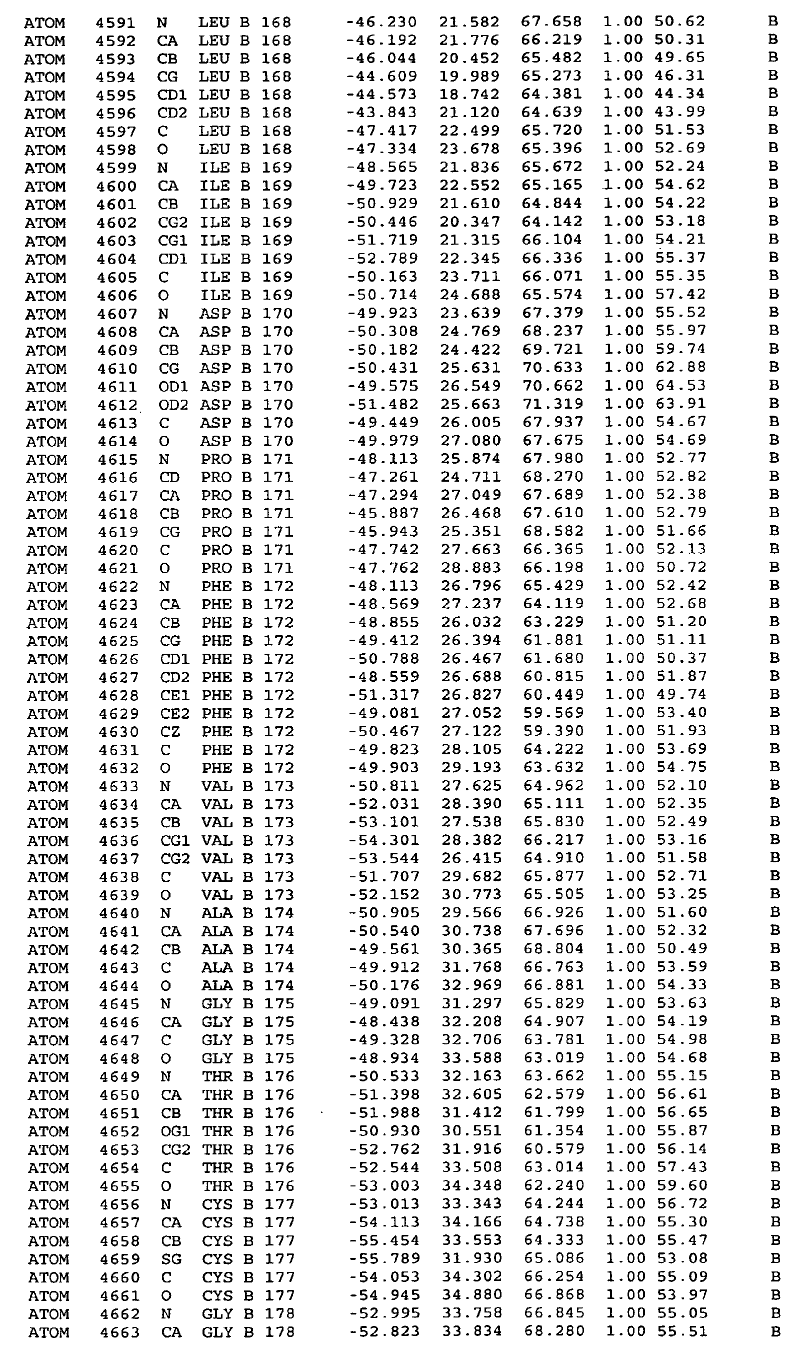

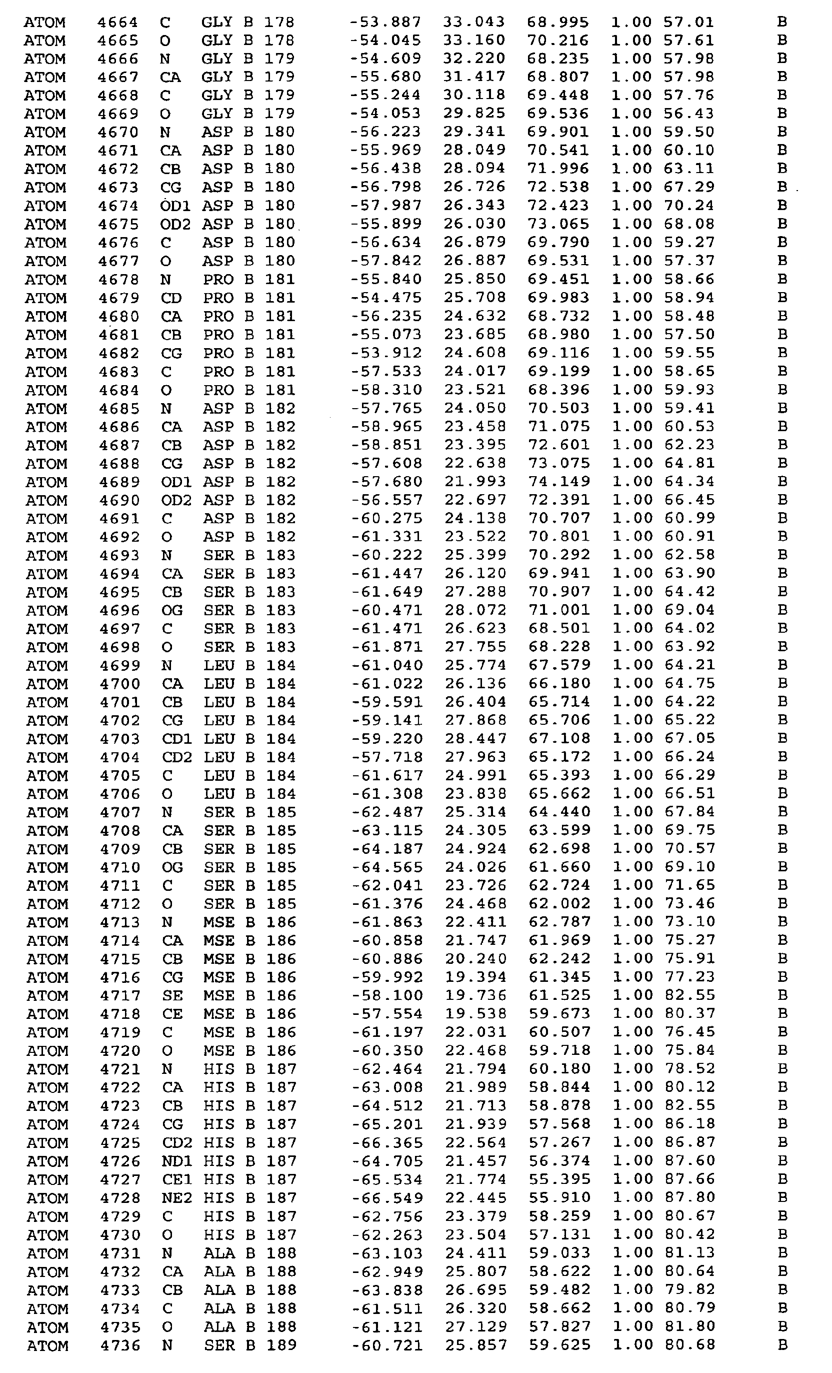

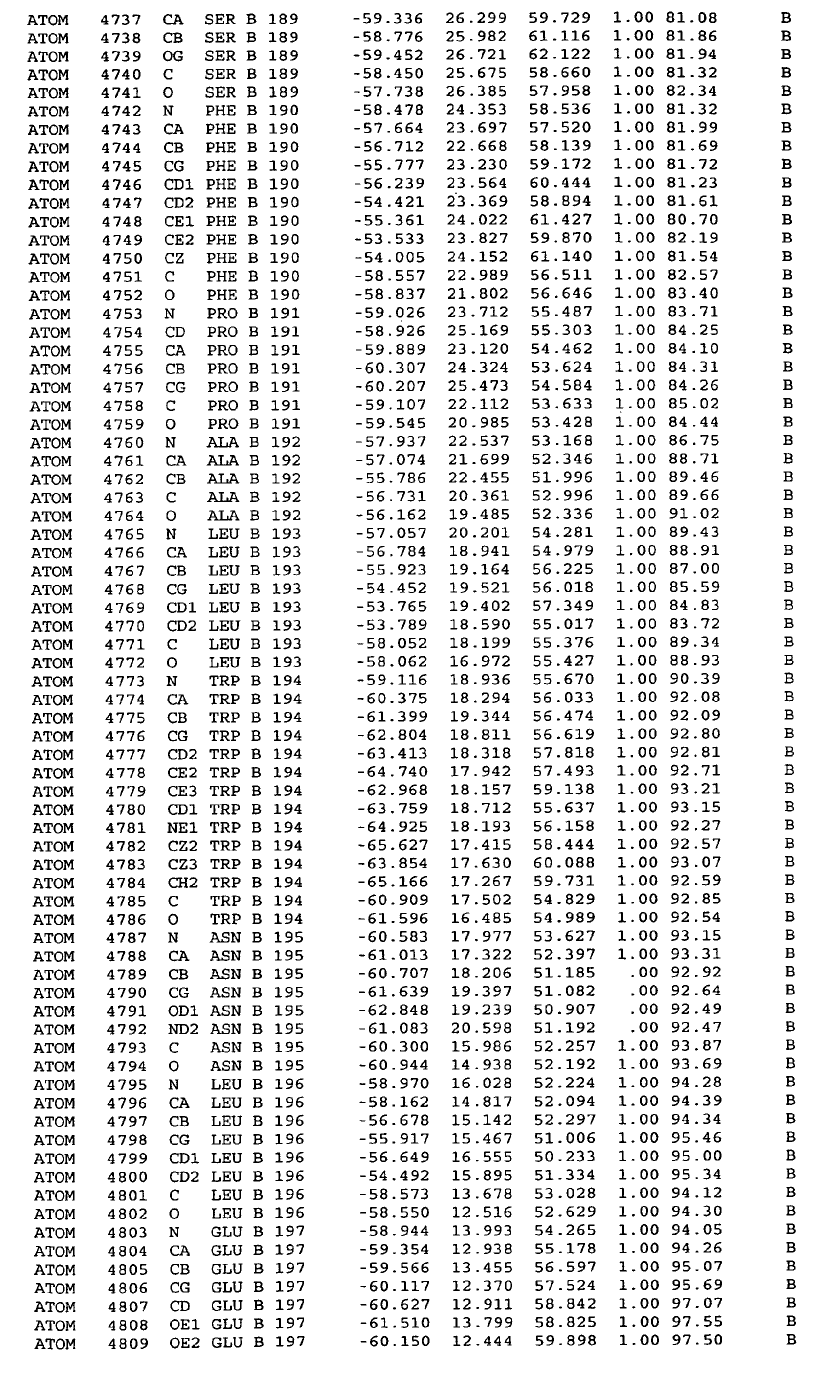

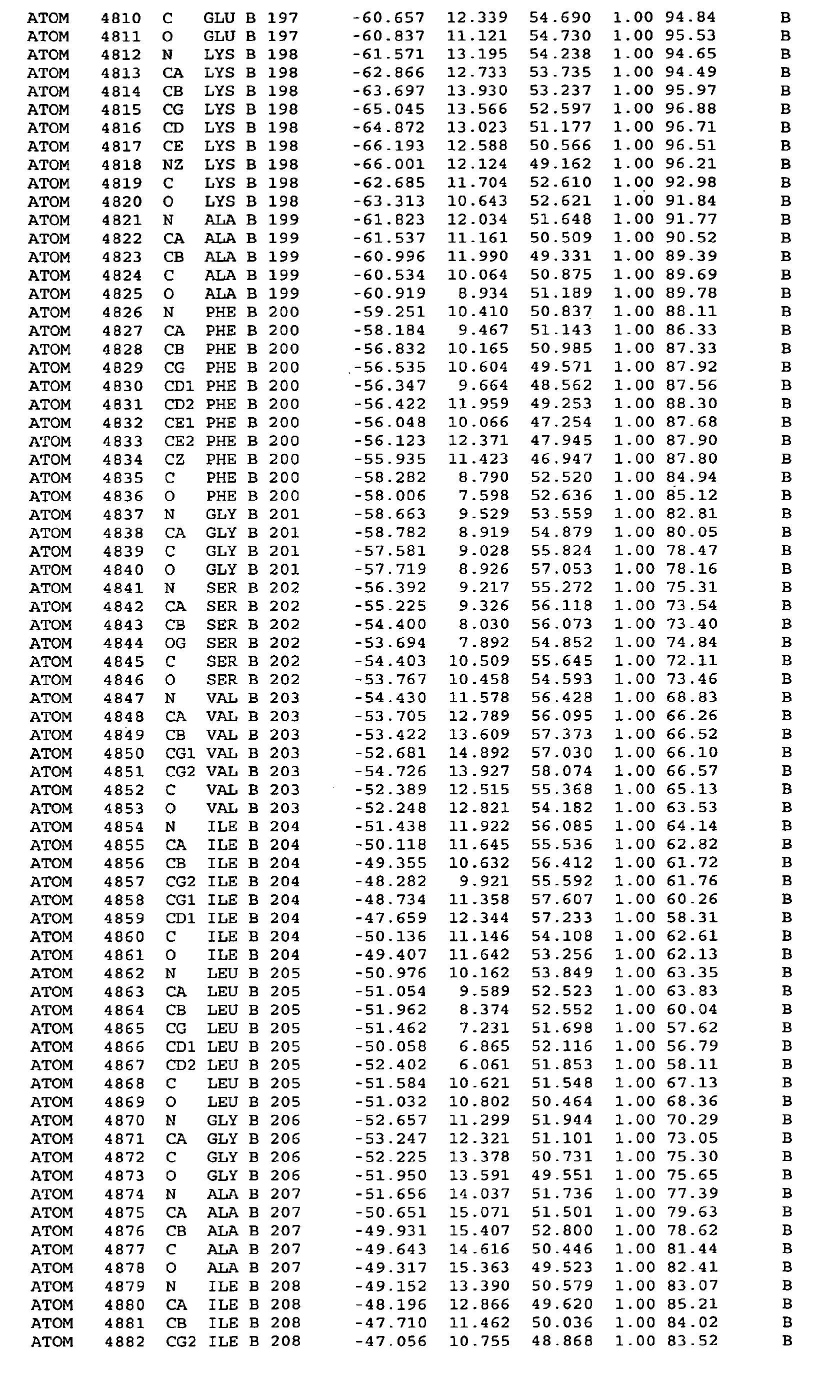

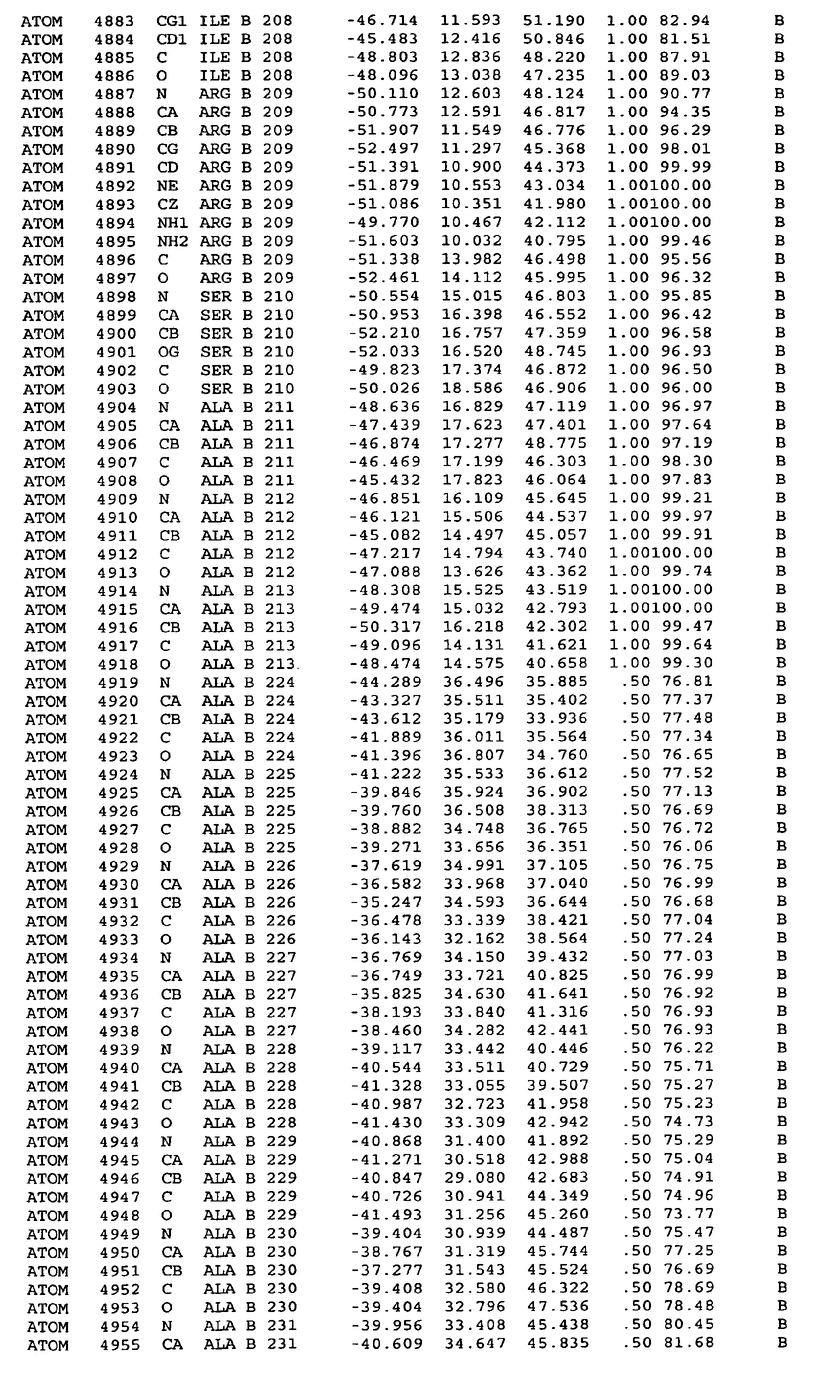

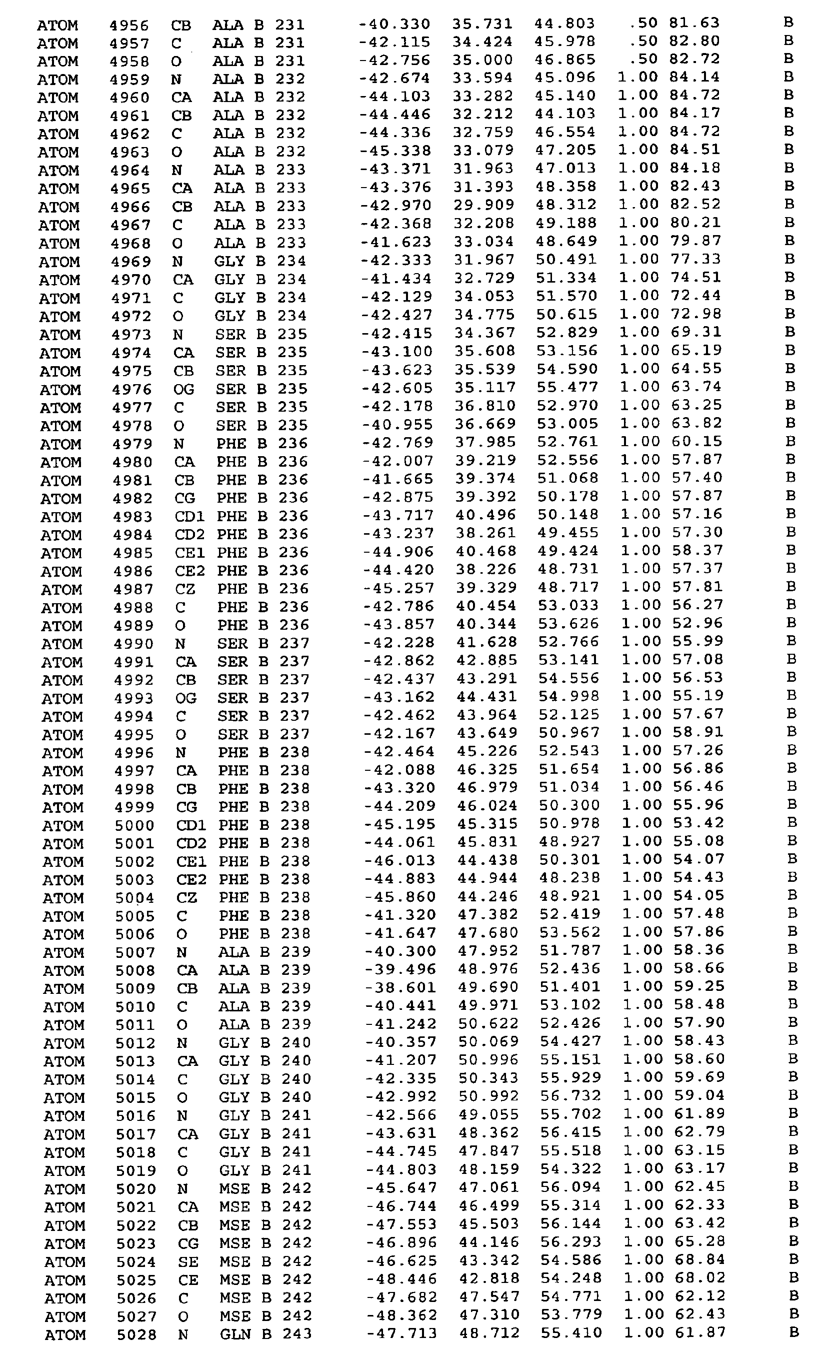

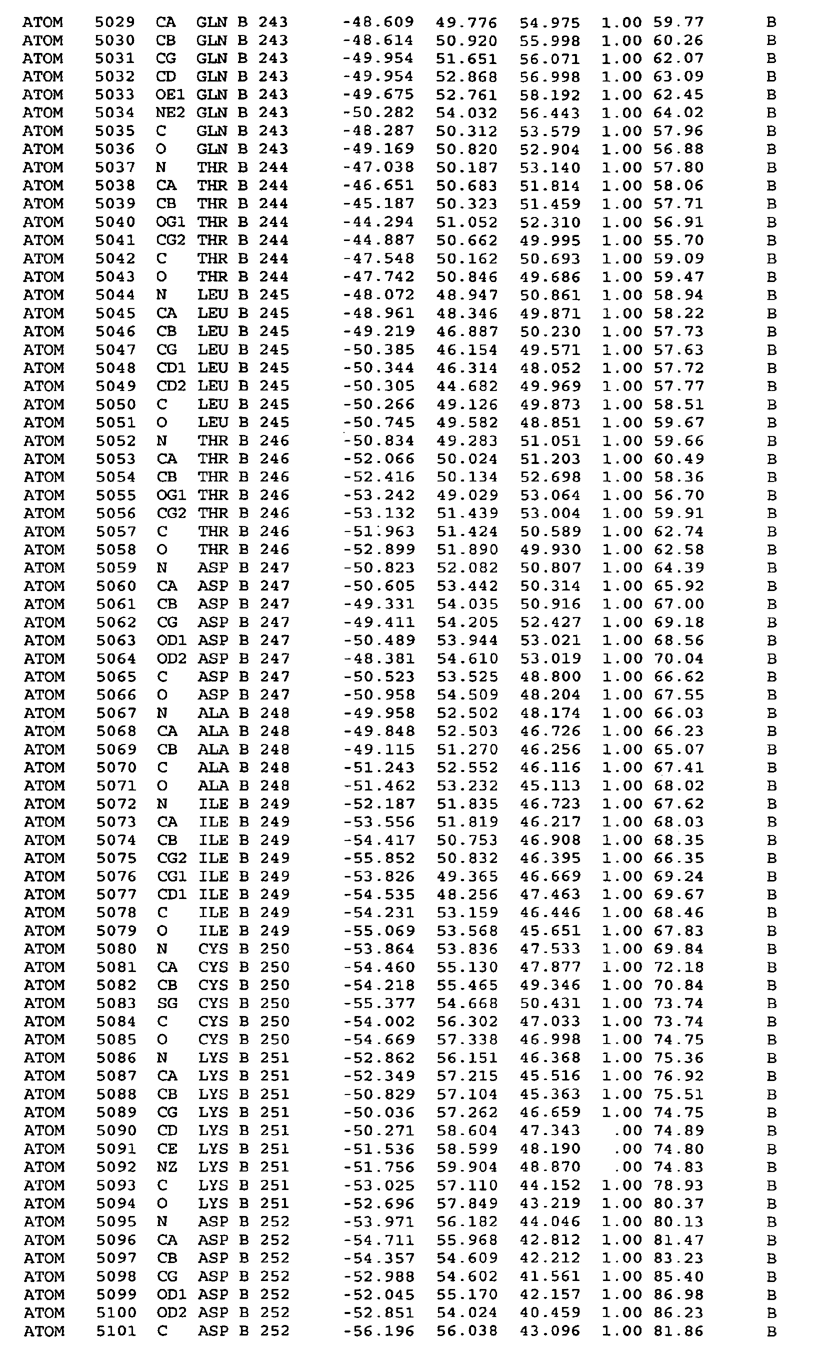

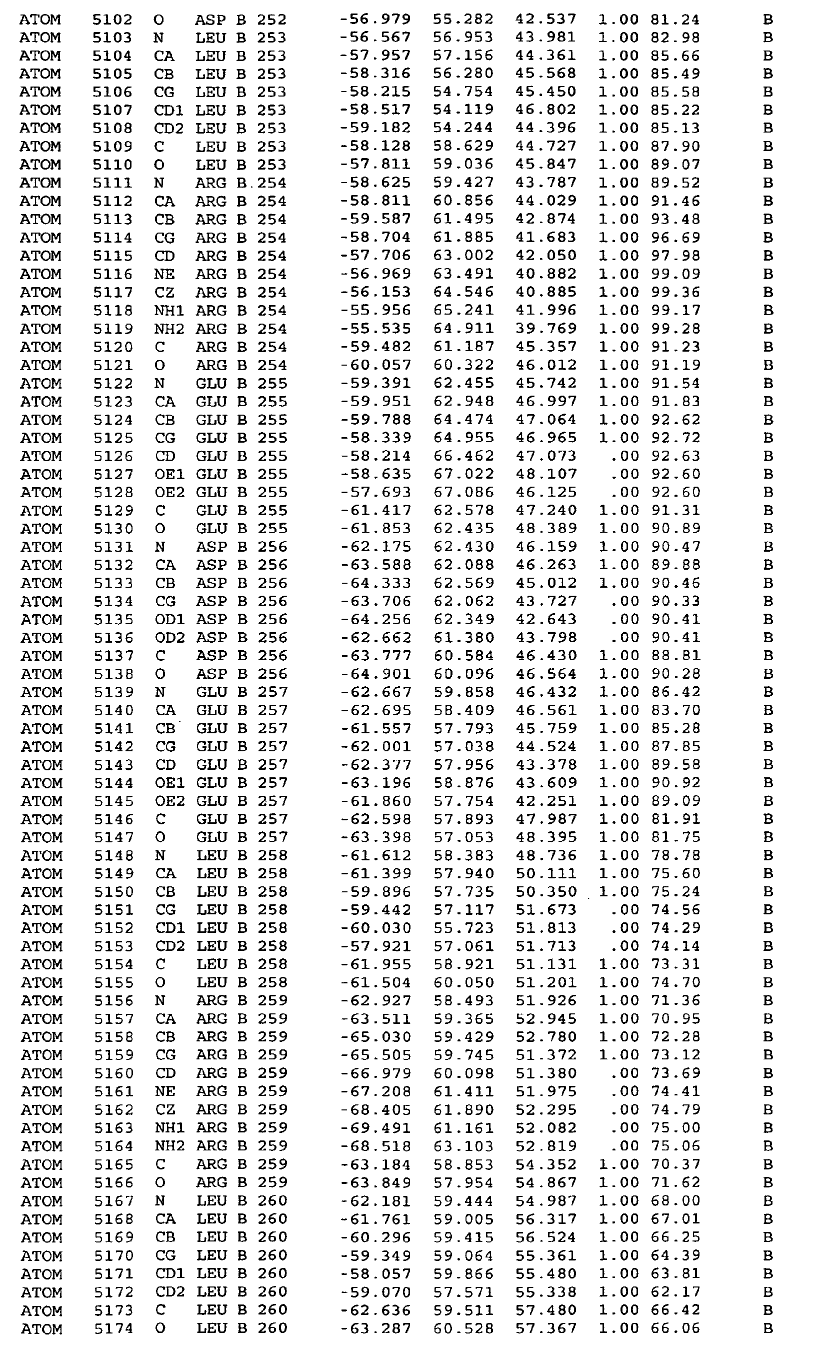

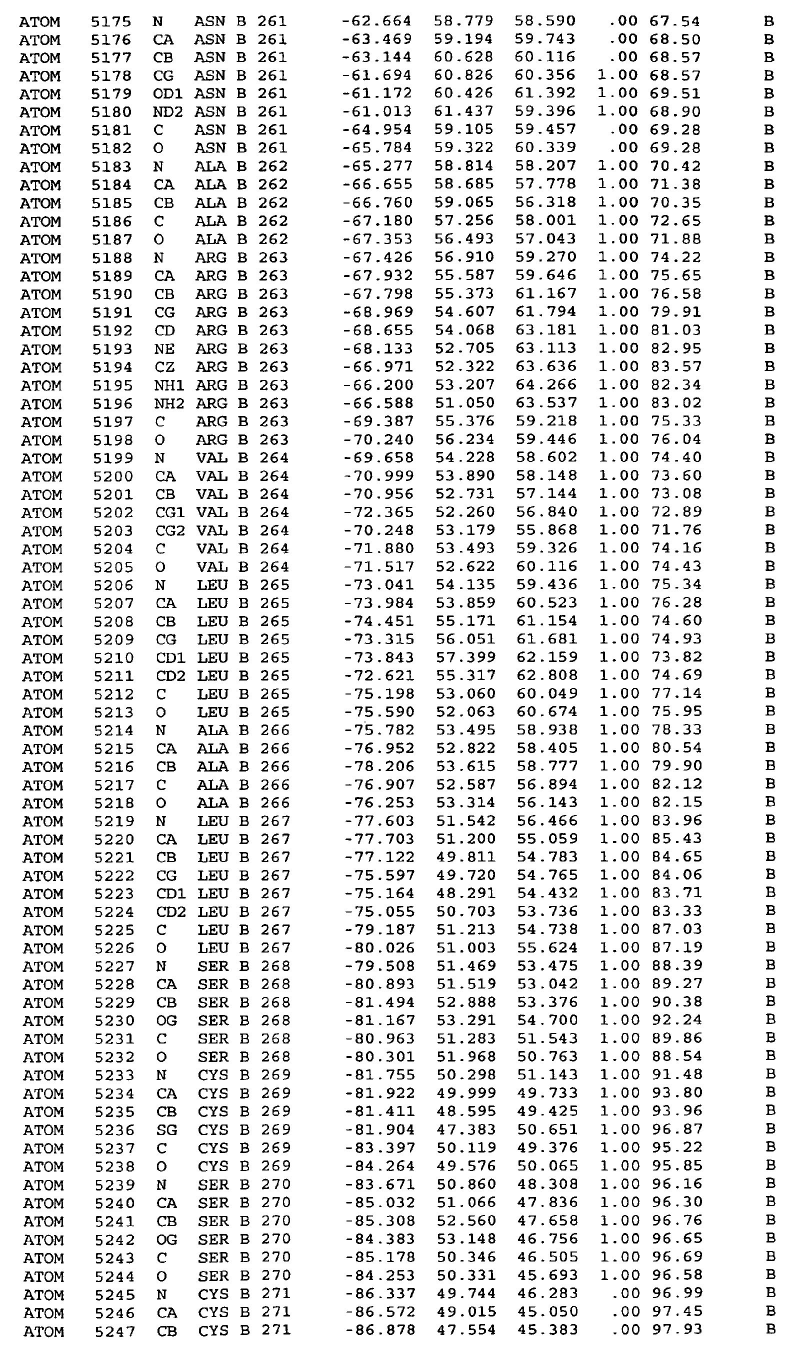

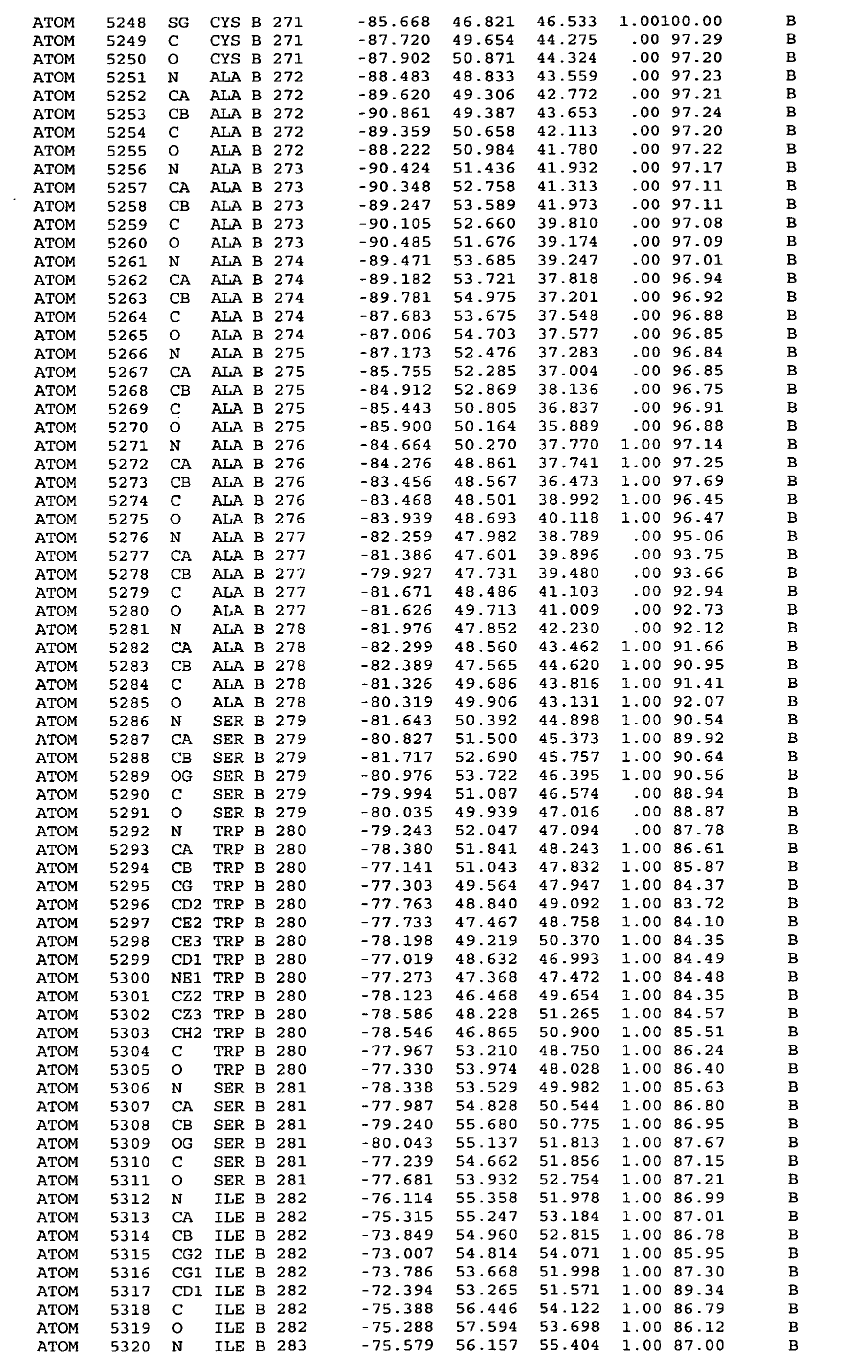

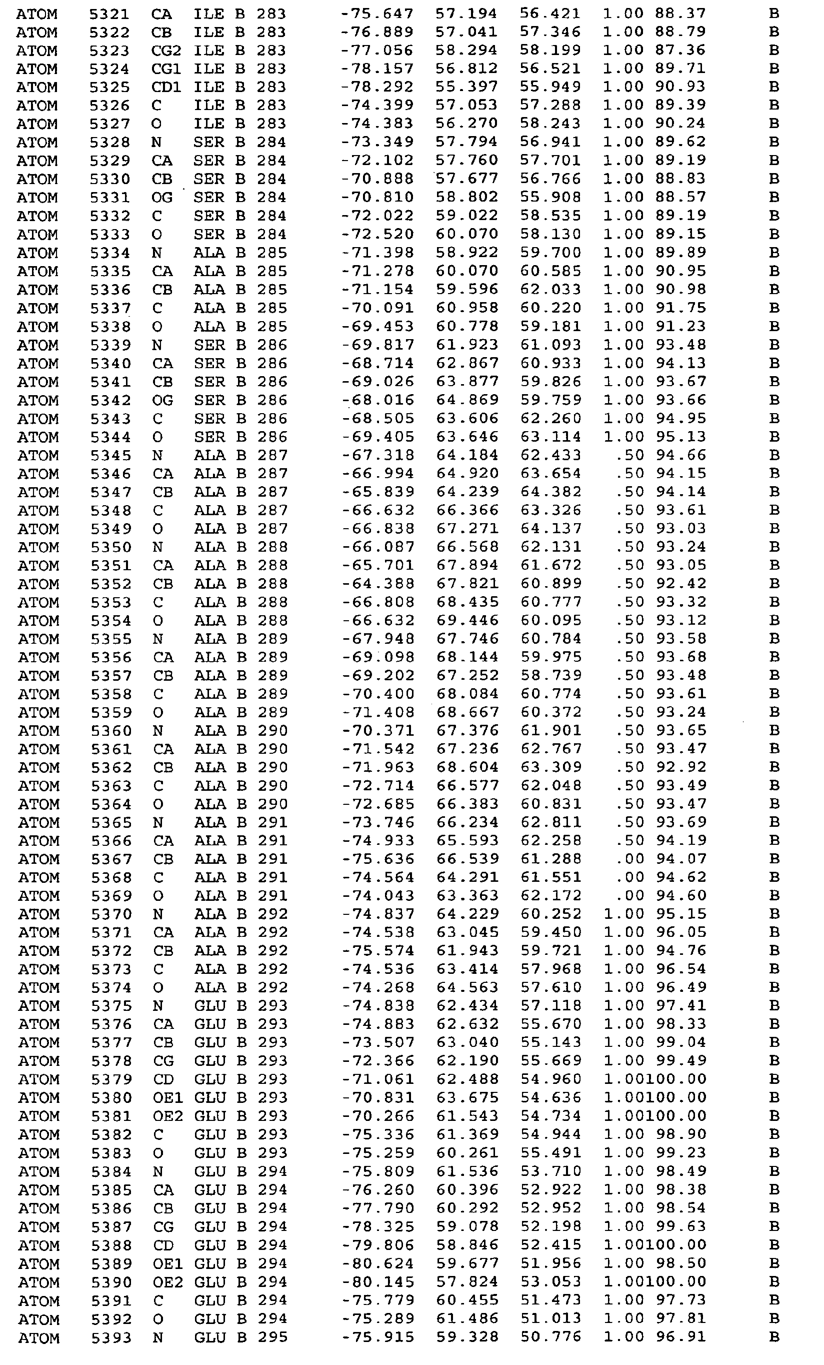

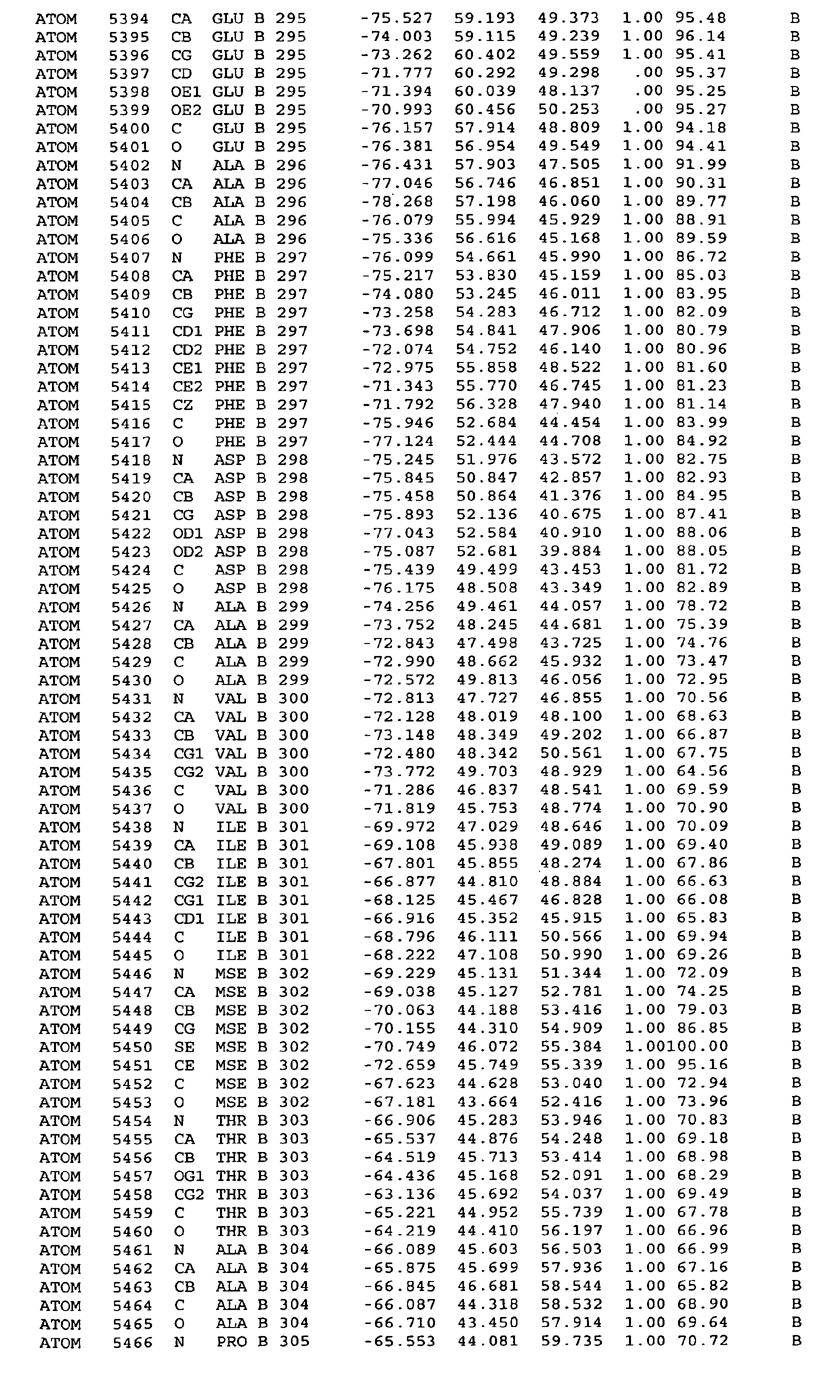

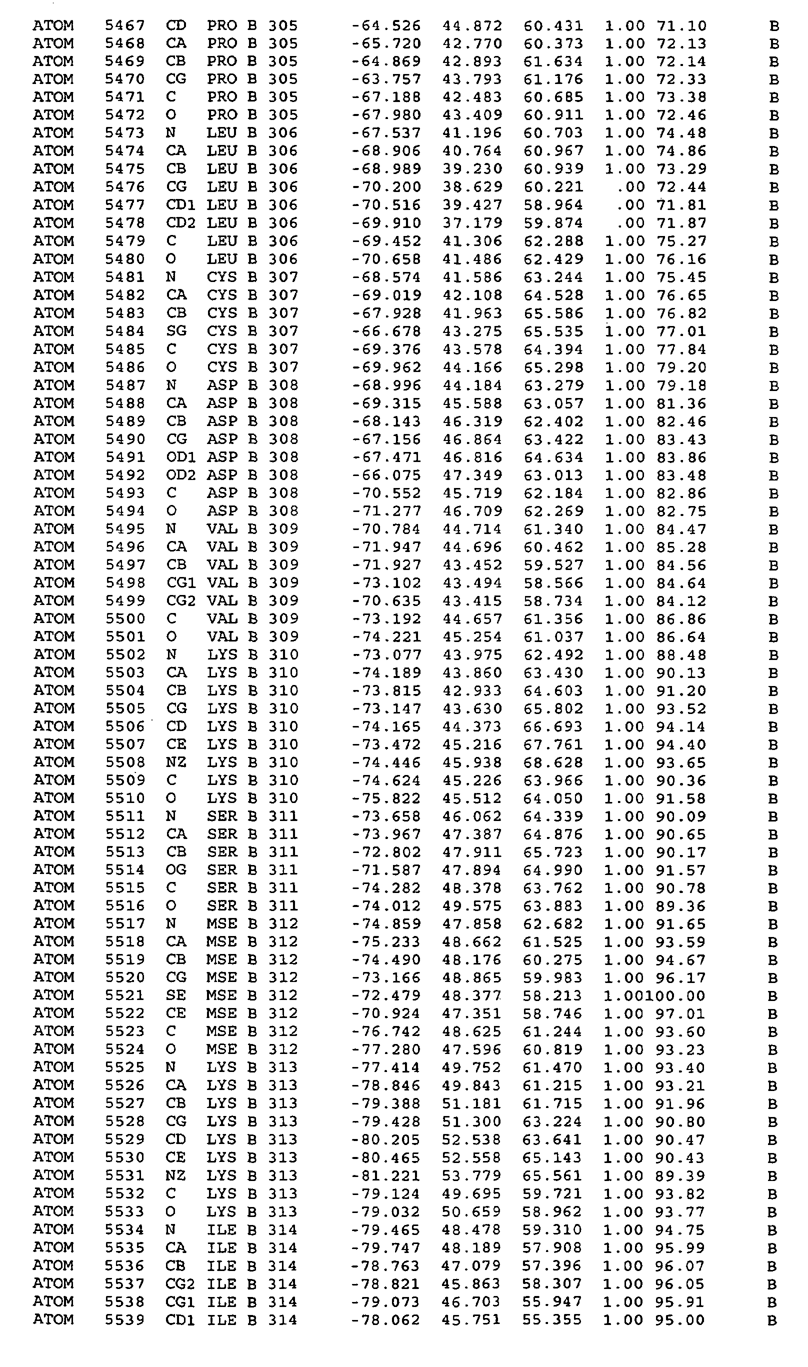

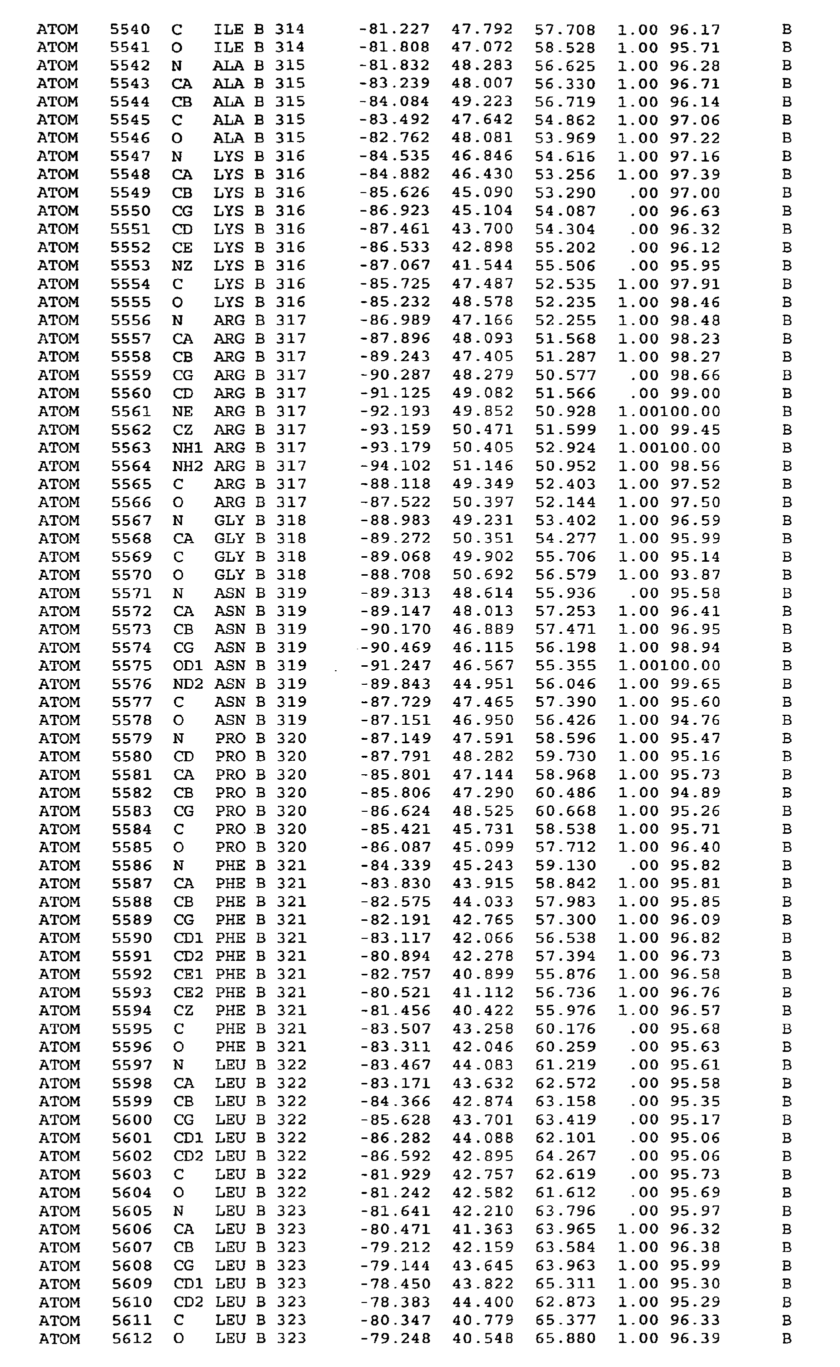

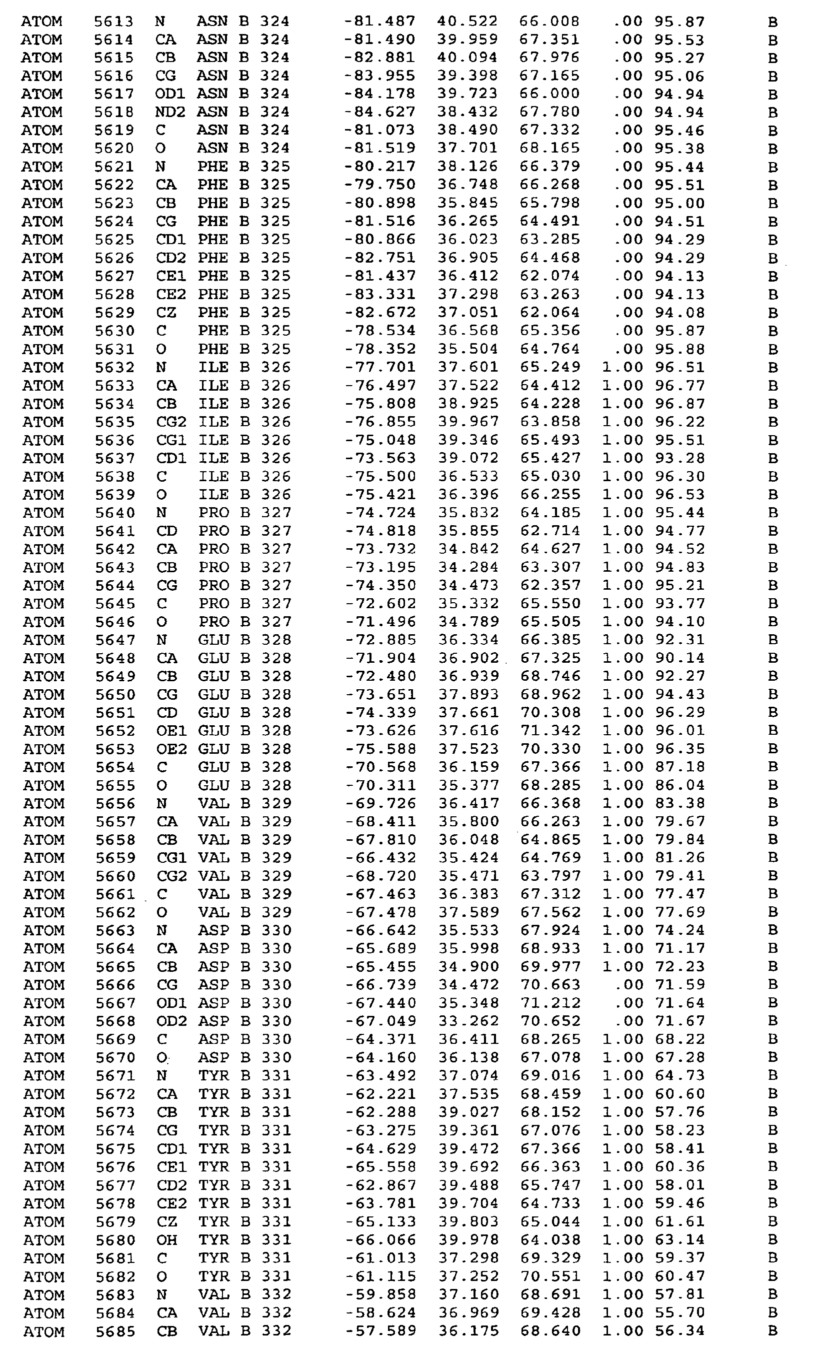

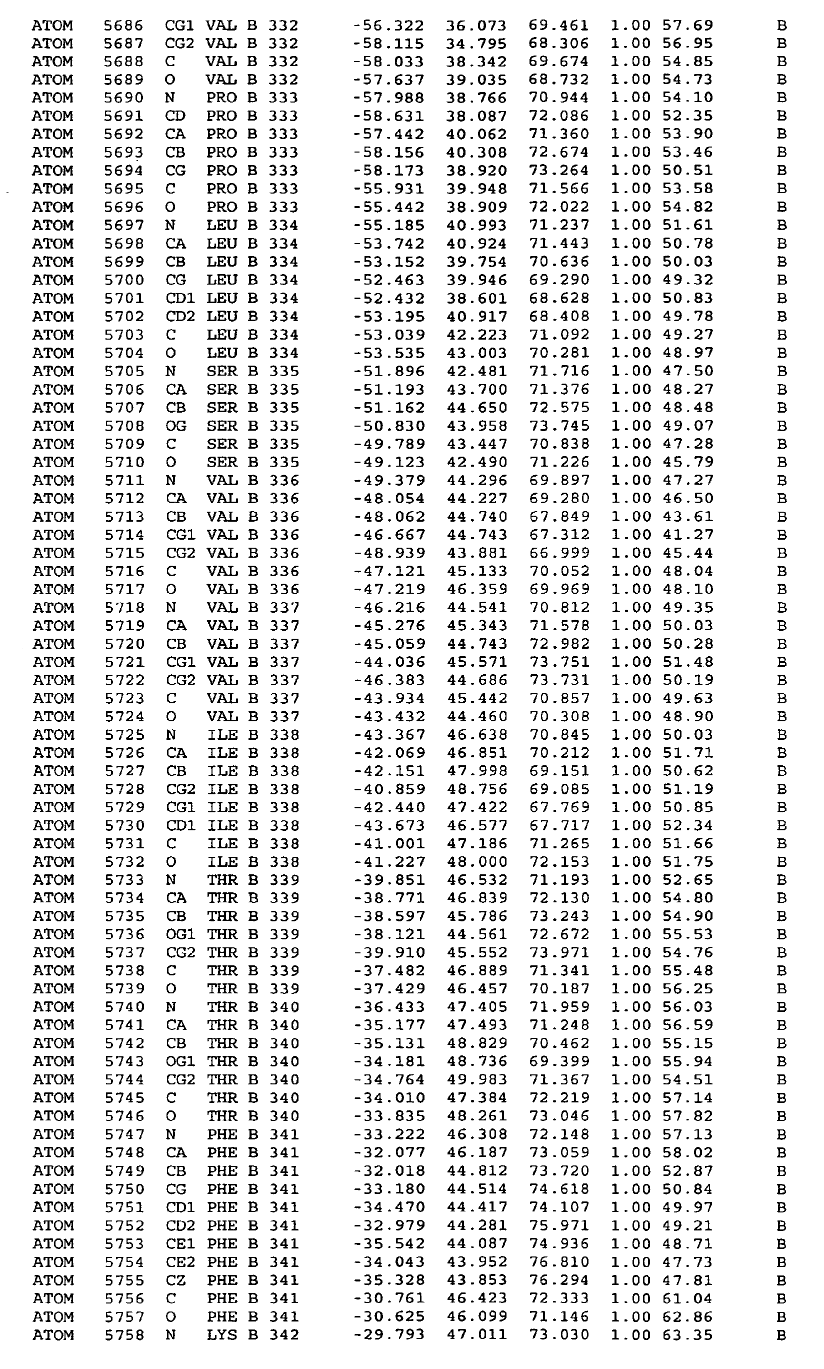

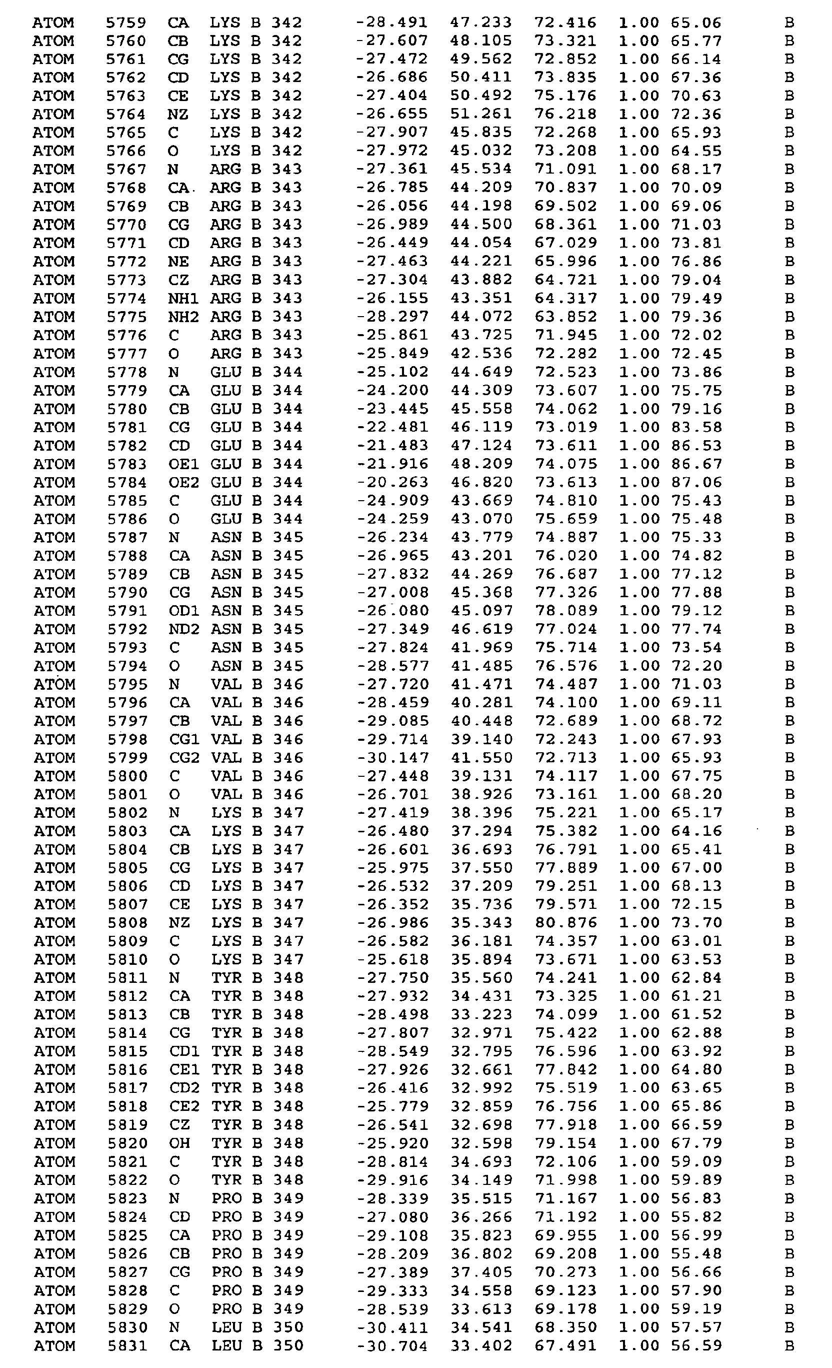

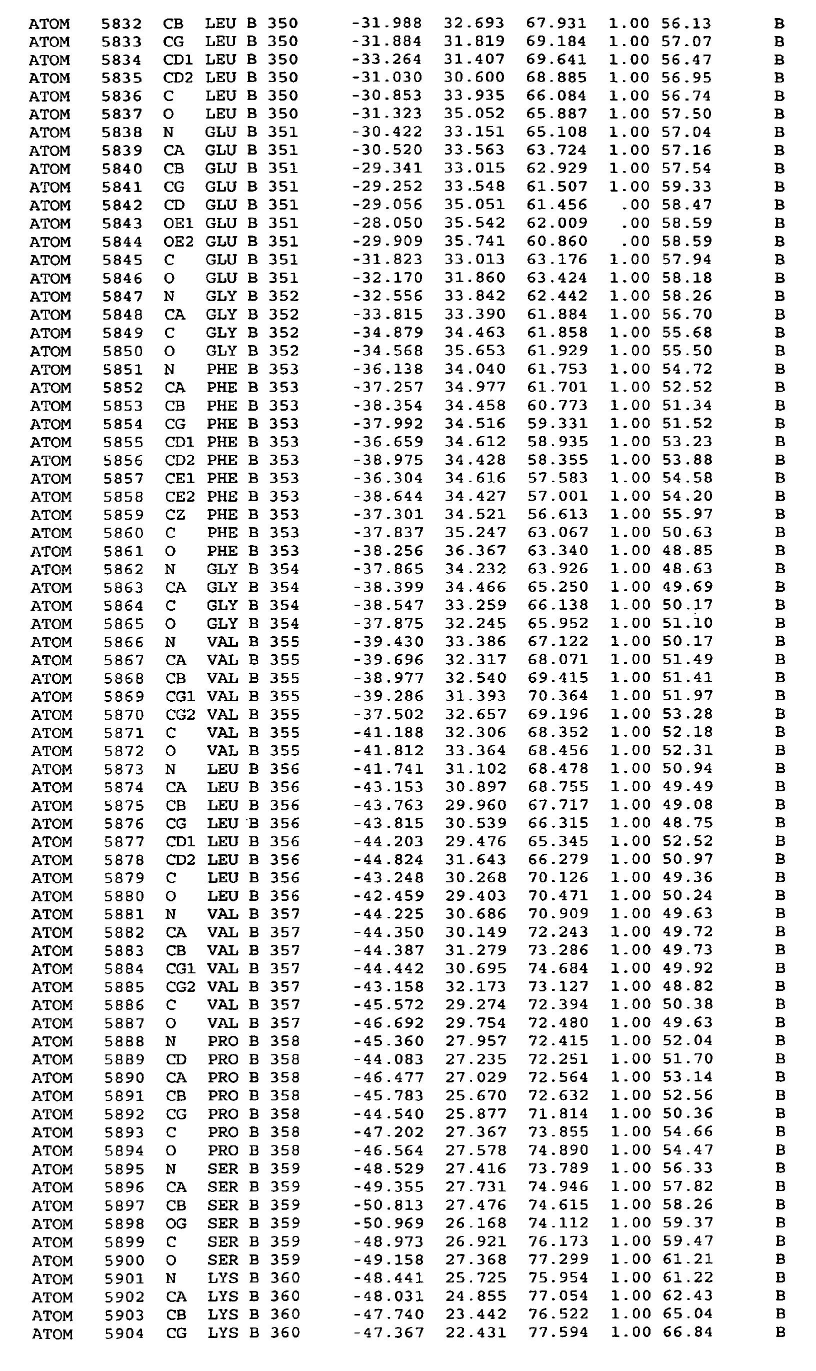

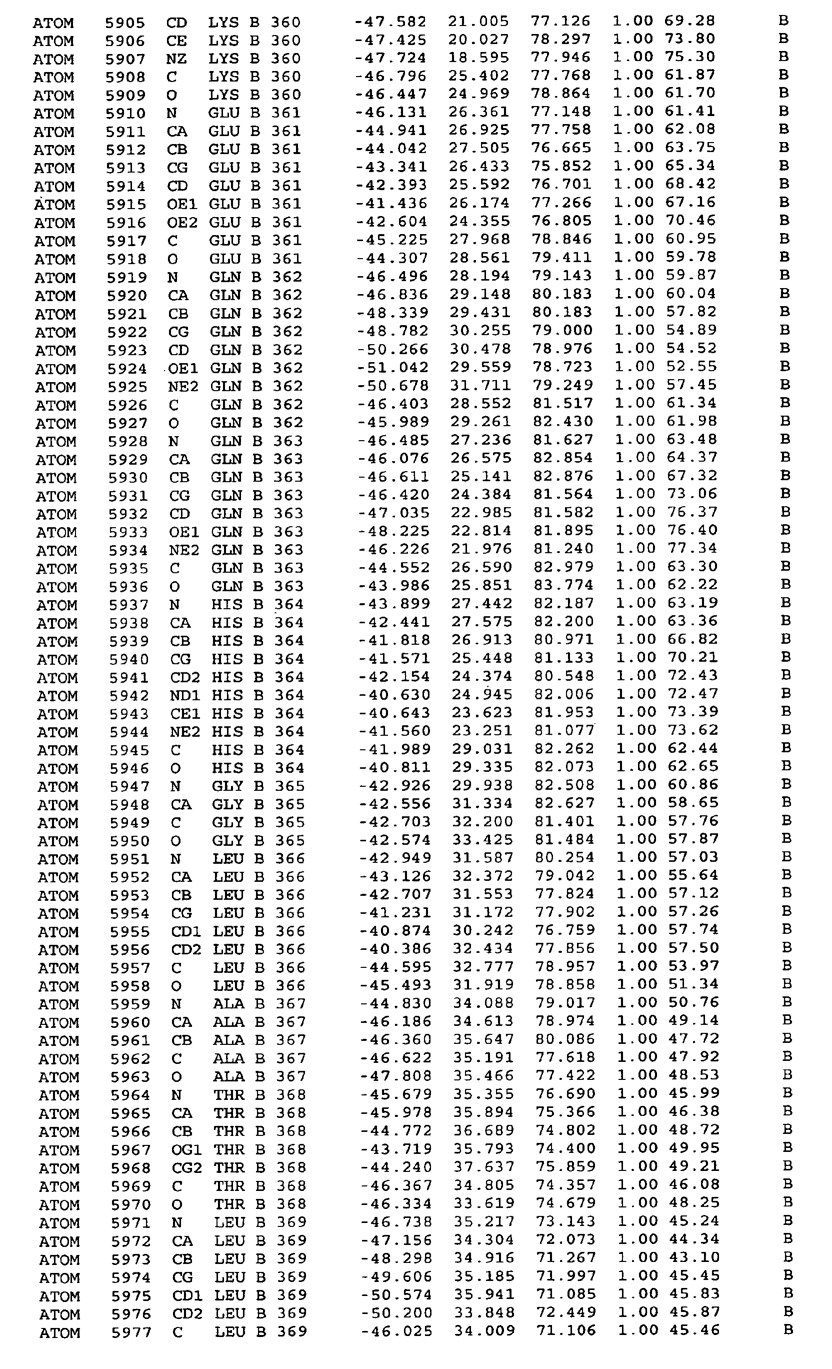

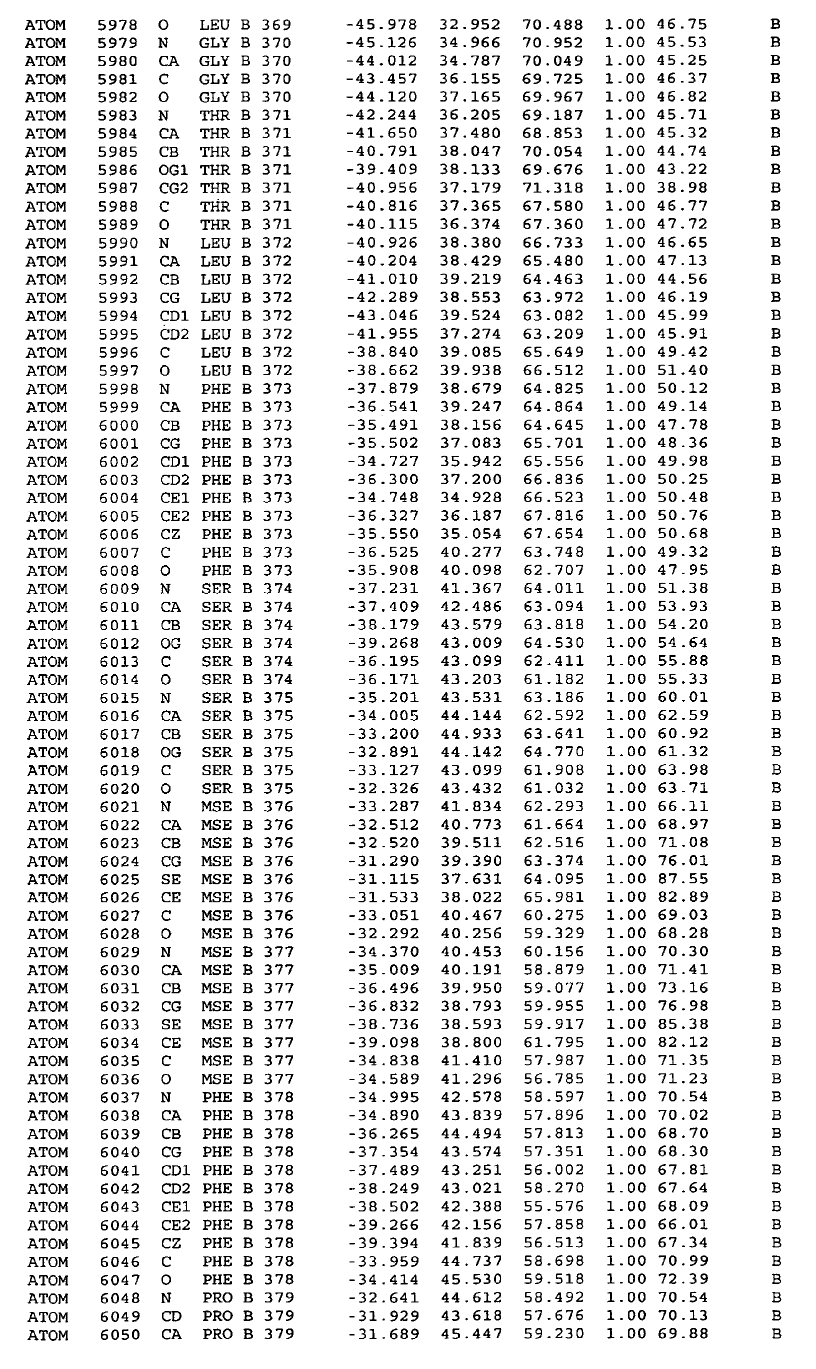

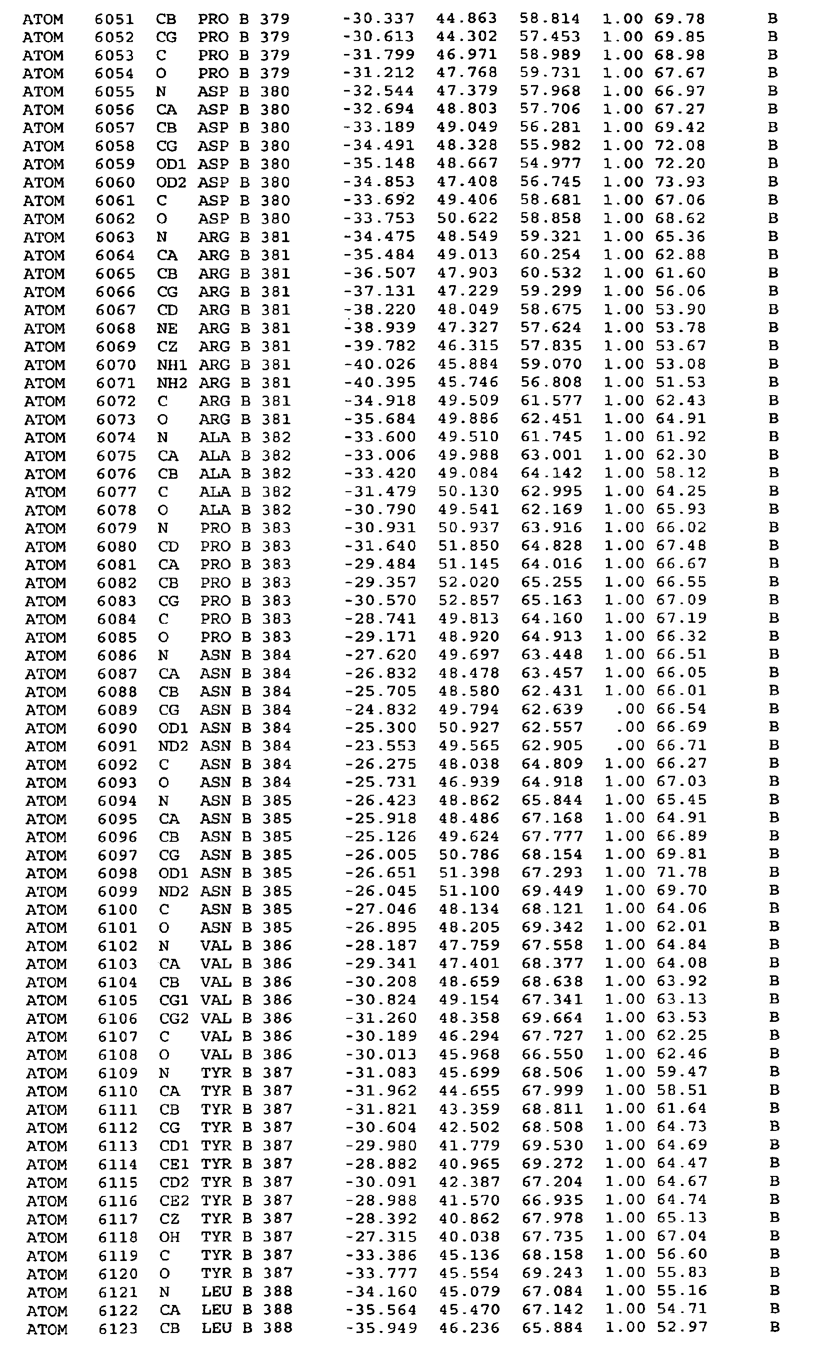

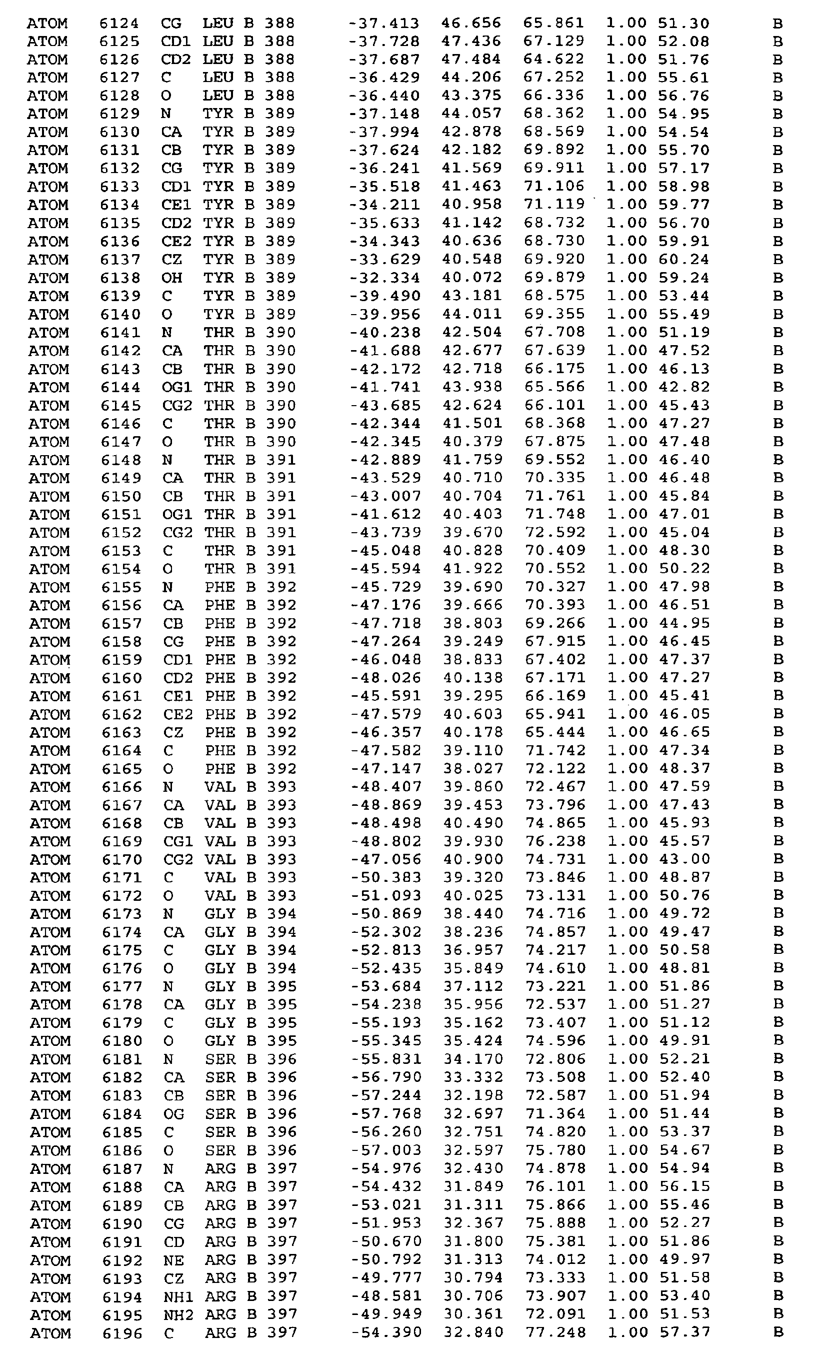

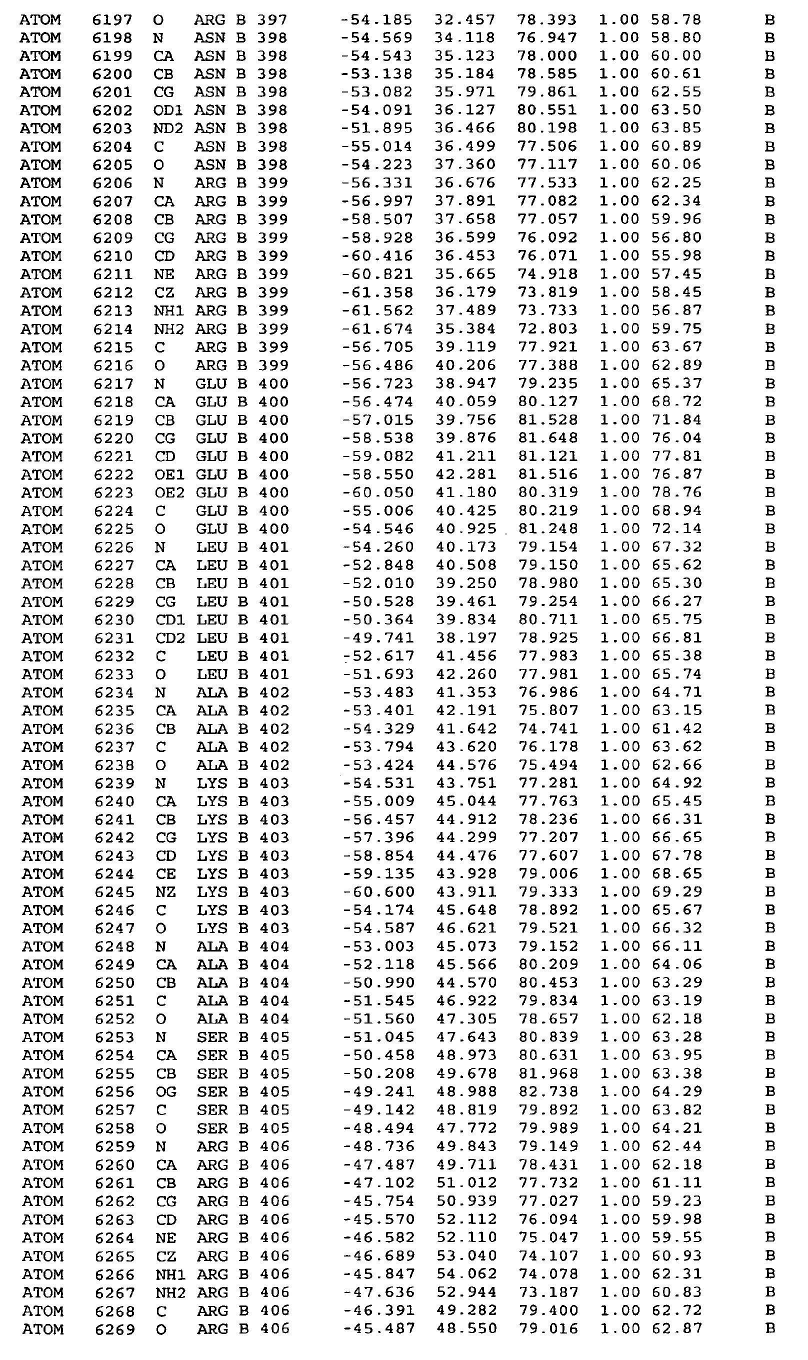

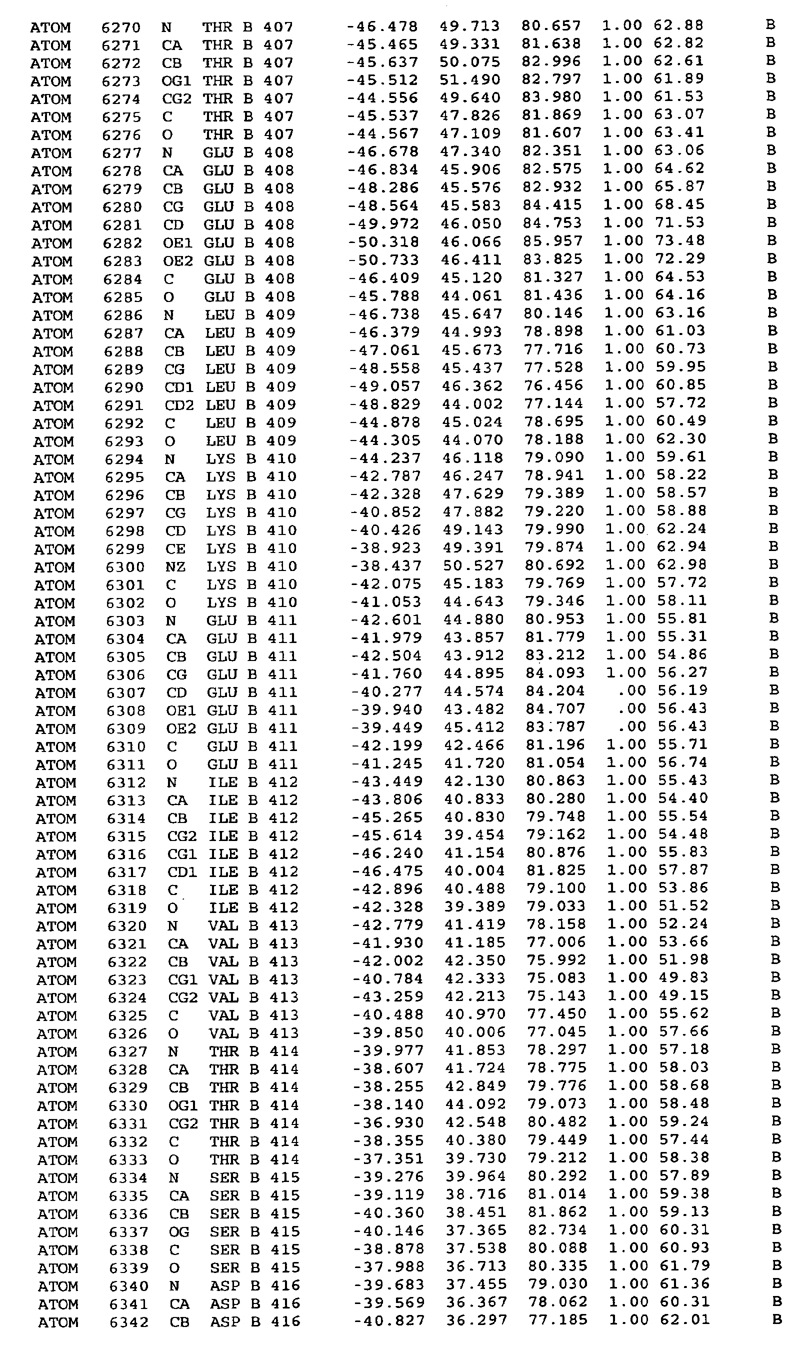

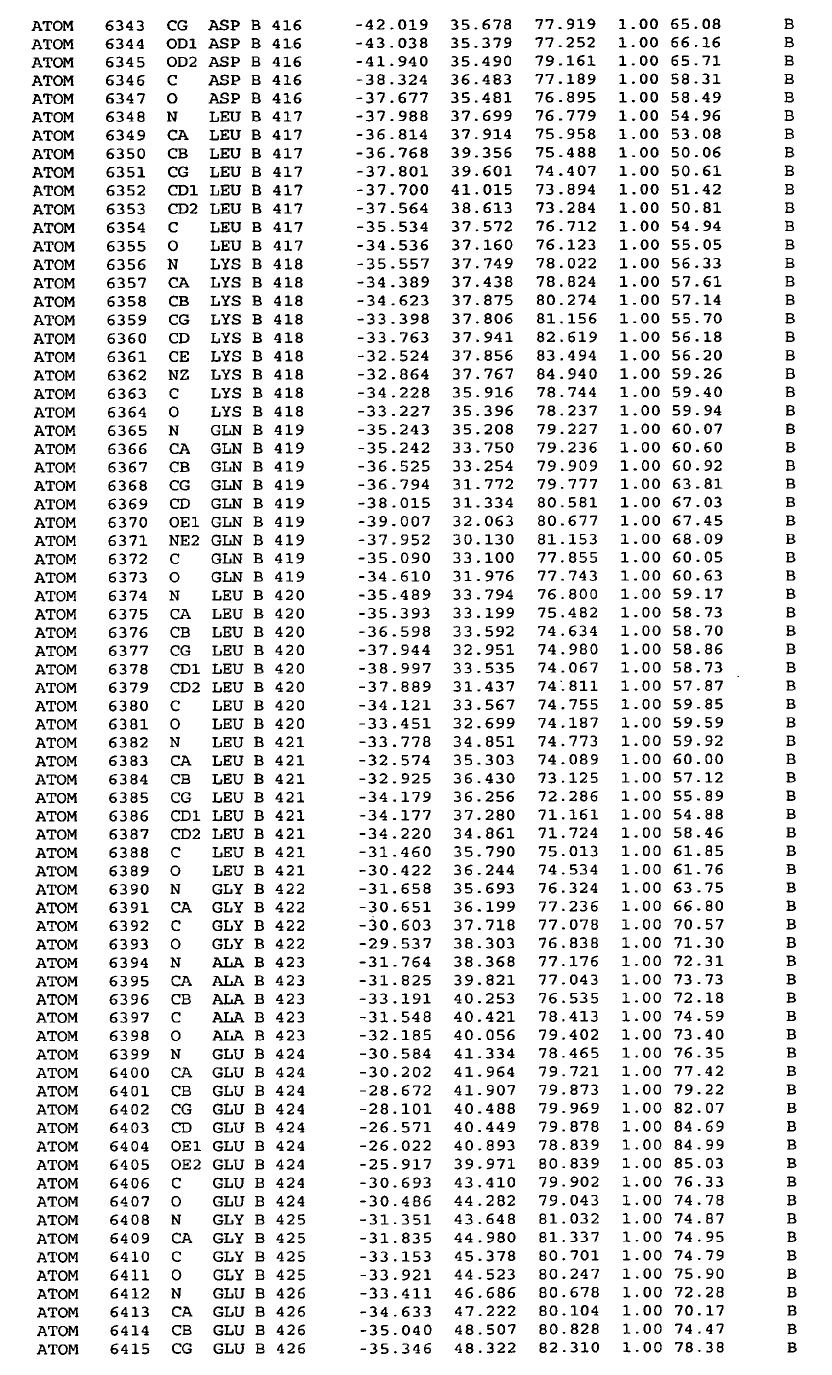

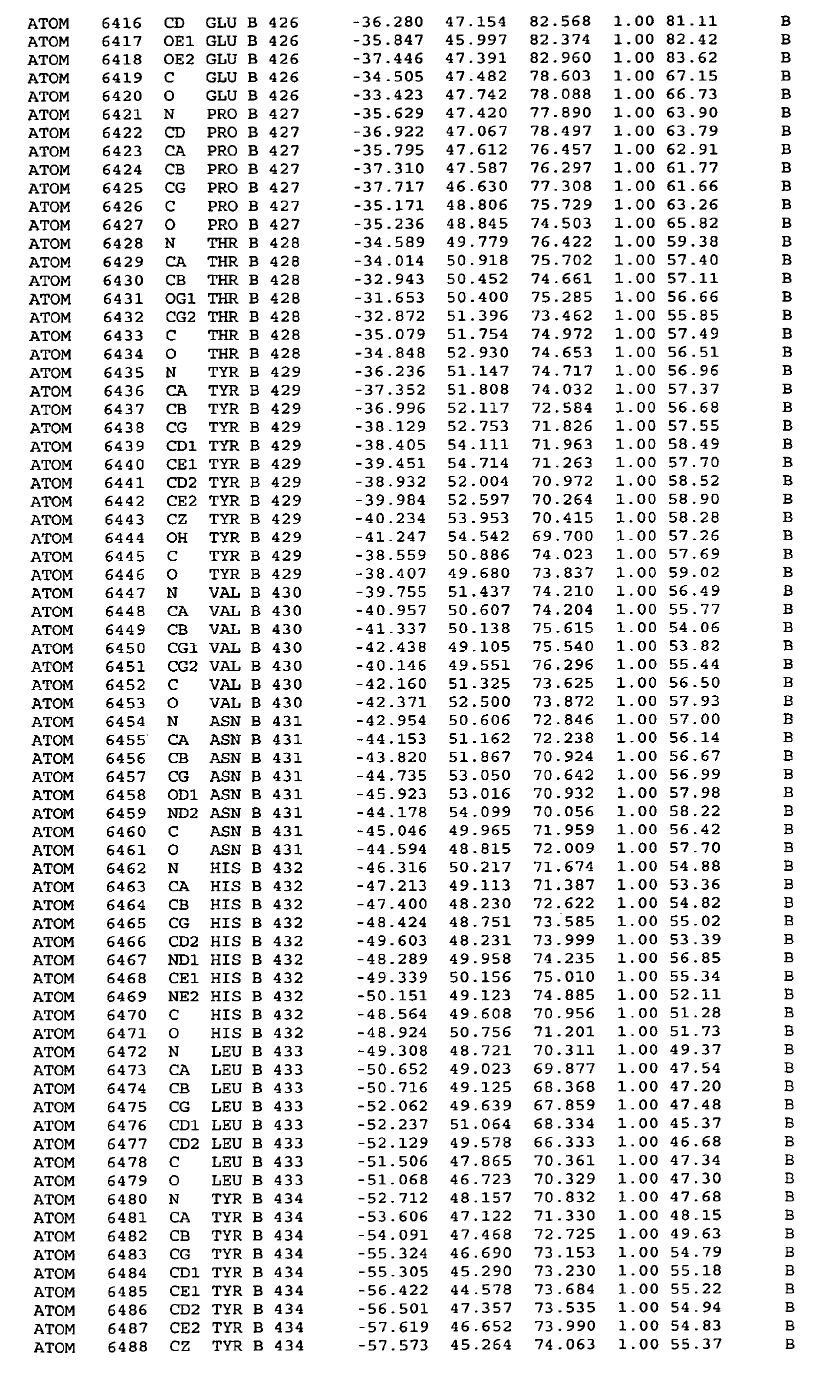

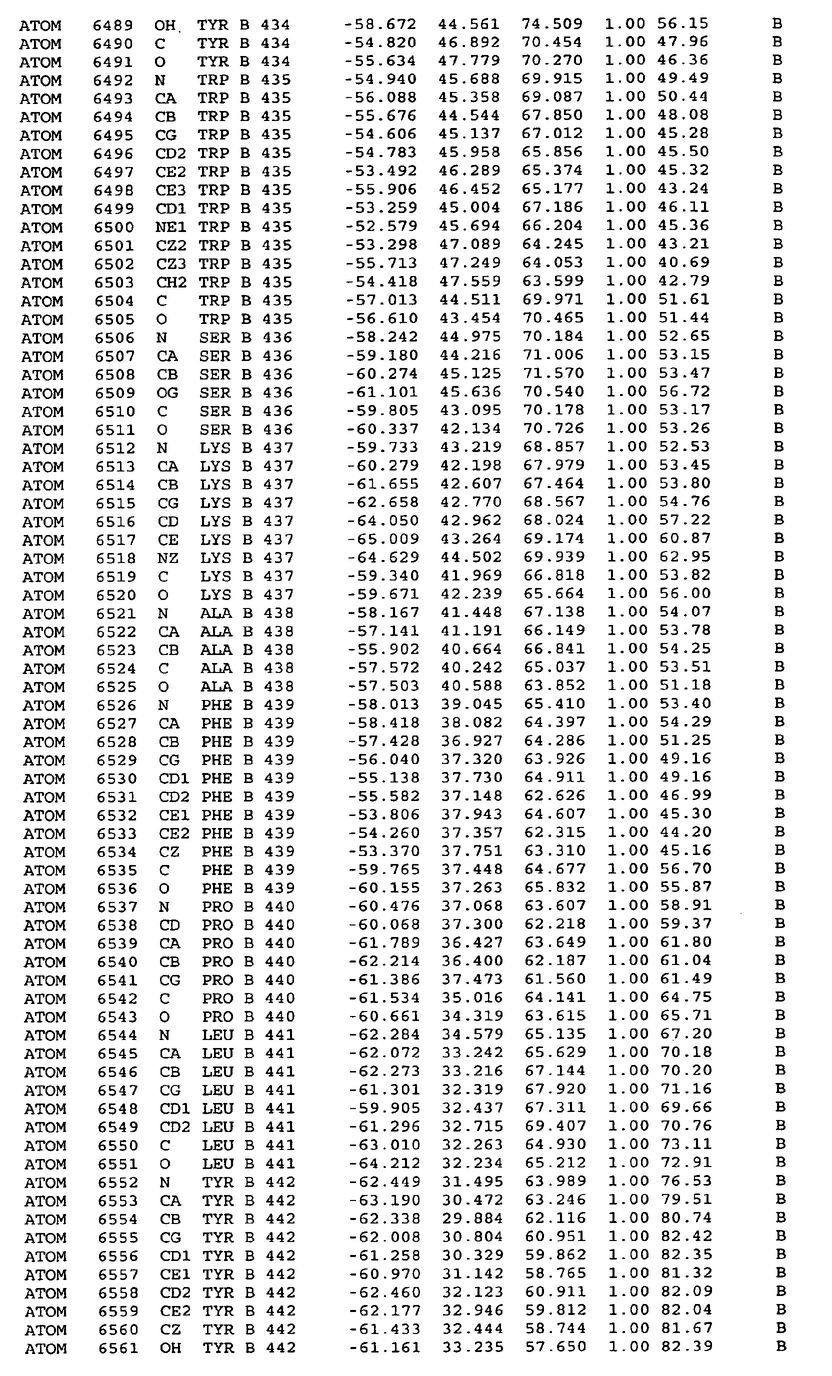

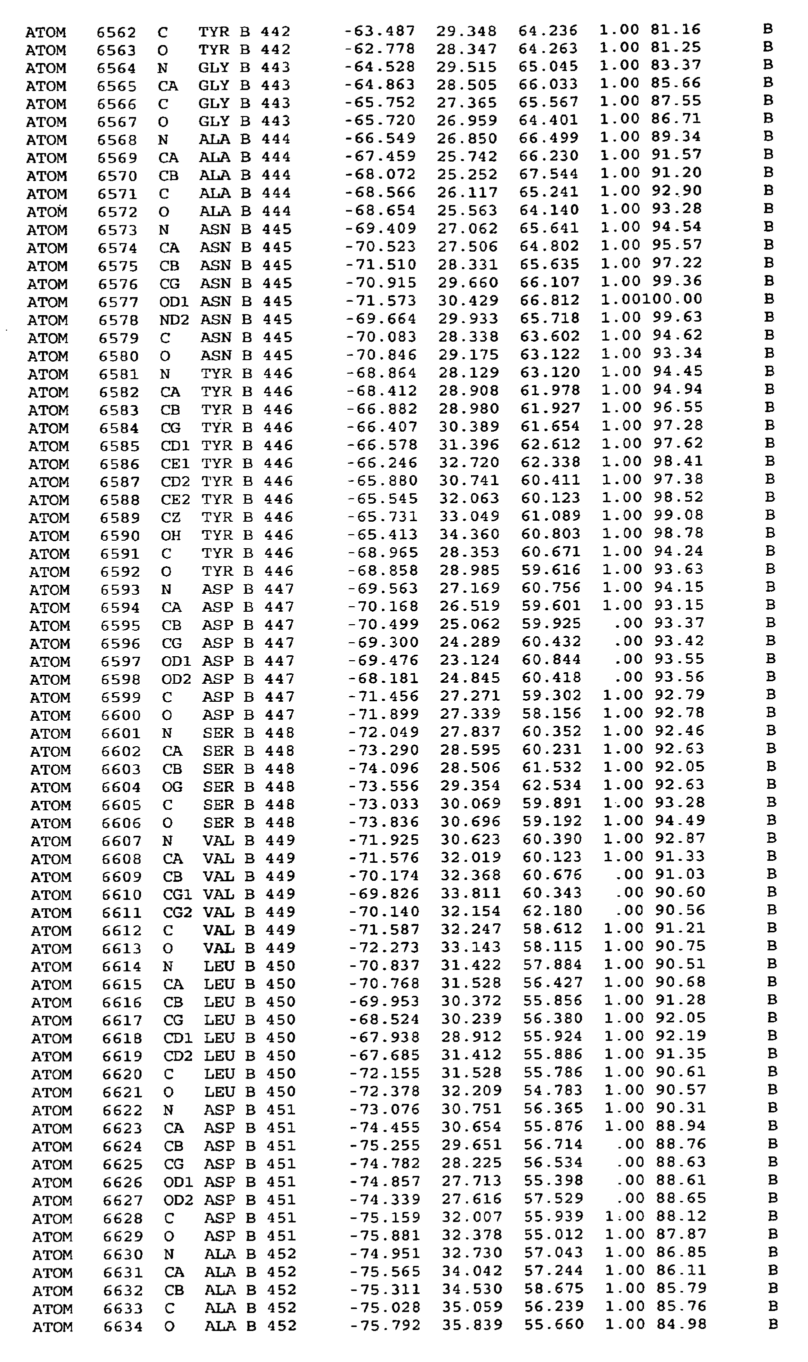

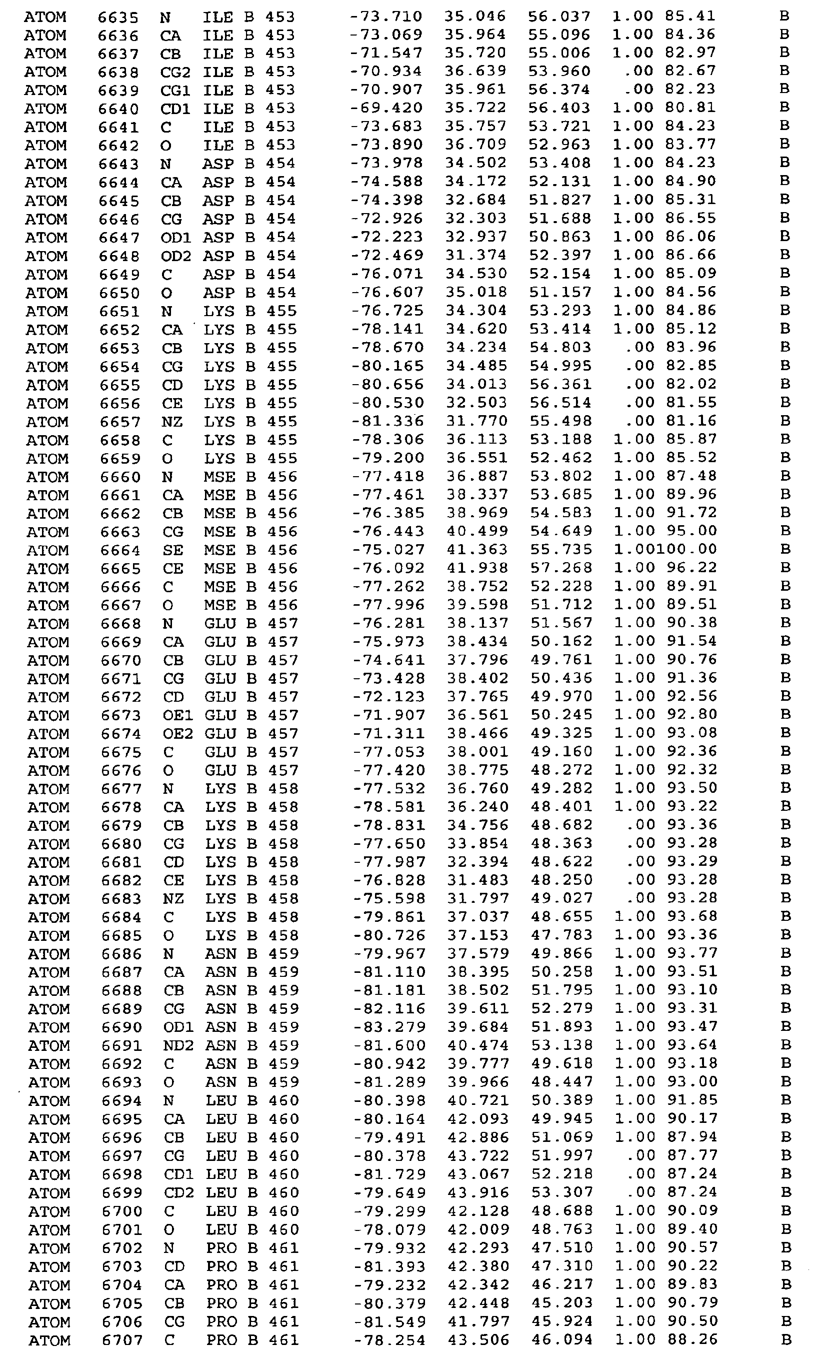

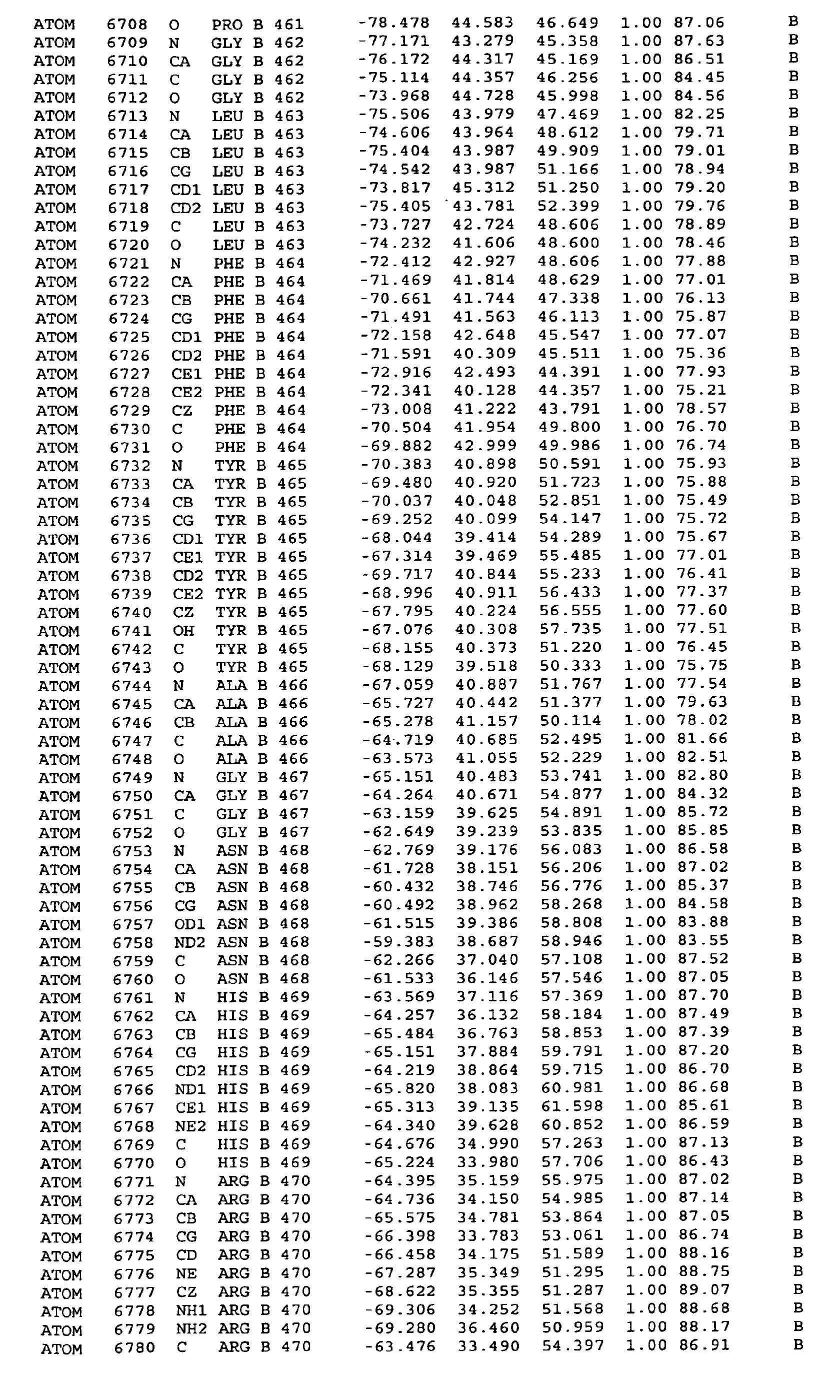

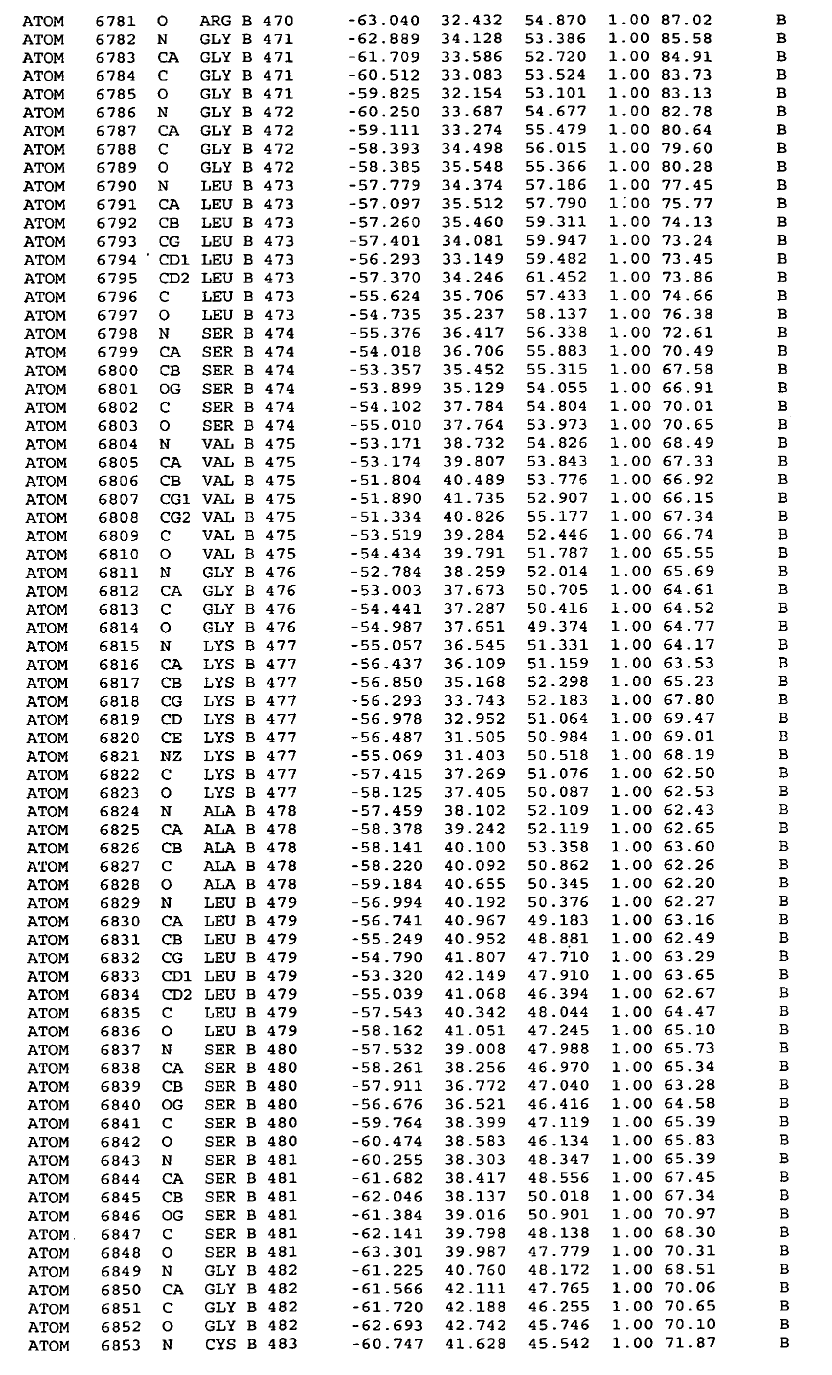

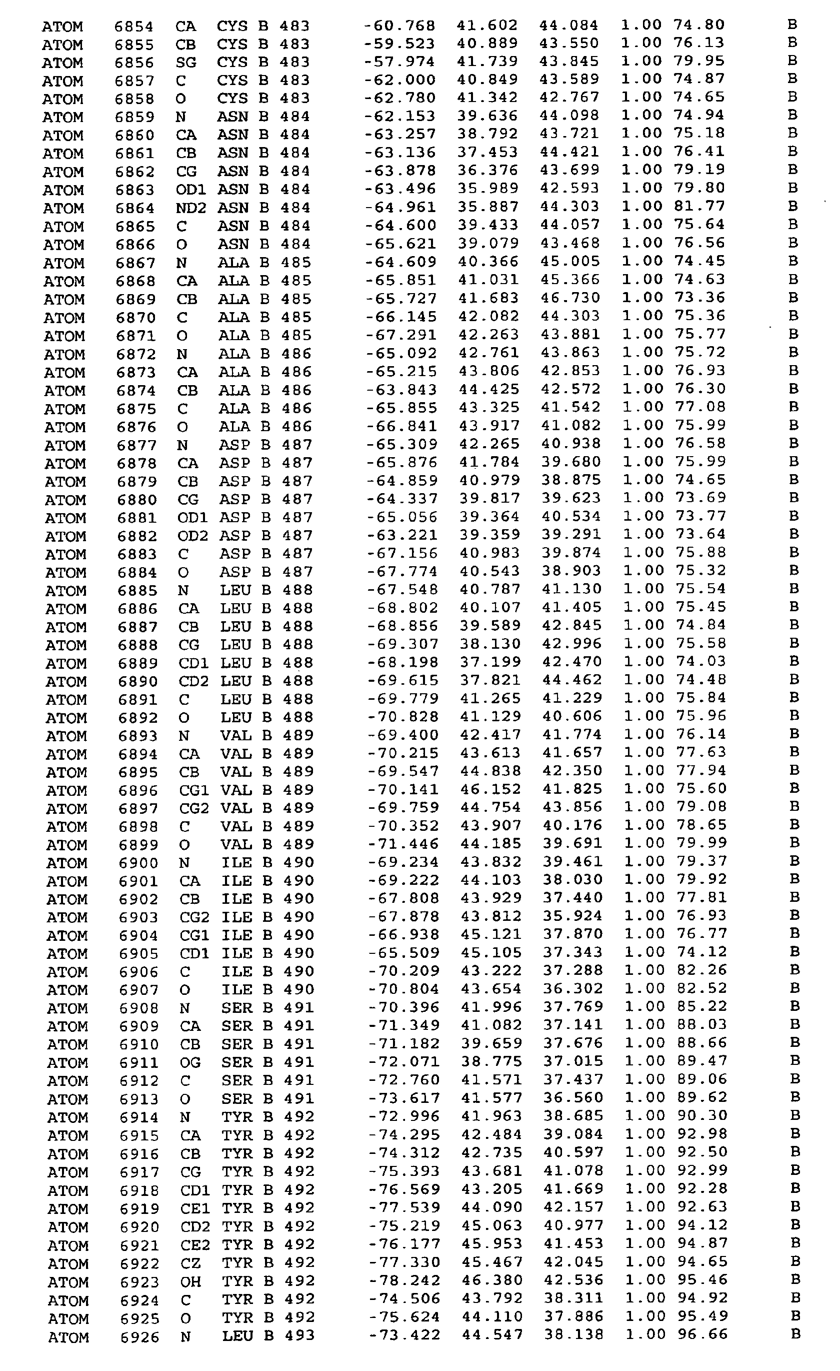

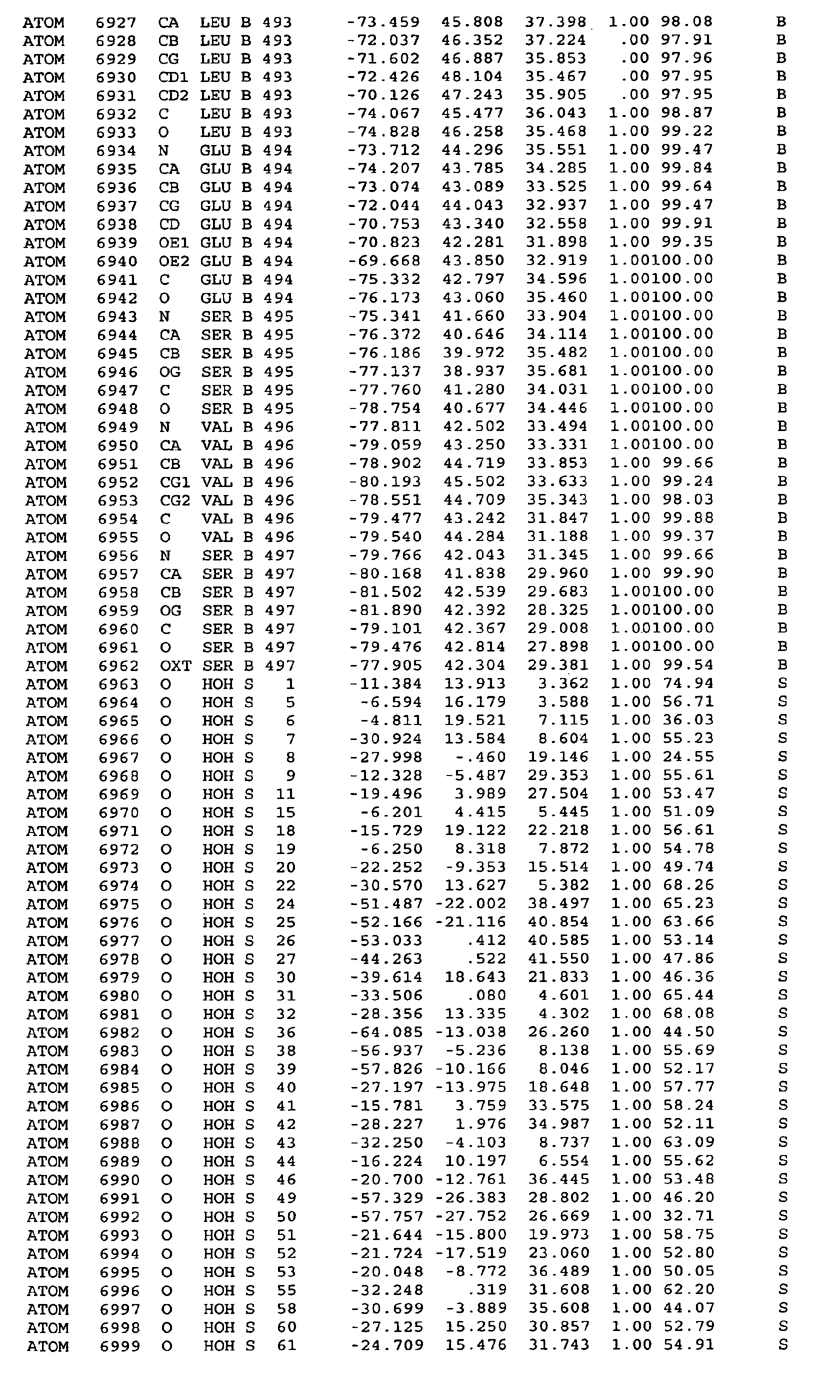

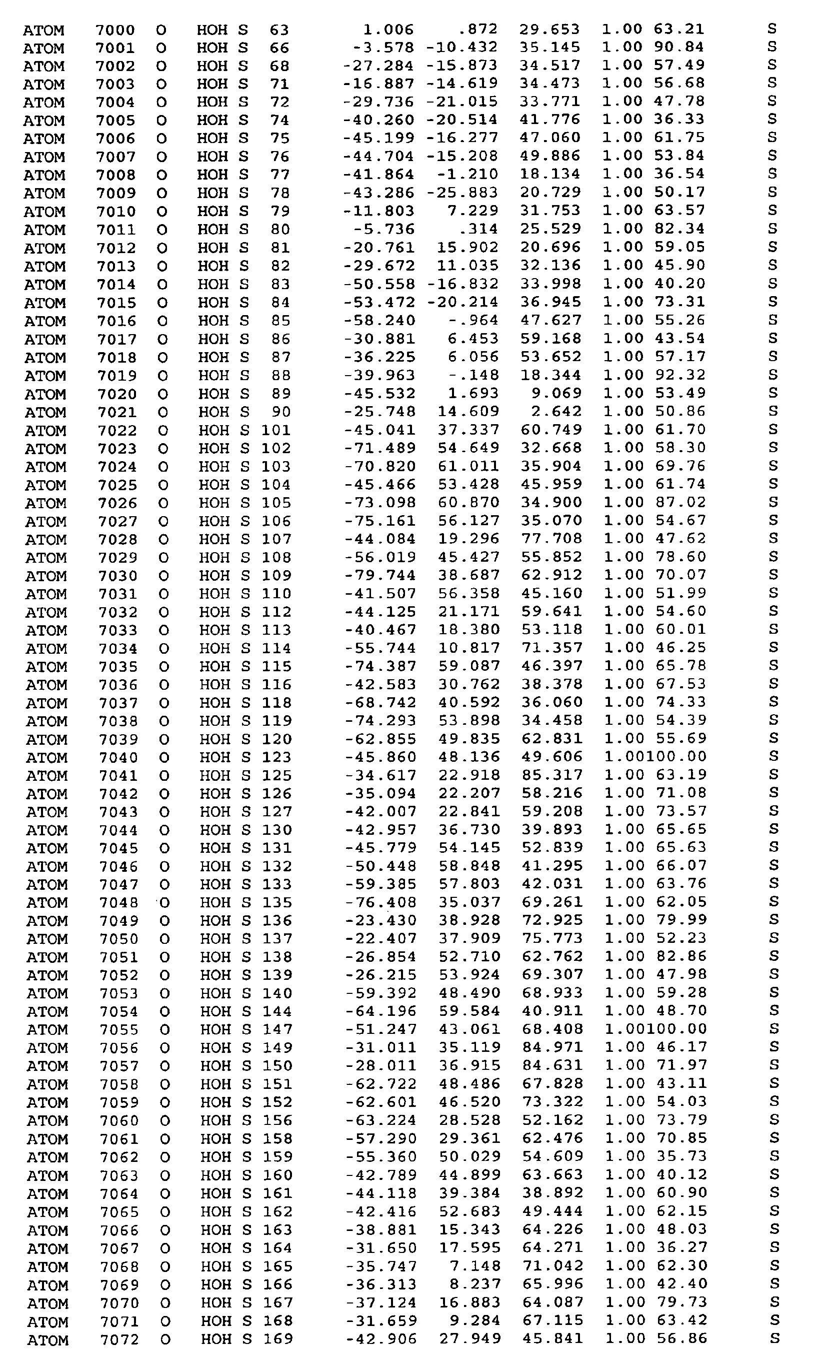

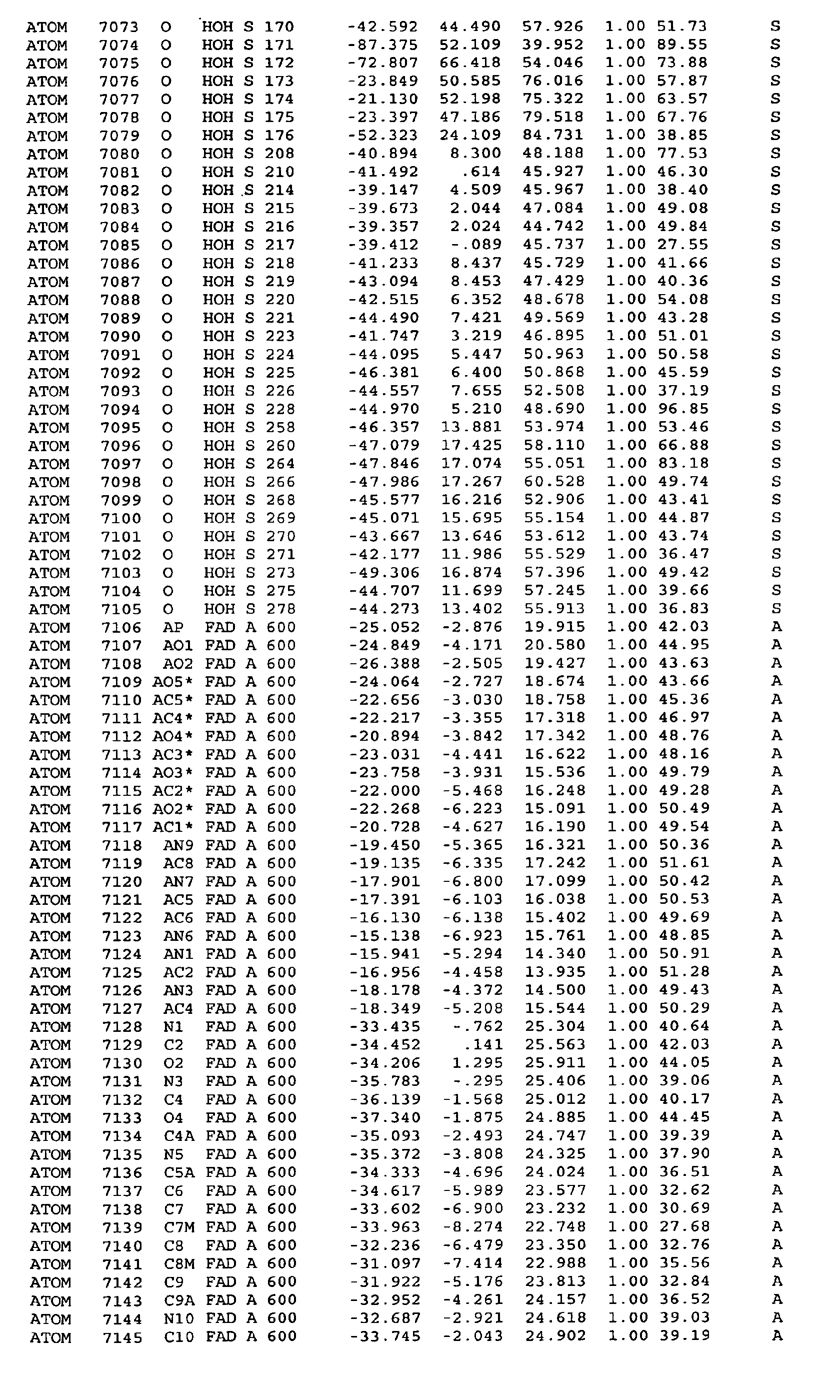

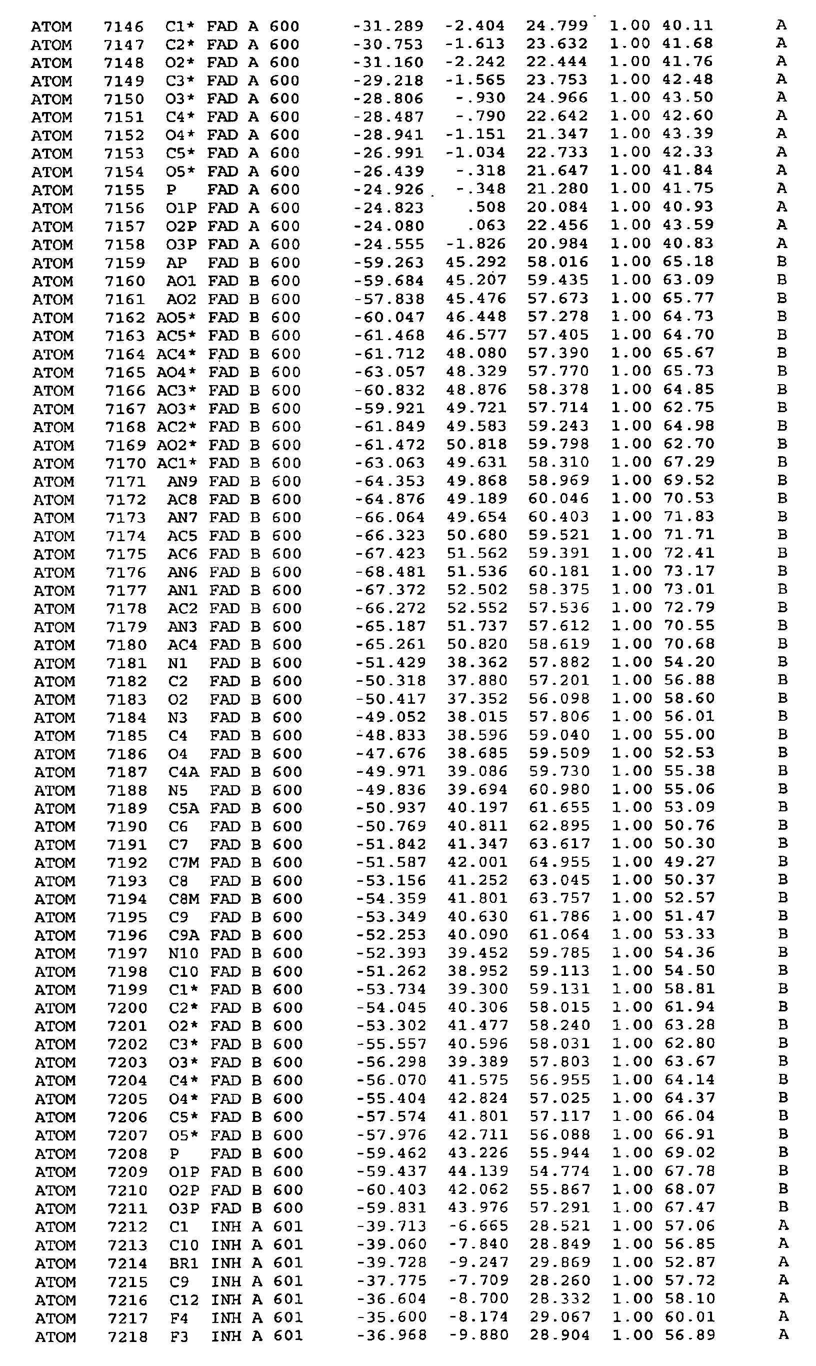

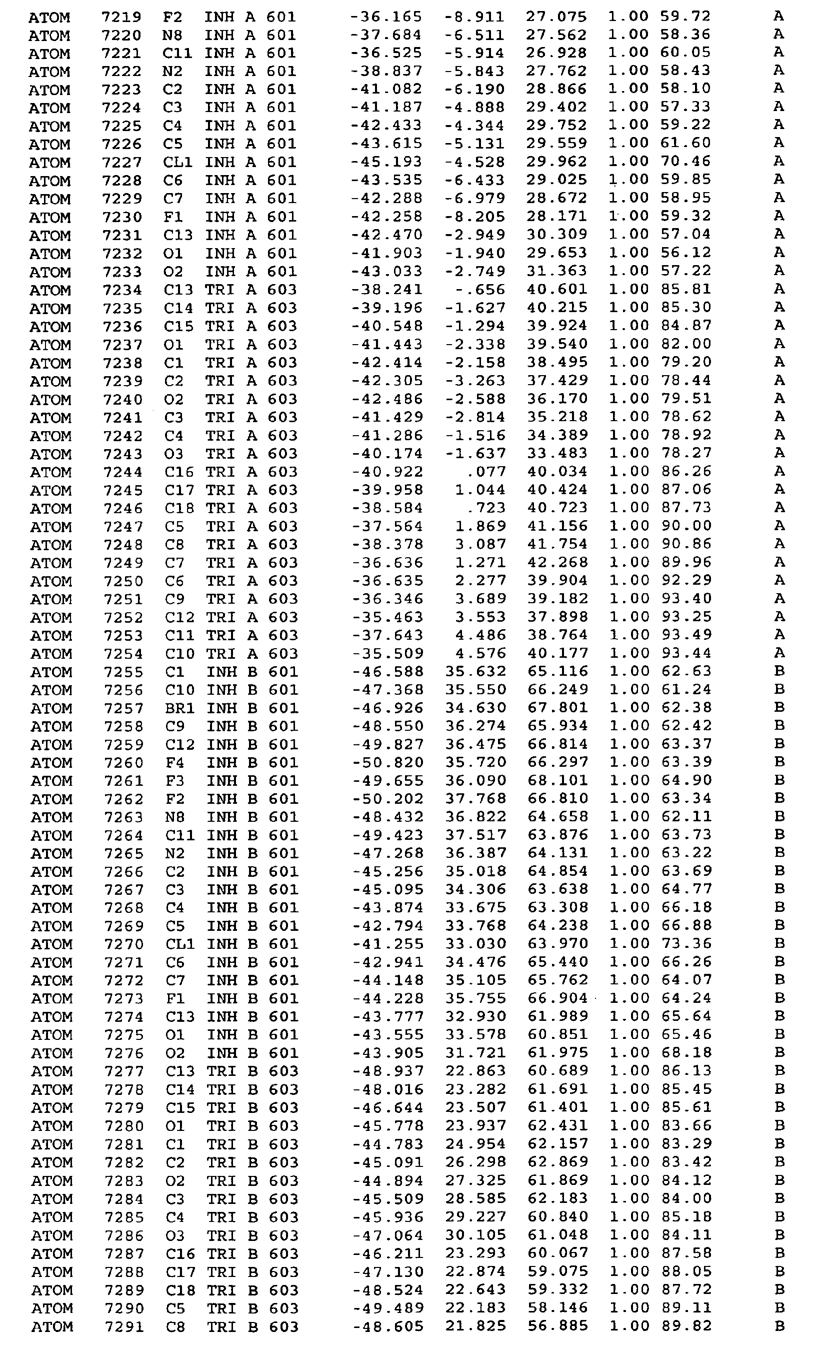

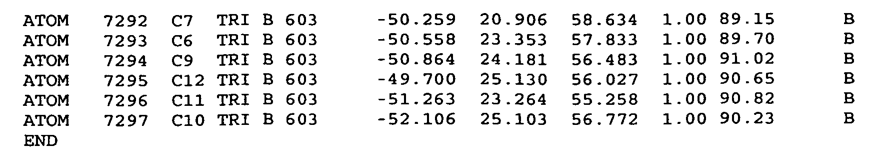

### Literatur:

1. Arnould S., Takahashi M. & Camadro J. M. (1999): Acylation stabilizes a proteaseresistant conformation of protoporphyrinogen oxidase, the molecular target of diphenyl ether-type herbicides. Proc. Natl. Acad. Sci. USA 96, 14825-14830.
2. Binda C. et al. (1999): A 30 angstrom long U-shaped catalytic tunnel in the crystal structure ofpolyamine oxidase. Structure 7, 265-276.
3. Binda C., Newton-Vinson P., Hubalek F., Edmondson D. E. & Mattevi A. (2002): Structure of human monoamine oxidase B, a drug target for the treatment of neurological disorders. Nature Struct. Biol. 9, 22-26 .
4. Blundel, T., Carney, D., Gardner, S., Hayes, F., Howlin, B., Hubbard, T., Overington, J., Singh, D.A., Sibanda, B.L., und Sutcliffe, M. (1988): Knowledge-based protein modelling and design. Eur. J. Biochemo 172, 513-520.
5. Böhm H.J. (1992): LUDI: Rule-Based Automatic Design of New Substituents for Enzyme Inhibitor Leads. J. Comp.-Aided Mol. Design 6, 593-606.
6. Brünger et al. (1998): Recent developments for the efficient crystallographic refinement of macromolecular structures. Current Opinion in Structural Biology 8, 606-611.
7. Brünger A.T., Adams P.D., Clore G.M., DeLano W.L., Gros P., Grosse-Kunstleve R.W., Jiang J.-S., Kuszewski J., Nilges M., Pannu Navraj S., Read R.J., Rice L.M., Simonson T., Warren G.L. (1998): Crystallography & NMR System: a new software suite for macromolecular structure determination. Acta Crystallographica, Section D, 54 905-921.
8. Fortelle E. de la & Bricogne G. (1997). Maximum-Likelihood Heavy-Atom Parameter Refinement for Multiple Isomorphous Replacement and Multiwavelength Anomalous Diffraction Methods. In Methods in Enzymology, Vol. 276. Macromolecular Crystallography Part A. C.W. Carter and R.M. Sweet ed. Academic Press, San Diego, pp. 472-494.
9. Greer, J. (1985): Model structure for the Inflammatory Protein C5a. *Science* 228, 1055.
10. Jones T.A., Zou J.Y., Cowan S.W. & Kjeldgaard M. (1991): Improved methods for building protein models in electron density maps and the location of errors in these models. Acta Cryst. A 47, 110-119.
11. Jones G. et al. (1997): Development and Validation of a Genetic Algorithm for Flexible Docking. J. Mol. Biol. 267, 727-748.
12. Kabsch W. (1999): Automatic Processing of Rotation Diffraction Data from Crystals of Initially Unknown Symmmetry and Cell Constants. J. Appl. Cryst. 32, 120-124.
13. Lermontova I., Kruse E., Mock H. P. & Grimm B. (1997): Cloning and characterization of a plastidal and a mitochondrial isoform of tobacco protoporphyrinogen IX oxidase. Proc. Natl. Acad. Sci. USA 94, 8895-8900.
14. Loll P. J., Picot D. & Garavito R. M. (1995): The Structural Basis of Aspirin Activity Inferred from the Crystal-Structure of Inactivated Prostaglandin H-2 Synthase. Nature Structural Biology 2, 637-643.
15. Mizutani, H. et al. (1996): Three-dimensional structure of porcine kidney D-amino acid oxidase at 3.0 angstrom resolution. J. Biochem. 120, 14-17.
16. Moon J.B. & Howe W.J. (1991): Computer design of bioactive molecules: a method for receptor-based de novo ligand design. Proteins 11, 314-328.
17. Navaza (1994), AMoRe: an Automated Package for Molecular Replacement. Acta Crystallographica, A50, 157-163.
18. Nishibata Y. & Itai A. (1991): Automatic creation of drug candidate structures based on receptor structure. Starting point for artificial lead generation. Tetrahedron 47, 8985-8990.
24. Rarey M. et al. (1996): Predicting Receptor-Ligand Interactions by an Incremental Construction Algorithm. J. Mol. Biol. 261, 470-489.
25. Weeks C. M. & Miller R. (1999): The design and implementation of SnB version 2.0. J. Appl. Crystall. 32, 120-124.

## Patentansprüche

1. Ein Kristall einer Protoporphyrinogen-IX-Oxidase.

2. Ein Kristall gemäß Anspruch 1, **dadurch gekennzeichnet, dass** er die Raumgruppe C222₁ besitzt.

3. Ein Kristall gemäß Anspruch 1 oder 2, **dadurch gekennzeichnet, dass** er die Zellkonstanten 119,1, 147,3 und 127,0 Å aufweist.

4. Ein Kristall gemäß einem der Ansprüche 1 bis 3, **dadurch gekennzeichnet, dass** es sich um den Kristall einer Protoporphyrinogen-IX-Oxidase aus *Nicotiana tabacum* handelt.

5. Ein Kristall gemäß einem der Ansprüche 1 bis 4, **dadurch gekennzeichnet, dass** die Protoporphyrinogen-IX-Oxidase im Komplex mit einem niedermolekularen Molekül vorliegt.

6. Ein Kristall gemäß einem der Ansprüche 1 bis 5, **dadurch gekennzeichnet, dass** die Protoporphyrinogen-IX-Oxidase im Komplex mit 4-Brom-3-(5'-carboxy-4'-chlor-2'-fluorphenyl)-1-methyl-5-trifluormethyl-pyrazol vorliegt.

7. Ein Kristall gemäß einem der Ansprüche 1 bis 6, **dadurch gekennzeichnet, dass** er die in Tabelle 2 definierten Strukturkoordinaten besitzt.

8. Ein Kristall gemäß einem der Ansprüche 1 bis 7, **dadurch gekennzeichnet, dass** er eine Ligandenbindenische umfasst, welche durch die folgenden Aminosäuren im 4,5 Å Abstand vom gebundenen Liganden definiert ist: Arg⁹⁸, Ala¹⁷⁴, Gly¹⁷⁵, Thr¹⁷⁶, Cys¹⁷⁷, Gly¹⁷⁸, Leu ³³⁴, Phe³⁵³, Gly³⁵⁴, Val³⁵⁵, Leu³⁵⁶, Leu³⁶⁹, Gly³⁷⁰, Thr³⁷¹, Leu³⁷², Phe³⁹², Phe⁴³⁹.

9. Ein Kristall mit einer im Wesentlichen gleichen Struktur wie in Anspruch 7 definiert, dadaurch **gekennzeichnet**, dass zumindest eine Aminosäure gemäß SEQ ID NO:1 durch eine andere Aminosäure ersetzt wurde und/oder eine Aminosäure deletiert und/oder eine Aminosäure eingefügt wurde.

10. Ein Verfahren zum Kristallisieren eines Seleno-Methionin Derivats einer PPO umfassend die folgenden Schritte:
(a) Rekombinante Herstellung einer PPO in einer Wirtszelle in Gegenwart von Seleno-Methionin,
(b) Isolieren der PPO aus den Wirtszellen
(c) Reinigen der PPO, und
(d) Herstellen eines Kristalls gemäß einem der Ansprüche 1 bis 9.

11. Ein Verfahren gemäß Anspruch 10, **dadurch gekennzeichnet, dass** man den Kristall mittels Dampfdiffusion im sitzendem Tropfen erzeugt.

12. Ein Verfahren gemäß Anspruch 10 oder 11, **dadurch gekennzeichnet, dass** die Kristallisationslösung 10% Polyethylenglykol 1000, 0,1 M Natriumcitrat/HCl, pH 4,0 und 0.2 M Natriumchlorid enthält.

13. Verfahren zur Bestimmung der Struktur einer PPO, welche eine Identität von zumindest 40% zu der Sequenz gemäß SEQ ID NO:1 aufweist, umfassend die folgenden Schritte:
(a) Alignment der Aminosäuresequenz der PPO mit der Aminosäuresequenz gemäß SEQ ID NO:1,
(b) Modellieren der Struktur der übereinstimmenden homologen Regionen der PPO gemäß den Strukturdaten für diese Regionen aus Tabelle 2, und
(c) Bestimmen der Konformation der PPO.

14. Verfahren zum Identifizieren von Liganden einer Protoporphyrinogen-IX-Oxidase, umfassend die folgenden Schritte:
(a) Erstellen einer dreidimensionalen Struktur einer PPO,
(b) Erstellen der dreidimensionalen Struktur eines Kandidatenmoleküls, und
(c) Einpassen der dreidimensionalen Struktur des Kandidatenmoleküls in die dreidimensionale Struktur der PPO, und
(d) Auswählen des Kandidatenmoleküls, welches in der Lage ist mit der PPO spezifisch zu interagieren.

15. Verfahren zum Identifizieren von Liganden einer Protoporphyrinogen-IX-Oxidase, umfassend die folgenden Schritte:
(a) Erstellen einer dreidimensionalen Struktur einer PPO,
(b) Modellieren einer Verbindung, die in der Lage ist spezifische Wechselwirkungen mit der PPO einzugehen.

16. Verfahren gemäß Anspruch 14 oder 15, **dadurch gekennzeichnet, dass** in Schritt (a) nur die Koordinaten von zumindest zwei Atomen einer PPO erstellt werden.

17. Verfahren gemäß Anspruch 14 bis 16, **dadurch gekennzeichnet, dass** in Schritt (a) die Koordinaten von Atomen ausgewählt aus den Aminosäuren Arg⁹⁸, Ala¹⁷⁴, Gly¹⁷⁵, Thr¹⁷⁶, Cys¹⁷⁷, Gly¹⁷⁸, Leu ³³⁴, Phe³⁵³, Gly³⁵⁴, Val³⁵⁵, Leu³⁵⁶, Leu³⁶⁹, Gly³⁷⁰, Thr³⁷¹, Leu³⁷², Phe³⁹², Phe⁴³⁹ erstellt werden.

18. Verfahren gemäß einem der Ansprüche 14 bis 17, **dadurch gekennzeichnet, dass** die Koordinaten in Schritt (a) den Strukturkoordinaten gemäß Tabelle 2 entsprechen.

19. Verfahren gemäß einem der Ansprüche 14 bis 18, umfassend zusätzlich die folgenden Schritte:
(a) Synthetisieren des identifizierten Liganden,
(b) Bildung eines Komplexes aus einer PPO und dem Liganden,
(c) Röntgenstrukturanalyse des Komplexes, und
(d) Bestimmung der Fähigkeit des Liganden spezifische Wechselwirkungen mit der PPO einzugehen.

20. Verfahren gemäß einem der Ansprüche 14 bis 18, umfassend zusätzlich die folgenden Schritte:
(a) Synthetisieren des identifizierten Liganden, und
(b) Bestimmung der Fähigkeit des Liganden die enzymatische Aktivität der PPO *in vitro* zu inhibieren.

21. Ein Verfahren zur Bestimmung der Struktur eines an eine PPO gebundenen Liganden, umfassend die folgenden Schritte:
(a) Herstellung eines Kristalls des Komplexes aus der PPO und dem Liganden, und
(b) Bestimmung der Struktur des Komplexes unter Verwendung der Strukturkoordinaten gemäß Tabelle 2.

22. Ein Verfahren zur Bekämpfung unerwünschter Pflanzen, **dadurch gekennzeichnet, dass** man Verbindungen, die durch ein Verfahren gemäß einem der Ansprüche 14 bis 20 aufgefunden wurden, auf die unerwünschten Pflanzen und/oder ihren Lebensraum einwirken lässt.

23. Verwendung eines Kristalls einer Protoporphyrinogen-IX-Oxidase gemäß einem der Ansprüche 1 bis 9 zum Auffinden von Herbiziden.


SEQUENCE LISTING

<110>  Bayer CropScience AG

<120>  Kristallstruktur der mitochondrialen
       Protoporphyrinogen-IX-Oxidase

<130>  BCS 03-3112

<160>  1

<170>  PatentIn version 3.2

<210>  1
<211>  503
<212>  PRT
<213>  Nicotiana tabacum

<220>
<221>  PRT
<222>  (1)..(503)

<400>  1

Ala Pro Ser Ala Gly Glu Asp Lys His Ser Ser Ala Lys Arg Val Ala
1               5                   10                  15

Val Ile Gly Ala Gly Val Ser Gly Leu Ala Ala Ala Tyr Lys Leu Lys
            20                  25                  30

Ile His Gly Leu Asn Val Thr Val Phe Glu Ala Glu Gly Lys Ala Gly
        35                  40                  45

Gly Lys Leu Arg Ser Val Ser Gln Asp Gly Leu Ile Trp Asp Glu Gly
    50                  55                  60

Ala Asn Thr Met Thr Glu Ser Glu Gly Asp Val Thr Phe Leu Ile Asp
65                  70                  75                  80

Ser Leu Gly Leu Arg Glu Lys Gln Gln Phe Pro Leu Ser Gln Asn Lys
                85                  90                  95

Arg Tyr Ile Ala Arg Asn Gly Thr Pro Val Leu Leu Pro Ser Asn Pro

            100                 105                 110

Ile Asp Leu Ile Lys Ser Asn Phe Leu Ser Thr Gly Ser Lys Leu Gln
        115                 120                 125

Met Leu Leu Glu Pro Ile Leu Trp Lys Asn Lys Lys Leu Ser Gln Val
    130                 135                 140

Ser Asp Ser His Glu Ser Val Ser Gly Phe Phe Gln Arg His Phe Gly
145                 150                 155                 160

Lys Glu Val Val Asp Tyr Leu Ile Asp Pro Phe Val Ala Gly Thr Cys
                165                 170                 175

Gly Gly Asp Pro Asp Ser Leu Ser Met His His Ser Phe Pro Glu Leu
            180                 185                 190

Trp Asn Leu Glu Lys Arg Phe Gly Ser Val Ile Leu Gly Ala Ile Arg
        195                 200                 205

Ser Lys Leu Ser Pro Lys Asn Glu Lys Lys Gln Gly Pro Pro Gln Thr
    210                 215                 220

Ser Ala Asn Lys Lys Arg Gln Arg Gly Ser Phe Ser Phe Leu Gly Gly
225                 230                 235                 240

Met Gln Thr Leu Thr Asp Ala Ile Cys Lys Asp Leu Arg Glu Asp Glu
                245                 250                 255

Leu Arg Leu Asn Ser Arg Val Leu Glu Leu Ser Cys Ser Cys Thr Glu
            260                 265                 270

Asp Ser Ala Ile Asp Ser Trp Ser Ile Ile Ser Ala Ser Pro His Lys
        275                 280                 285

Arg Gln Ser Glu Glu Glu Ser Phe Asp Ala Val Ile Met Thr Ala Pro
    290                 295                 300

Leu Cys Asp Val Lys Ser Met Lys Ile Ala Lys Arg Gly Asn Pro Phe
305                 310                 315                 320

Leu Leu Asn Phe Ile Pro Glu Val Asp Tyr Val Pro Leu Ser Val Val
                325                 330                 335

Ile Thr Thr Phe Lys Arg Glu Asn Val Lys Tyr Pro Leu Glu Gly Phe
            340                 345                 350

Gly Val Leu Val Pro Ser Lys Glu Gln Gln His Gly Leu Lys Thr Leu
        355                 360                 365

Gly Thr Leu Phe Ser Ser Met Met Phe Pro Asp Arg Ala Pro Asn Asn
    370                 375                 380

Val Tyr Leu Tyr Thr Thr Phe Val Gly Gly Ser Arg Asn Arg Glu Leu
385                 390                 395                 400

Ala Lys Ala Ser Arg Thr Glu Leu Lys Glu Ile Val Thr Ser Asp Leu
                405                 410                 415

Lys Gln Leu Leu Gly Ala Glu Gly Glu Pro Thr Tyr Val Asn His Leu
            420                 425                 430

Tyr Trp Ser Lys Ala Phe Pro Leu Tyr Gly His Asn Tyr Asp Ser Val
        435                 440                 445

Leu Asp Ala Ile Asp Lys Met Glu Lys Asn Leu Pro Gly Leu Phe Tyr
    450                 455                 460

Ala Gly Asn His Arg Gly Gly Leu Ser Val Gly Lys Ala Leu Ser Ser
465                 470                 475                 480

Gly Cys Asn Ala Ala Asp Leu Val Ile Ser Tyr Leu Glu Ser Val Ser
                485                 490                 495

Thr Asp Ser Lys Arg His Cys
            500